# EUROPEAN PATENT SPECIFICATION

(11) **EP 1 389 997 B1**
(45) Date of publication and mention of the grant of the patent: **08.12.2010**
(21) Application number: 02725654.4
(22) Date of filing: 12.04.2002
(51) Int. Cl.: C12N 15/82, C12N 15/36, A01H 5/00

(54) **METHODS AND COMPOSITIONS FOR STABLE TRANSGENIC PLANT PHARMACEUTICALS AND THEIR USE AS CONTRACEPTIVES**
VERFAHREN UND ZUSAMMENSETZUNGEN FÜR STABILE TRANSGENE PHARMAZEUTIKA UND IHRE VERWENDUNG ALS KONTRAZEPTIVA
METHODES ET COMPOSITIONS POUR MEDICAMENTS STABLES A BASE DE PLANTES TRANSGENIQUES ET LEUR UTILISATION COMME CONTRACEPTIFS

(30) Priority: 13.04.2001 US 283884 P
(43) Date of publication of application: 25.02.2004
(73) Proprietor: BOYCE THOMPSON INSTITUTE FOR PLANT RESEARCH, Ithaca New York 14853-1801 (US)
(72) Inventor: KIRK, Dwayne, Mesa, AZ 85215 (US); MASON, Hugh, Ithaca, NY 14850 (US); WALMSLEY, Amanda, Mesa, AZ 85215 (US); ARNTZEN, Charles, Superstition Mountain, AZ 85216-1806 (US)
(74) Representative: Soames, Candida Jane
(86) International application number: PCT/US2002/011693
(87) International publication number: WO 2002/083072

(56) References cited:
- EP-A- 0 787 496
- WO-A1-86/06076
- WO-A1-99/65940
- FR-A- 2 760 366
- US-A- 5 484 719
- US-A- 5 679 880
- US-A- 5 686 079
- DOMANSKY N.: 'Transgenic plants as edible vaccines-reality and future prospects' BIOPOLIMERY I KLETKA vol. 15, no. 1, 1999, pages 5 - 9, XP002955962
- OGRA P. ET AL.: 'Vaccination strategies for mucosal immune responses' CLINICAL MICROBIOLOGY REVIEWS vol. 14, no. 2, April 2001, pages 430 - 445, XP002955963
- MASON H. ET AL.: 'Expression of norwalk virus capsid protein in transgenic tobacco and potato and its oral immunogenicity in mice' PROC. NATL. ACAD. SCI. USA vol. 93, no. 11, May 1996, pages 5335 - 5340, XP002025358
- MASON ET AL.: 'Edible vaccine protects mice against escherichia coli heat-labile enterotoxin (LT): potatoes expressing a synthetic LT-B gene' VACCINE vol. 16, no. 13, 1998, pages 1336 - 1343, XP004124602
- STOGER ET AL.: 'Cereal crops as viable production and storage systems for pharmaceutical scFv antibodies' PLANT MOLECULAR BIOLOGY vol. 42, no. 4, March 2000, pages 583 - 590, XP002955964
- LARRICK ET AL.: 'Production of antibodies in transgenic plants' RESEARCH IN IMMUNULOGY vol. 149, no. 6, 1998, pages 603 - 608, XP002955965
- MASON ET AL.: 'Transgenic plants as vaccine production systems' TRENDS IN BIOTECHNOLOGY vol. 13, no. 9, September 1995, pages 388 - 392, XP002024035
- MESTECKY ET AL.: 'Routes of immunization and antigen delivery systems for optimal mucosal immune responses in humans' BEHR. INST. MITT. vol. 98, 1997, pages 33 - 43, XP000993208
- TACKET ET AL.: 'Immunogenicity in humans of a recombinant bacterial antigen delivered in a transgenic potato' NATURE MEDICINE vol. 4, no. 5, May 1998, pages 607 - 609, XP001063283
- SMITH ET AL.: 'Plant-derived immunocontraceptive vaccines' REPRODUCTION, FERTILITY AND DEVELOPMENT vol. 9, no. 1, 1997, pages 85 - 89, XP002955966
- RUEDL ET AL.: 'Features of oral immunization' INT. ARCH. ALLERGY IMMUNOL. vol. 108, no. 4, 1995, pages 334 - 339, XP000993237
- YU ET AL.: 'Assembly of cholera toxin-antigen fusion proteins in transgenic potato' TRANSGENICS vol. 3, no. 2-4, 2001, pages 153 - 162, XP002955967
- ARAKAWA ET AL.: 'Suppression of autoimmune diabetes by a plant-derived cholera toxin B subunit-human glutamate decarboxylase fusion protein' TRANSGENICS vol. 3, no. 1, 1999, pages 51 - 60, XP001040321
- WONGSAMUTH et al. 'Production of monoclonal antibody by tobacco hairy roots'. Book of Abstracts, 211th ACS nation meeting, New Orleans, LA March 24-28 1996, BIOT-166, publisher, American Chemical Society, Washington, abstract no. 166. XP002104103

## Description

### BACKGROUND OF THE INVENTION

This invention relates to processing techniques for the production of pharmaceutically active proteins such as vaccines, antibodies or other therapeutic compounds derived from transgenic plants, as well as to their administration to humans and animals for immunocontraception, vaccination against pathogenic diseases, or therapeutic treatment against infection of other immunoreactive compounds. The development of plant based vaccines has significantly advanced the available approaches to vaccination of humans and animals. The use of transgenic plants for the development of vaccines, antibodies, and other human and veterinary therapeutics has not only increased the efficiency of therapeutic protein production, but it has also substantially reduced primary production costs.

With regards to production of plant based vaccines, there has been significant advancements in expression of proteins in transgenic plants. For example, various expression forms, aggregates or fusions have been used to increase production of vaccine subunits, antibodies, and therapeutic or contraceptive proteins, as described in U.S. patents 5,679,880, 5,484,719, 5,686,079 and U.S. patent application 2002/0006411. However, the current state of transgenic plant vaccine technology still requires that the pharmaceutical protein be purified away from the transgenic plant material, or that standardized raw crude plant tissues be consumed. Unfortunately, these options for producing administratable plant-derived pharmaceuticals have inherent drawbacks.

The preferred plant-derived pharmaceutical is standardized plant tissue that can be administered orally. More specifically, it is preferred that the plant material can be stored, shipped and administered at ambient temperatures. However, unpurified transgenic plant based vaccines have the inherent problems of i) variable protein expression levels found from plant to plant and ii) perishability, hence protein degradation. Freshly harvested produce such as fruit, tubers, roots, foliage or any other plant tissue is limited due to their relatively short shelf life, in comparison to conventional pharmaceutical requirements. In addition, the variable level of transgenic protein accumulation found between different plants, clones or plant tissues poses significant difficulty in achieving dose consistency, which is mandatory for pharmaceutical application.

Conventional pharmaceutical technology provides methods for the extraction, purification and concentration of pharmaceutical proteins, however this is expensive, introduces the need for refrigeration of the purified product, and does not allow for protective encapsulation of the transgenic protein by the plant cell itself. Thus, there is a need in the art for production methods of stable transgenic plant based pharmaceuticals that both bypass the drawback of variable protein expression found from plant to plant and that do not require further purification from the original plant material. The processing methods described herein provide for a manner of producing a room temperature stable homogenous formulation that can be used across a broad variety of applications. In addition, the ambient-stable homogenate can inherently be pooled and quality control tested on a batch per batch basis. Further, the described methods of transgenic plant processing allow for the convenient addition of adjuvants, buffers, antioxidents, stabalizers, or antigenic powders throughout the processing enabling a wide range of pharmaceutical formulations to be produced, efficiently and cost effectively. The stable dry homogenates can be used not only for vaccination and treatment of pathogenic diseases, but also may be used for prevention of fertilization.

Fertilization is a complex interaction between key regulatory molecules on the surface of the sperm and egg. The vital role that these molecules play in the reproductive process make them likely targets for control of fertility in ma species.

All mammalian eggs are surrounded by a zona pellucida, a relatively simple coat composed of three glycoproteins (ZP1, ZP2, and ZP3) that provide the egg's sperm receptor. The zona pellucida of some non-mammalian species also includes a fourth glycoprotein, ZP4. The glycoprotein components of the zona pellucida serve specific functions during fertilization and early development, which are well defined in the mouse. ZP1 is a structural protein that crosslinks filaments of ZP2 and ZP3. During fertilization sperm initially bind to ZP3, which induces the sperm acrosome reaction. After induction of the acrosome, ZP2 acts as a secondary sperm receptor which is necessary for penetration of the sperm through the zona pellucida. Following fertilization the proteolytic cleavage of ZP2 and modification of ZP3 are thought to be involved in the prevention of polyspermy.

The important role of the zona pellucida during fertilization, its unique expression in growing oocytes and strong immunogenicity has made it an attractive target for the development of immunocontraceptive vaccines (reviewed by Epifano and Dean Reprod Fertil Dev. 1994;6:319). Immunization with whole zonae or components of the zona pellucida, has been shown to inhibit fertilization in several species including primates, rabbits, rodents and dogs (reviewed by Prasad et al., (1996) J Reprod Fertil Suppl. 50:143). While the compositions described in these references may inhibit fertilization, they must be administered parenterally by darting, or injection, and are thus impractical for economical, wide spread use. The compositions described in these references, in contrast to the present invention, could not be effectively administered orally as a raw bait, or food, or as processed material for digestive consumption.

Fitchen et al. ((1995) Vaccine 13:1051) attempted to produce contraceptive proteins in plants for purified delivery to target or model animal species by injection. In this example, a murine ZP3 epitope was engineered within the sequence of tobacco mosaic virus (TMV) coat protein and expressed in tobacco during TMV infection. The virus particles (displaying the ZP epitope on the outer surface of the virus) were crudely purified from the plant material for parenteral administration. However, the viral particles were not effective in reducing litter size in the mouse model. Fitchen et al. does not relate to a method of inducing contraception in an animal, or to orally administering a transgenic plant to the animal, wherein the oral administration reduces average litter size by at least 50%.

There is a need in the art for a plant composition which may be conveniently administered orally to an animal as a raw bait, or food for digestive consumption, and which significantly reduces litter size in animals to which it is administered. There is a need in the art to avoid the use of plant viruses for expression. Therefore, there is a need in the art to express contraceptive proteins directly by the plant cells themselves, thus providing a mucosal immune response upon consumption of the plant material by an animal.

As the estimated human population continues to expand beyond the 6 billion marks, the population distribution of other wildlife species will continue to be heavily influenced by the environmental activities of industrialised man. During the twentieth century, man's use of land and water resources has had an increasingly profound affect on the population dynamics of animal species. The World Wildlife Fund now predicts that as much as one third of the world's species may be extinct within the next twenty years. In addition to reducing specific fauna and flora populations to extinction or endangerment, overabundance of particular species has also been induced by the modem human race. As a specific example, the survival of herbivorous animals has been leveraged throughout the ages by natural forces such as geographical containment, natural predators, climatic conditions, and availability of food. Amongst the influences too many to list, the industrialised world has cleared forests for agriculture and construction, reduced the population and natural range of predatory animals, introduced species across geographical barriers, provided unnatural shelters against climatic extremes, and spawned a social culture where proposed animal rights and human interest often conflict. There are now ma examples where overabundant herbivorous populations are in direct conflict with human coexistence and agricultural production. Due to an assortment of reasons, population control for ma of these species has become unachievable, and alternative methods are sought.

Therefore, there exists the need for the development of humane techniques of population management to mitigate the damage that results from the unchecked proliferation of animal species, whether the damage is economic, environmental, or to animal welfare. Furthermore, there exists a need to do this with formulations that can be produced efficiently and cost effectively.

### SUMMARY OF THE INVENTION

The present invention provides methods for the production of transgenic plant biopharmaceuticals. The methods described herein allow for the production of dry transgenic plant material that is stable at room temperature, homogeneous, and pharmaceutically potent. In general, either intact or partitioned transgenic plants are dried or freeze dried through food processing techniques known in the art. The dry homogenate, which contains the biopharmaceutical product, can then be used therapeutically without the need to further extract, purify, or precipitate the pharmaceutical protein. The stable dry homogenates can be used for vaccination, treatment of pathogenic diseases, and as a contraceptive. The present invention further provides a safe and effective vaccine for the control of fertiliy and fecundity. Thus, the invention satisfies the need in the art for efficient methods of producing stable plant-based pharmaceuticals and the need for convenient and cost effective methods of population control for domestic animals, feedlot species, zoological captive specimens, game reserve populations and humans.

Herein methods for the production and use of plant based pharmaceuticals are disclosed.

The present invention provides a method for producing a stable dry homogenate of a transgenic plant expressing a heterologous protein comprising: i) obtaining either intact or partitioned transgenic plant material, ii) dehydrating said transgenic plant material and, iii) mixing said transgenic plant material into a homogenate either before or after said dehydrating step, thereby producing a stable dry homogenate of a transgenic plant expressing a heterologous pharmaceutical protein.

In one embodiment, the dehydration is accomplished by freeze drying, air drying, or spray drying said material.

In another embodiment, the method further comprises the step of partitioning the dehydrated material before said mixing step.

In a still further embodiment, the method additionally comprises the addition of an excipient.

The invention further provides for the stable dry homogenate that is produced by the methods described above.

In one embodiment the stable dry homogenate contains an antigen or an antibody.

The invention provides for a stable dry homogenate comprising a dehydrated transgenic plant material containing a transgenic protein.

Further disclosed is a pharmaceutical composition comprising a stable dry homogenate that comprises a dehydrated transgenic plant material containing a transgenic protein, optionally mixed with a pharmaceutically acceptable carrier.

Still further disclosed is a method for preventing a pathogenic disease in an animal comprising administering the pharmaceutical composition comprising a stable dry homogenate that comprises a dehydrated transgenic plant material containing a transgenic protein, optionally mixed with a pharmaceutically acceptable carrier.

Also disclosed is a method for delivering a transgenic protein to an animal comprising administering to an animal the pharmaceutical composition comprising a stable dry homogenate that comprises a dehydrated transgenic plant material containing a transgenic protein, optionally mixed with a pharmaceutically acceptable carrier.

In one embodiment, the transgenic protein present in the stable dry homogenate is a vaccine.

The invention further provides for methods of contraception using transgenic plant based pharmaceuticals.

The present invention utilizes plant chromosomal expression of the contraceptive protein in the absence of a viral intermediate.

The invention provides a method of contraception comprising:
administering transgenic plant material comprising a nucleic acid molecule which encodes a contraceptive polypeptide to an animal; and
wherein said administering reduces the average number of offspring produced per breeding cycle by said animal by at least 50%.

The invention also provides a method of contraception comprising:
administering transgenic plant material comprising a nucleic acid molecule which encode a contraceptive polypeptide to an animal in an amount effective to induce contraception; and
wherein said administering induces a mucosal immune response in said animal to said contraceptive protein.

The invention further provides a method of contraception comprising:
administering transgenic plant material comprising a nucleic acid molecule which encodes a contraceptive polypeptide to an animal, wherein said nucleic acid is integrated into a plant chromosome; and
wherein said method of contraception reduces the average number of offspring per breeding cycle produced by said animal to which said transgenic plant material has been administered by at least 50%.

The invention further provides a method of contraception comprising:
administering transgenic plant material comprising a nucleic acid molecule which encodes a contraceptive polypeptide to an animal in an amount effective to induce contraception, wherein said nucleic acid is integrated into a plant chromosome; and
wherein said method of contraception reduces the average number of offspring per breeding cycle produced by said animal to which said transgenic plant material has been administered by at least 50%.

In a preferred embodiment, said transgenic plant material is selected from the group consisting of: leaf, root, shoot, stem, fruit, tuber, flower, and seed.

In a more preferred embodiment, said transgenic plant material is edible. In another preferred embodiment, said contraceptive polypeptide encoded by said nucleic acid is selected from the group consisting of a zona pellucida glycoprotein, GnRH, LHRH, LH, LDH and anti-sperm antigens.

In a more preferred embodiment, said zona pellucida glycoprotein is selected from the group consisting of ZP1, ZP2, ZP3, and ZP4.

In a more preferred embodiment, said zona pellucida glycoprotein is ZP3.

In a preferred embodiment, said contraceptive protein is species-specific.

In another preferred embodiment, said nucleic acid encoding a contraceptive protein further comprising a sequence encoding a mucosal targeting protein.

On a more preferred embodiment, said mucosal targeting protein is the *E. coli* enterotoxin subunit B.

In another preferred embodiment, said transgenic plant material is administered as a whole plant.

In another preferred embodiment, said transgenic plant material is administered by mucosal delivery.

In yet another preferred embodiment, said mucosal administration is oral, nasal, or ocular delivery. The invention provides a method of inducing a mucosal immune response comprising:
administering to an animal transgenic plant material comprising a nucleic acid sequence which encodes a contraceptive polypeptide, wherein said administering induces a mucosal immune response in said animal to said contraceptive protein, and wherein said administering reduces the average number of offspring produced per breeding cycle by said animal by at least 50%.

In a preferred embodiment, said transgenic plant material is selected from the group consisting of: leaf, root, shoot, stem, fruit, tuber, flower, and seed.

In another preferred embodiment, said transgenic plant material is in the form of an edible plant.

In yet another embodiment, said contraceptive polypeptide encoded by said nucleic acid is selected from the group consisting of a zona pellucida glycoprotein, GnRH, LHRH, LH, LDH and anti-sperm antigen.

In a preferred embodiment, said zona pellucida glycoprotein is selected from the group consisting of ZP1, ZP2, ZP3, and ZP4.

In a more preferred embodiment, said zona pellucida glycoprotein is ZP3.

In another embodiment, said contraceptive protein is species-specific.

In another preferred embodiment, said nucleic acid encoding a contraceptive polypeptide further encodes a mucosal targeting protein.

In a more preferred embodiment, said mucosal targeting protein is the *E. coli* enterotoxin subunit B.

The invention provides a transgenic plant comprising transgenic plant material which express a nucleic acid sequence encoding a contraceptive polypeptide, wherein administering said transgenic plant material to an animal induces a mucosal immune response in said animal to said contraceptive protein, and wherein said administering reduces the average number of offspring produced per breeding cycle by said animal by at least 50%.

The invention also provides a transgenic plant comprising transgenic plant material which express a nucleic acid sequence encoding a contraceptive polypeptide, wherein said nucleic acid sequence encoding a contraceptive polypeptide is not expressed on a viral particle, and wherein administration of said transgenic plant material to an animal induces a mucosal immune response in said animal to said contraceptive protein.

The invention further provides a transgenic plant comprising transgenic plant material which express a nucleic acid sequence encoding a contraceptive polypeptide, and wherein said administration reduces the average number of offspring per breeding cycle produced by said animal to which said transgenic plant material is administered by at least 50%.

The invention further provides an edible transgenic plant comprising transgenic plant material which express a nucleic acid sequence encoding a contraceptive polypeptide, wherein administration of said transgenic plant material to an animal induces a mucosal immune response in said animal to said contraceptive protein, and wherein said nucleic acid is integrated into a plant chromosome.

In one embodiment, said contraceptive polypeptide is selected from the group consisting of a zona pellucida glycoprotein, GnRH, LHRH, LH, LDH, and anti-sperm antigens.

In a preferred embodiment, said zona pellucida glycoprotein is selected from the group consisting of ZP1, ZP2, ZP3, and ZP4.

In a more preferred embodiment, said zona pellucida glycoprotein or fragment thereof is species-specific.

In another more preferred embodiment, said zona pellucida glycoprotein is ZP3 or a fragment thereof.

In one embodiment, said nucleic acid sequence further encodes a mucosal targeting protein.

In a preferred embodiment, said mucosal targeting protein is the *E. coli* enterotoxin subunit B.

In another preferred embodiment, said plant is monocotyledonous.

In yet another preferred embodiment, said plant is dicotyledonous.

In still another preferred embodiment, said plant is selected from the group consisting of tomato plants, rice plants, wheat plants, corn plants, carrot plants, potato plants, apple plants, soybean plants, alfalfa plants, medicago plants, vegetable plants, and fruit plants.

The present invention provides a plant cell comprising a nucleic acid vector, wherein the nucleic acid vector encodes a contraceptive polypeptide, wherein the nucleic acid vector is incorporated into and expressed from the chromosome of the plant cell, and wherein administration of the plant cell to an animal reduces the average number of offspring produced per breeding cycle by the animal by at least 50%.

In one embodiment, the contraceptive polypeptide encoded by the nucleic acid vector is selected from the group consisting of a zona pellucida glycoprotein, GnRH, LH, LDH, and anti-sperm antigens.

In a further embodiment, the zona pellucida glycoprotein is selected from the group consisting of ZP1, ZP2, ZP3, and ZP4.

In a still further embodiment, the contraceptive polypeptide is species-specific.

In a preferred embodiment, the zona pellucida glycoprotein is ZP3.

In one embodiment, the nucleic acid vector further encodes a mucosal targeting protein.

In a preferred embodiment, the mucosal targeting protein is the E. coli enterotoxin subunit B.

In one embodiment, the plant cell is obtained from a monocotyledonous plant.

In one embodiment, the plant cell is obtained from a dicotyledonous plant.

In a preferred embodiment, the plant cell is selected from the group consisting of tomato plant cells, rice plant cells, wheat plant cells, corn plant cells, carrot plant cells, potato plant cells, apple plant cells, soybean plant cells, vegetable plant cells, and fruit plant cells.

The present invention further provides a plasmid vector for transforming a plant cell comprising: a nucleic acid sequence encoding a contraceptive polypeptide, a plant-functional promoter operably linked to the nucleic acid sequence encoding the contraceptive polypeptide which is capable of directing the synthesis of the contraceptive polypeptide in the plant cell, and wherein administering the plant cell comprising the plasmid vector to an animal induces a mucosal immune response in the animal, and wherein the administering reduces the average number of offspring produced per breeding cycle by the animal by at least 50%.

In one embodiment, the contraceptive polypeptide encoded by the nucleic acid sequence is selected from the group consisting of a zona pellucida glycoprotein, GnRH, LHRH, LH, LDH, and anti-sperm antigens.

In a preferred embodiment, the zona pellucida glycoprotein is selected from the group consisting of ZP1, ZP2, ZP3, and ZP4.

In a further embodiment, the plant cell is obtained from an edible plant.

In one embodiment, the plasmid vector further comprises a nucleic acid sequence encoding a mucosal targeting protein.

In a preferred embodiment, the mucosal targeting protein is the E. coli enterotoxin subunit B.

The invention further provides a food composition comprising at least a portion of the transgenic plant described above, capable of being ingested by an animal, wherein ingestion of the transgenic plant induces a mucosal immune response in the animal, and wherein the ingestion reduces the average number of offspring produced per breeding cycle by the animal by at least 50%.

The invention further provides a pharmaceutical composition comprising at least a portion of the transgenic plant described above in conjunction with a pharmaceutically acceptable carrier, wherein administration of the pharmaceutical composition to an animal reduces the average number of offspring produced per breeding cycle by the animal by at least 50%.

The present invention further provides a composition comprising a saponin adjuvant in admixture with processed transgenic plant material for oral administration to an animal, wherein administration of the composition reduces the number of offspring produced by the animal per breeding cycle by at least 50%.

As used herein, "contraceptive" refers to an agent which is used for contraception. "Contraception" is defined as reducing the number of offspring produced by an animal in a single breeding cycle, or preventing the fertilization of an oocyte by a spermatozoon in a sexual reproduction. The "prevention" of fertilization may be confirmed by a reduction in the number of offspring produced by a female animal to which the "contraceptive" is administered as described below. As used herein, "contraceptive" refers to the described reduction or prevention of fertilization, or prevention of zygote implantation, for a minimum of one breeding cycle.

As used herein, "sexual reproduction" refers to the exclusive form of reproduction for mammals. It involves the fertilization of one type of sex cell by another type (gamete: ovum for female and sperm for male), producing a new cell (called a zygote), which develops into a new organism. Ova (plura for Ovum) or sperms are produced in the reproduction organs of the female or the male. In a single sexual reproduction process, after a sperm fertilizes the ovum, the female carrying the fertilized ovum is usually not capable of producing more mature ova until the offspring is reproduced (born).

As used herein, one "breeding cycle" refers to a single process of sexual reproduction which comprises the fertilization of a mature ovum and the reproduction of offspring before a subsequent reproduction can occur. One "breeding cycle", according to the invention, also refers to a potential single reproduction process blocked by contraception which otherwise might occur.

As used herein, "contraceptive protein" refers to polypeptide, or fragment of a polypeptide, which reduces the number of offspring produced by an animal per breeding cycle. For animal species which produce an average litter of 3 offspring or more, a "contraceptive protein" is a polypeptide which reduces litter size by at least 60%, 70%, 80%, 90% and even up to 100%. For animal species which produce an average litter of 1 or 2 offspring, a "contraceptive protein" refers to a protein or polypeptide which reduces litter size by at least 50% (that is, from 2 offspring to 1), and up to 100% (that is, from 1 or 2 offspring to no offspring). Alternatively a "contraceptive protein" can refer to a polypeptide which prevents the fertilization of an oocyte by a spermatozoon, or which prevents the implantation of a fertilized egg in the uterus of an animal, and thus reduces average litter size as described above. Alternatively, a "contraceptive protein" refers to a polypeptide which is capable of stimulating a mucosal immune response in an animal, wherein the immune response raises antibodies to the "contraceptive protein". The antibodies may bind to sites on sex hormones, the oocyte or the spermatocyte and prevent sexual maturity, fertilization of the oocyte and/or implantation of a fertilized oocyte in the uterus of the animal, thus reducing litter size. Examples of "contraceptive proteins" useful in the present invention include, but are not limited to zona pellucida glycoproteins, gonadotrophin releasing hormone (GnRH), leutinizing hormone releasing hormone (LHRH), leutinizing hormone (LH), lactate dehydrogenase (LDH), and anti-sperm antigens. "Anti-sperm antigens" include, but are not limited to SP5, SP56, fertilin, PH30, LDH-C4, and P10.

As used herein, "an amount effective to induce contraception", as it refers to an amount of transgenic plant material which induces contraception. Preferably, transgenic plant material in "an amount effective to induce contraception" is an amount of plant material which reduces litter size by 10%, 30%, 60%, 80%, and up to 100%. For animal species which produce an average litter of 1 or 2 offspring per breeding cycle, transgenic plant material in "an amount effective to induce contraception" is an amount of plant material which reduces litter size by at least 50%, and up to 100%. As used herein, "an amount effective to induce contraception" refers to an amount of transgenic plant material which induces contraception is at least about 5 µg of contraceptive protein per gram of dry transgenic plant material by weight, preferably at least 10 µg, 20 µg, 40 µg, 60 µg, 80 µg, 100 µg, 120 µg, 140 µg, 150 µg or more, of contraceptive protein per gram of dry transgenic plant weight.

As used herein, "trangenic plant" refers to a plant, the cells of which stably express a "heterologous" foreign gene, wherein the foreign gene is integrated into the plant cell chromosome and does not carry with it a viral vector sequence unique to a virus, where the foreign gene is passed onto the next plant generation and is capable of being expressed from the host plant cell chromosome. A "transgenic plant" comprises a "plurality of transgenic plant cells". A "transgenic plant" refers to the whole plant, or a part thereof including, but not limited to roots, stems, leaves, stalks, seeds, fruit, tubers, flowers, pollen, and the like. Examples of heterologous foreign genes include, but are not limited to, Norwalk virus capsid protein (NVCP), Avian Influenza hemagglutination antigen (AIV-HA), Newcastle Disease Virus huraminidase (NDV-HN), zona pellucida glycoprotein 3 (ZP3), and Hepatitis B surface Antigen (HBsAg).

As used herein, a "heterologous protein" refers to a protein expressed from a "heterologous gene". A "heterologous gene" is a gene or gene fragment that encodes a protein obtained from one or more sources other than the genome of the species of plant within which it is ultimately expressed. The source can be natural, e.g., the gene can be obtained from another source of living matter, such as bacteria, virus, yeast, fungi and the like, or a different species of plant. The source can also be synthetic, e.g., the gene or gene fragment can be prepared in vitro by chemical synthesis. "Heterologous" can also be used in situations where the source of the gene fragment is elsewhere in the genome of the plant in which it is ultimately expressed. The "heterologous protein" may be an antigen, or other therapeutic proteins, such as, but not limited to endostatin or angiostatin. The "heterologous protein" may also be an antibody or antibody complex, for example "plantibodies". "Plantibodies" are antibodies that are made in plants.

As used herein, "transgenic plant material" refers to a trangenic plant, either processed or not processed as defined herein. The "transgenic plant material", according to the invention, may be a whole transgenic plant, or a portion of a transgenic plant including, but not limited to a "transgenic leaf", "tansgenic root", "transgenic shoot", "transgenic stem", "transgenic fruit", "transgenic tuber", "transgenic flower" and "transgenic seed". "Transgenic plant material" also refers to one or a plurality of "transgenic plant cells". A "transgenic plant cell" refers to a plant cell which stably expresses a foreign gene, wherein the foreign gene is integrated into the plant cell chromosome and does not carry with it a viral vector sequence unique to a virus, where the foreign gene is passed onto the next cell generation and is capable of being expressed from the host plant cell chromosome. In addition, "transgenic plant material" refers to a "transgenic cell suspension" comprising one or a plurality of "transgenic plant cells" obtained by well-known cell culture techniques (Street, HE. 1973, Plant tissue and cell culture: botanical monographs. Vol II, University of California, Berkely)

As used herein "intact" transgenic plant material refers to the whole plant or plant parts that have not been further processed to reduce their size for example, by slicing, dicing, cutting, or pureeing. In general, the plant material is "intact" or "whole" as harvested but may be simply part of the transgenic plant. For example an intact, "transgenic leaf", "tansgenic root", "transgenic shoot", "transgenic stem", "transgenic fruit", "transgenic tuber", "transgenic flower" or "transgenic seed".

As used herein, "partitioned" transgenic plant material refers to a "whole" plant or "intact" plant parts that have been reduced in size by means known in the art. Means for "partitioning" include, but are not limited to, cubing, quartering, mincing slicing, dicing, pureeing, or pulping, milling, crushing, pressing, or cracking. The transgenic plant material, either cut or uncut, cooked or uncooked, may be pulped by any process known to those of skill in the art including juicing, blending, or pureeing in a kitchen blender at room temperature for up to 20 minutes. The pulped plant material may be mixed with suitable excipients that are pharmaceutically acceptable and compatible with the transgenic protein. The transgenic plant material may be "partitioned" into a fine powder.

As used herein, "homogenation" refers to reduction of larger plant organs, to a uniform mixture whereby variance in the amounts of homologous protein from tissue to tissue is combined into a uniform batch of material. Thus the term "homogeneous" or "homogenate" also refers plant material which has been partitioned or reduced such that the plant tissues, which may contain different amounts of plant proteins and/or transgenic protein from tissue to tissue, is combined into a batch of material containing a uniform amount of such protein.

As used herein, an "edible plant" refers to a plant which may be consumed by an animal, has nutritional value and is not toxic. An "edible plant" may be a "food" which is a plant or a material obtained from a plant which is ingested by humans or other animals. The term "food" is intended to include plant material which may be fed to humans and other animals or a processed plant material which is fed to humans and other animals. Materials obtained from a plant are intended to include a component of a plant which is eventually ingested by a human or other animal. Examples of "edible plant" include, but are not limited to, tomato plants, rice plants, wheat plants, corn plants, carrot plants, potato plants, apple plants, soybean plants, alfalfa plants, medicago plants, vegetable plants, and fruit plants.

In some cases an "edible plant" is "capable of being ingested for its nutritional value", which refers to a plant or portion thereof that provides a source of metabolizable energy, supplementary or necessary vitamins or co-factors, roughage or otherwise beneficial effect upon ingestion by an animal. Thus, where the animal to be treated by the methods of the present invention is an herbivore capable of bacterial-aided digestion of cellulose, such a food might be represented by a transgenic grass plant. Other edible plants include vegetables and fruits. Similarly, although transgenic lettuce plants, for example, do not substantially contribute energy sources, building block molecules such as proteins, carbohydrates or fats, nor other necessary or supplemental vitamins or cofactors, a lettuce plant transgenic for the nucleic acid molecules described herein used as food for an animal would fall under the definition of a food as used herein if the ingestion of the lettuce contributed roughage to the benefit of the animal, even if the animal could not digest the cellulosic content of lettuce. An "edible plant" therefore excludes tobacco.

As used herein "processed" transgenic plant material can refer to plant material that is "cut", wherein "cut" refers to "chopping", "mincing", "dicing", or otherwise reducing the plant material to smaller pieces with a minimum size of about 1 mm². Transgenic plant material, either "cut" or uncut may be further processed by "cooking" the plant material using any technique known in the art including, but not limited to boiling, baking, and sautéing, wherein the temperature of the plant material is raised to at least 140° C, 160° C, 180° C, and up to 200° C for at least about 5 minutes, and up to about 30 minutes. "Cooked" transgenic plant material, useful in the present invention is administered without purification of the contraceptive protein, as the "cooked" plant material may comprise disrupted plant cells. Transgenic plant material, either "cut" or uncut, "cooked" or uncooked may be further processed by "combining" the plant material with non-transgenic plant or non-plant material by, for example, mixing, wherein the transgenic plant material comprises at least 50% by weight of the combination of transgenic plant material and non-transgenic or non-plant material. For example, transgenic corn may be "processed" by "combining" the corn kernels with other vegetables, or transgenic soy plants may be "processed" by "combining" the plant material with other ingredients such as spices, and/or other flavorings to produce, for example soy burgers, soy shakes, or soy milk.

Alternatively, transgenic plant material, useful in the present invention may be "processed" by "liquefying" the transgenic plant material, either cut or uncut, cooked or uncooked, by any process known to those of skill in the art including juicing, blending, or pureeing in a kitchen blender at room temperature for up to 20 minutes. The "liquefied" plant material may then be administered directly to an animal, or may be further processed by mixing the "liquefied" plant material with suitable excipients which are pharmaceutically acceptable and compatible with the contraceptive protein. The "liquefied" plant material may be further "processed" by mixing the liquid plant material with a pharmaceutically acceptable cream, ointment, or salve. It is possible that during the "liquefication" of transgenic plant material, transgenic plant cells may become disrupted, thereby liberating their contents into the "liquefied" mixture. A "liquefied" mixture of this nature is useful in the present invention provided that the contraceptive proteins expressed by the transgenic plant cells is not purified from the "liquefied" plant material.

Alternatively, transgenic plant material, either cut or uncut, cooked or uncooked, useful in the present invention, may be "processed" by "lyophilizing" or "dehydrating" the plant material to remove water. "Dehydration" may be performed by air drying or spray drying or by "lyophilization", wherein the "lyophilization" refers to the preparation of a plant composition in dry form by rapid freezing and dehydration in the frozen state (sometimes referred to as sublimation). This process may take place under vacuum at a pressure sufficient to maintain frozen product with the ambient temperature of the containing vessel at about room temperature, preferably less than about 500 mTorr, more preferably less than about 200 mTorr, even more preferably less than about 1 mTorr, for between about 1 hour to 72 hours. Plant material may be "dehydrated" by placing the plant material in an oven with a temperature between about 60° C and 200° C for between about 1 hour to 72 hours. Plant material is deemed to be sufficiently "lyophilized" or "dehydrated" when the weight of the plant material ceases to change over time. For example, the weight of plant material placed in an oven at temperatures described above will decrease over time as water evaporates. When the weight ceases to change, all of the water has evaporated, and the plant material can be said to be "dehydrated". Preferably, by whatever drying method is used, the final material is dehydrated sufficiently to remove at least 90% of all the water content by weight. The "lyophilized" or "dehydrated" plant material may be further "processed" by emulsifying the "lyophilized" or "dehydrated" plant material with excipients which are pharmaceutically acceptable and compatible with the contraceptive polypeptide. Suitable excipients include, for example, water, saline, dextrose, glycerol, ethanol, or the like and combinations thereof, wherein the "lyophilized" or "dehydrated" plant material comprises at least 40% and preferably at least 50% by weight of the excipient mixture. In addition, if desired, the "lyophilized" or "dehydrated" plant material may contain minor amounts of auxiliary substances such as wetting or emulsifying agents, pH buffering agents, or adjuvants which enhance the effectiveness of the plant material. As used herein, "lyophilized" or "dehydrated" plant material may be further processed by admixing the plant material with a pharmaceutically acceptable creams, ointments, salves, or suppositories, wherein the plant material comprises at least 40% and preferably at least 50% by weight of the admixture. Alternatively, "dehydrated" plant material may be further processed by reconstituting the plant material with a liquid, such as, but not limited to, fruit juice, vegetable juice, milk, water, or other vaccine formulations to form multivalent or muticomponent vaccines.

As used herein "excipients" refer to extraneous material added at any stage of processing. For example, excipients may be added when partitioning the transgenic plant material, or added during dehydration, or added upon mixing pre- or post dehydration. Suitable excipients include, but are not limited to, adjuvants, buffers, sugars, antioxidents, stabalizers, or antigenic powders.

As used herein, "polypeptide" refers to a series of amino acid residues linked through peptide bonds between the ∝-carboxyl carbon of one amino acid residue and the ∝-nitrogen of the next. A "polypeptide" as used herein can refer to a whole protein, a fragment of a protein, or an immunogenic epitope of a protein.

As used herein, "zona pellucida glycoprotein" refers to a group of three sulfated glycoproteins which comprise the mammalian zona pellucida. The zona pellucida further comprises a layer consisting of microvilli of the oocyte, and cellular processes of follicular cells. One "zona pellucida glycoprotein" in particular, ZP3, functions as the primary binding site for mammalian sperm. The zona pellucida, and, in particular the zona pellucida glycoprotein ZP3 may be identified using ZP3 specific antibodies and standard immunohistochemical techniques well described in the art (East et al., 1985, Dev. Biol. 109: 268). The nomenclature used in the art to describe ZP glycoproteins is variable. Example of ZP glycoproteins include, but are not limited to, ZP proteins isolated from pig: PZI, PZIII, 90K, 65K, 55K, 25K, ZP1, ZP2, ZP3 (PPZA), ZP4, 87K (ZP1/ZP2), 58K; ZP proteins isolated from rabbit: RZI, RZII, RZIII, ZP1, ZP2, ZP3, RZII; ZP proteins isolated from mouse: ZP1, ZP2, ZP3; ZP proteins isolated from cat: CZI and CZII; ZP proteins isolated from dog: DZI, DZII, and DZIII; ZP proteins isolated from squirrel monkey: ZP1, ZP2, ZP3, and ZP4; ZP proteins isolated from human: ZP1, ZP2, and ZP3. In addition to the ZP peptides described above, the nucleotide sequence encoding ZP glycoproteins of a number of species (e.g., mouse, human, hamster, rabbit) have been elucidated and are thus, useful in the present invention as they encode contraceptive proteins. The present invention uses the designation of ZP1, 2, or 3 for the three ZP glycoproteins. However, others in the art have grouped the ZP glycoproteins into ZPA, B, and C wherein ZPA includes those peptides described as ZP1, and ZP2, ZPB includes those peptides described as ZP3∝ and rc55, and ZPC includes those peptides described as ZP3β and ZP3 (see, for example U.S. Pat. No. 5,989,550).

As used herein, "immunocontraception" refers to temporary, reversible contraception, and also to permanent non-reversible contraception, or sterilization, resulting from an immune response to a contraceptive protein. As used herein, "immunocontraception" is deemed to be "effective" if the average number of offspring per breeding cycle produced by an animal subjected to "immunocontraception" by at least 60%, 70%, 80%, 90% and even up to 100% for animal species which produce an average litter size of three or more offspring. For animal species which produce an average litter of less than 3 offspring, "effective" immunocontraception would result in a reduction in litter size by at least 50%, and up to 100%.

As used herein, "immune response" refers to a response made by the immune system of an organism to a substance, which includes but is not limited to foreign or self proteins. There are three general types of "immune response" including, but not limited to mucosal, humoral and cellular "immune responses." A "mucosal immune response" results from the production of secretory IgA (sIgA) antibodies in secretions that bathe all mucosal surfaces of the respiratory tract, gastrointestinal tract and the genitourinary tract and in secretions from all secretory glands (McGhee, J. R. et al., 1983, Annals NY Acad. Sci. 409). These sIgA antibodies act to prevent colonization of pathogens on a mucosal surface (Williams, R. C. et al., Science 177, 697 (1972); McNabb, P. C. et al., Ann. Rev. Microbiol. 35, 477 (1981)) and thus act as a first line of defense to prevent colonization or invasion through a mucosal surface. The production of sIgA can be stimulated either by local immunization of the secretory gland or tissue or by presentation of an antigen to either the gut-associated lymphoid tissue (GALT or Peyer's patches) or the bronchial-associated lymphoid tissue (BALT; Cebra, J. J. et al., Cold Spring Harbor Symp. Quant. Biol. 41, 210 (1976); Bienenstock, J. M., Adv. Exp. Med. Biol. 107, 53 (1978); Weisz-Carrington, P. et al., J. Immunol 123, 1705 (1979); McCaughan, G. et al., Internal Rev. Physiol 28, 131 (1983)). Membranous microfold cells, otherwise known as M cells, cover the surface of the GALT and BALT and may be associated with other secretory mucosal surfaces. M cells act to sample antigens from the luminal space adjacent to the mucosal surface and transfer such antigens to antigen-presenting cells (dendritic cells and macrophages), which in turn present the antigen to a T lymphocyte (in the case of T-dependent antigens), which process the antigen for presentation to a committed B cell. B cells are then stimulated to proliferate, migrate and ultimately be transformed into an antibody-secreting plasma cell producing IgA against the presented antigen. When the antigen is taken up by M cells overlying the GALT and BALT, a generalized mucosal immunity results with sIgA against the antigen being produced by all secretory tissues in the body (Cebra et al., supra; Bienenstock et al., supra; Weinz-Carrington et al., supra; McCaughan et al., supra). Oral immunization is therefore an important route to stimulate a generalized mucosal immune response and, in addition, leads to local stimulation of a secretory immune response in the oral cavity and in the gastrointestinal tract.

An "immune response" may be measured using a technique known to those of skill in the art. For example, serum, blood or other secretions may be obtained from an organism for which an "immune response" is suspected to be present, and assayed for the presence of a of the above mentioned immunoglobulins using an enzyme-linked immuno-absorbant assay (ELISA; U.S. Pat. No. 5,951,988; Ausubel et al., Short Protocols in Molecular Biology 3rd Ed. John Wiley & Sons, Inc. 1995). According to the present invention, a contraceptive protein of the present invention can be said to stimulate an "immune response" if the quantitative measure of immunoglobulins in an animal treated with a contraceptive protein detected by ELISA is statistically different from the measure of immunoglobulins detected in an animal not treated with a contraceptive protein, wherein said immunoglobulins are specific for the contraceptive protein. A statistical test known in the art may be used to determine the difference in measured immunoglobulin levels including, but not limited to ANOVA, Student's T-test, and the like, wherein the P value is at least <0.1, <0.05, <0.01, <0.005, <0.001, and even <0.0001.

An "immune response" may be measured using other techniques such as immunohistochemistry using labeled antibodies which are specific for portions of the immunoglobulins raised during the "immune response". Tissue (e.g., ovarian tissue) from an animal to which a contraceptive protein has been administered according to the invention may be obtained and processed for immunohistochemistry using techniques well known in the art (Ausubel et al., Short Protocols in Molecular Biology 3rd Ed. John Wiley & Sons, Inc. 1995). Microscopic data obtained by immunohistochemistry may be quantitated by scanning the immunohistochemically stained tissue sample and quantitating the level of staining using a computer software program known to those of skill in the art including, but not limited to NIH Image (National Institutes of Health, Bethesda, MD). According to the present invention, a contraceptive protein of the present invention can be said to stimulate an "immune response" if the quantitative measure of immunohistochemical staining in an animal treated with a contraceptive protein is statistically different from the measure of immunohistochemical staining detected in an animal not treated with a contraceptive protein, wherein said histochemical staining requires binding specific for that contraceptive protein. A statistical test known in the art may be used to determine the difference in measured immunohistochemical staining levels including, but not limited to ANOVA, Student's T-test, and the like, wherein the P value is at least <0.1, <0.05, <0.01, <0.005, <0.001, and even <0.0001.

A "mucosal immune response" may be "detected" using a of the above referenced techniques. For example, an ELISA assay may be employed using anti-IgA antibodies to detect and measure the mucosal-specific immunoglobulins (Dickinson, B.L. & Clements, J.D. Dissociation of Escherichia coli heat-labile enterotoxin adjuvanticity from ADP-ribosyltransferase activity. Infect Immun 63, 1617-1623 (1995)).

As used herein, an "adjuvant" is a compound which when administered in conjunction with an antigen enhances the immune response to the antigen [(Coligan LG et al. eds. Current Protocols in Immunology, New York: John Wiley & Sons, 1995)]. An adjuvant can be effective by retarding the destruction of an antigen and allow the persistence of low but effective levels of antigen in the tissues or by nonspecifically activating the immune system by provoking an inflammatory response or other immune reaction which provides a heightened immune sensitivity.

As used herein, "species-specific" refers to a nucleic acid or amino acid sequence which is unique to the species from which it is derived. For example a "species-specific" nucleic acid sequence encoding a zona pellucida glycoprotein refers to a nucleic acid sequence that shares no greater than at least between 95-70% sequence identity with a nucleic acid sequence encoding a corresponding zona pellucida glycoprotein from a different species, no greater than between 80-50% sequence identity, and no greater than between 60-40% sequence identity. The "species-specificity" of a particular nucleic acid or amino acid sequence may be determined using a technique known to those of skill in the art including BLAST analysis.

As used herein, a "mucosal targeting protein" refers to a polypeptide which, when conjugated to, or expressed as a fusion protein with a second peptide, increases the potential for the combination of peptides to elicit a mucosal immune response. A "mucosal targeting protein" may be a polypeptide which is not endogenous to the organism to which it is introduced. Evidence (Black et al., Infect. Immunol. 55:1116 (1987)) indicates that "mucosal targeting proteins" when administered orally with an antigen (e.g., ZP glycoprotein) serves as an adjuvant to enhance the protective immune response. It therefore follows, that a "mucosal targeting protein" is a protein having ganglioside binding affinity for the mucosal epithelium when administered at least 10 µg doses, 1 µg doses, 500 ng doses, and even 1 ng doses, thus causing translocation of the protein across the mucosal epithelial membrane, such that "mucosal targeting proteins" may be important in eliciting a secretory immune response. It can be anticipated then that the product of a gene fusion of a contraceptive protein and a "mucosal targeting protein" will be more readily transported into cells of the mucosa and lead to enhanced local secretory immune responses. Examples of "mucosal targeting proteins" include, but are not limited to *E. coli* enterotoxin subunit B and A, Cholera toxin, shigatoxin B (StxB), staphylococcus enterotoxin B (SEB), Norwalk virus capsid protein (NVCP), and hepatitis B surface antigen. The ability of a polypeptide to target the immunogenicity of a second peptide to the mucosa may be examined by measuring the "mucosal immune response" (i.e., measuring the level of sIgA), for example, by ELISA or immunohistochemistry, which is stimulated by the administration of the combination of peptides and determining whether it is significantly increased compared to that observed when the second peptide is administered without the "mucosal targeting protein" using the statistical techniques described above for measuring an immune response.

As used herein, "animal" refers to a organism classified within the phylogenetic kingdom Animalia. As used herein, an "animal" also refers to a mammal. Animals, useful in the present invention, include, but are not limited to mammals, marsupials, mice, dogs, cats, cows, humans, deer, horses, sheep, livestock, poultry, chickens, turkeys, ostrich, fish, fin fish, shell fish, and the like.

As used herein, "monocotyledonous" refers to a type of plant whose embryos have one cotyledon or seed leaf. Examples of "monocots" include, but are not limited to lilies; grasses; corn; grains, including oats, wheat and barley; orchids; irises; onions and palms.

As used herein, "dicotyledonous" refers to a type of plant whose embryos have two seed halves or cotyledons. Examples of "dicots" include, but are not limited to tobacco; tomato; the legumes including alfalfa; oaks; maples; roses; mints; squashes; daisies; walnuts; cacti; violets and buttercups.

As used herein, "nucleic acid vector" refers to a nucleic acid molecule capable of transporting another nucleic acid to which it has been linked. One type of vector is a "plasmid", which refers to a circular double stranded nucleic acid loop into which additional nucleic acid segments can be ligated. Certain vectors are capable of autonomous replication in a host cell into which they are introduced (e.g., bacterial vectors having a bacterial origin of replication and episomal mammalian vectors). Other vectors (e.g., non-episomal mammalian vectors) are integrated into the genome of a host cell upon introduction into the host cell, and thereby are replicated along with the host genome. Moreover, certain vectors are capable of directing the expression of genes to which they are operatively linked. Such vectors are referred to herein as "expression vectors". In general, expression vectors of utility in recombinant nucleic acid techniques are often in the form of plasmids. In the present specification, "plasmid" and "vector" can be used interchangeably as the plasmid is the most commonly used form of vector.

As used herein, "promoter" refers to a sequence of DNA, usually upstream (5') of the coding region of a structural gene, which controls the expression of the coding region by providing recognition and binding sites for RNA polymerase and other factors which may be required for initiation of transcription. The selection of the promoter will depend upon the nucleic acid sequence of interest. A "plant-functional promoter" refers to a "promoter" which is capable of supporting the initiation of transcription in plant cells. "Plant-functional promoters" useful in the present invention include, but are not limited to the 35S promoter of the cauliflower mosaic virus (CaMV); promoters of seed storage protein genes such as Zma10Kz or Zmag12, light inducible genes such as ribulose bisphosphate carboxylase small subunit (rbcS), stress induced genes such as alcohol dehydrogenase (Adh1), or "housekeeping genes" that express in all cells (such as Zmact, a maize actin gene); the tomato E8 promoter; ubiquitin; mannopine synthesase (*mas*); rice *actin 1*; soybean seed protein glycinin (Gy1); soybean vegtative storage protein (*vsp*); and granule-bound starch synthase (*gbss*). Other "plant-functional promoters" include promoters for genes which are known to give high expression in edible plant parts, such as the patatin gene promoter from potato.

As used herein, "operably linked" refers to a juxtaposition wherein the components described are in a relationship permitting them to function in their intended manner. A control sequence "operably linked" to a coding sequence is ligated in such a way that expression of the coding sequence is achieved under conditions compatible with the control sequences. A promoter sequence is "operably-linked" to a gene when it is in sufficient proximity to the transcription start site of a gene to regulate transcription of the gene.

As used herein, "administered" refers to the delivery of the transgenic plant material, cells, compositions, and pharmaceutical formulations of the present invention to an animal in such a manner so to guarantee that the "delivered" material contacts a mucosal surface of the animal to which it was administered. Routes of "delivery" useful in the present invention include, but are not limited to oral delivery, nasal delivery, rectal or vaginal delivery (e.g., by suppository, or topical administration), or a route of delivery wherein the delivered material directly contacts a mucosal surface (i.e., "mucosal delivery"). As used herein, "pharmaceutically acceptable" means a non-toxic material that does not interfere with the effectiveness of the biological activity of the active ingredient(s). The characteristics of the carrier will depend on the route of administration.

As used herein, a "mucosal surface", "mucosal membrane", or "mucosa" refers to the well known medical definition of these structures, which is the surface or lining of a structure comprising an epithelium, lamina propria, and, in the digestive tract, a layer of smooth muscle. Examples of "mucosal surfaces" include, but are not limited to the inner coat of the bronchi, the mucous layer of the tympanic cavity, the inner mucous coat of the colon, the inner layer of the ductus deferens, the inner coat of the esophagus, the mucous coat of the small intestine, the mucous coat of the larynx, the mucous membrane of the tongue, the pituitary membrane, the mucous membrane of the oral cavity, the mucous membrane of the pharynx, the inner mucous layer of the trachea, the lining of the auditory tube, the mucous layer of the uterine tube, the inner layer of the ureter, the inner layer of the urethra, the endometrium, the mucous membrane of the vagina, the mucous layer of the stomach, the inner coat of the urinary bladder, and the mucous membrane of the seminal vesicle.

As used herein, "species" refers to a biological division between a genus of organisms and the individual organisms therein, in which all organisms within the species share close resemblance in the essential features of their general morphology, body plan, development, etc.

As used herein, "stable" refers to plant material that remains preserved at room temperature. Herein, "stable transgenic plant material" refers to plant material wherein the "heterologous" protein or "transgenic protein" remains potent without significant loss of immuno-reactivity, wherein immuno-reactivity is measured by an assay such as, Elisa, quantitative western blot, or agglutination. A "heterologous" protein is stable when at least 40% of the immuno-reactivity observed at the time of production, according to the inventive process, remains present for at least 5 months, and for longer periods, up to 10 months, or 12 months, or 18 months, or even longer.

### DESCRIPTION OF THE DRAWINGS

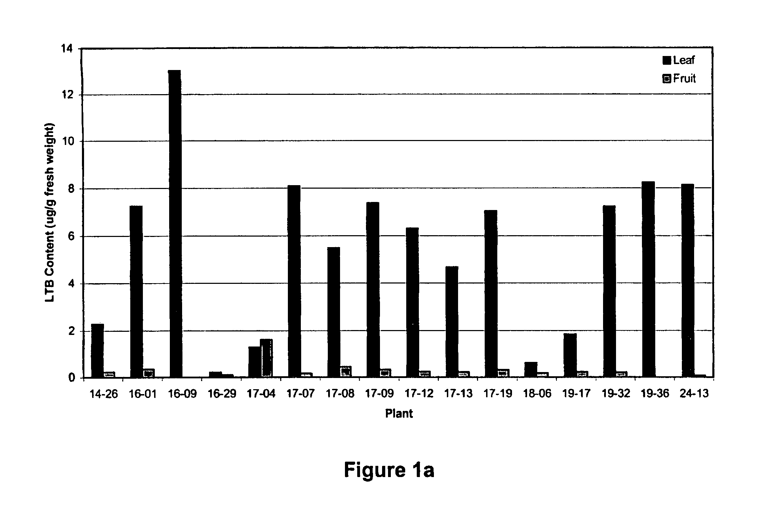

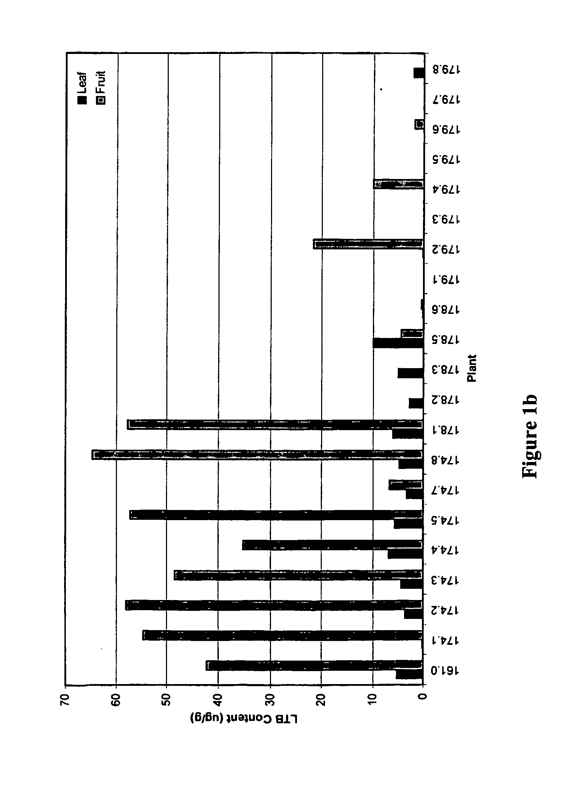

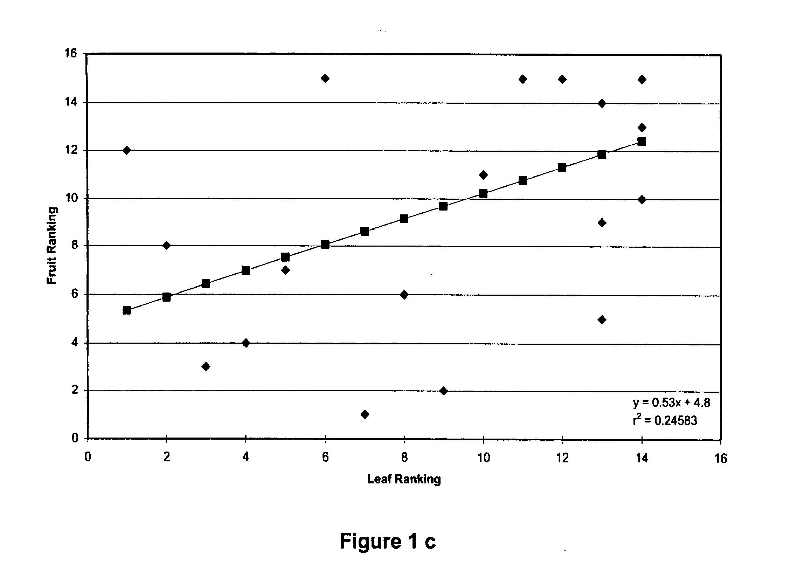

Figure 1 a LTB content of leaf and fruit samples from To plants as determined by Elisa.
Figure 1 b LTB content of leaf and fruit samples from T₁ plants as determinerd by Elisa.
Figure 1 c Comparison of leaf and fruit rankings with respect to LTB content as determinerd by Elisa.
Figure 2 a The average end point for dilutions of anti-LTB antibodies in tested mice. The bars represent the average of the serum dilution when OD₄₅₀ reading is equal to 0.1 of the six animals within the same treatment. Treatments include mice that were fed non-transgenic tomato paste (control), mice that were fed transgenic tomato paste (Test) and mice that were fed transgenic tomato paste mixed with 10 mg of Quillaja extract powder (Garuda). Error bars represent the standard error.

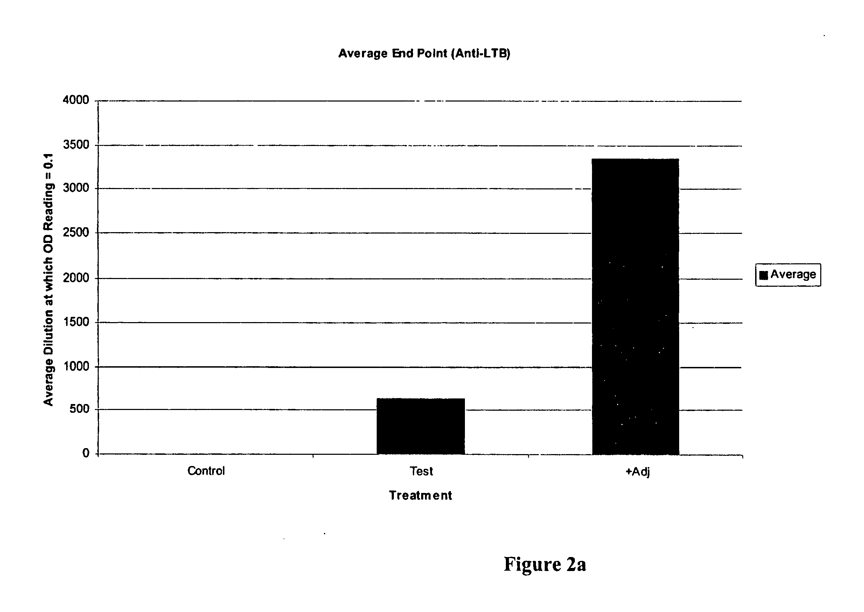

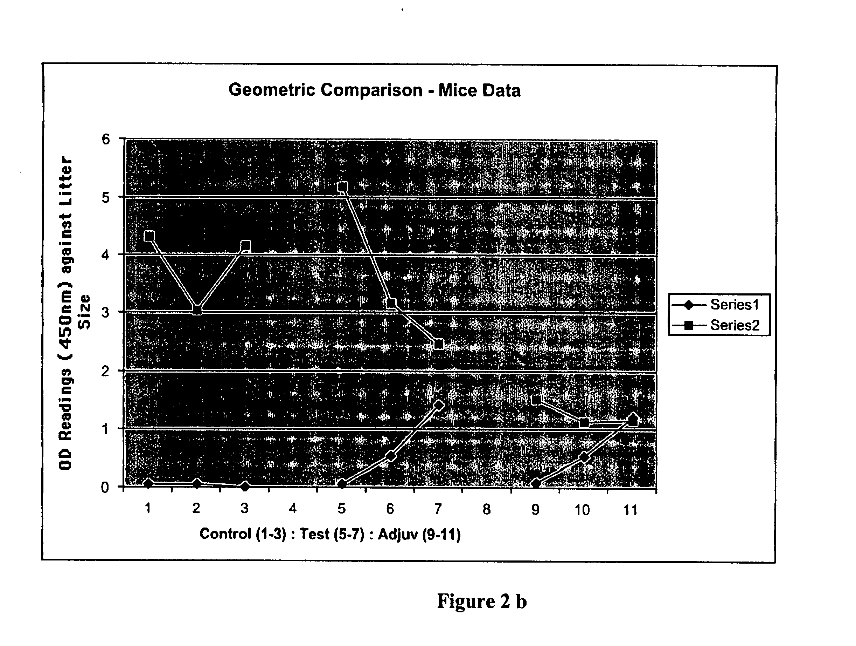

Figure 2 b This figure shows the relationship between increasing anti-LTB titers (Series 1), and decreasing fecundity (series 2), after oral administration of raw tomato material expressing a fusion protein consisting of LT-B fused to murine ZP3 epitope. The third set of data, represents the experimental group who received an additional adjuvant mixed into the tomato material.

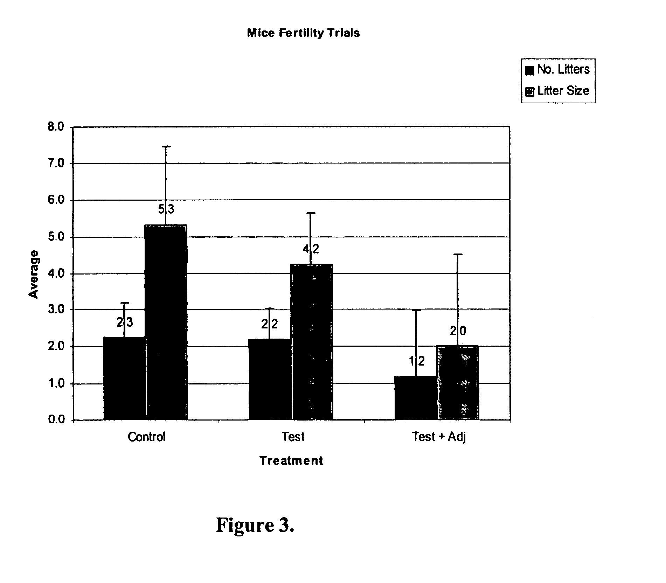

Figure 3 The effect of oral delivery of tested materials on the fertility of mice. Each bar represents the average of the six animals in a particular treatment where the control represents results from animals fed non-transgenic tomato paste, test represents results from animals fed transgenic tomato paste and test + adj represents results from animals fed transgenic tomato paste mixed with 10 mg adjuvant powder (Garuda). Error bars represent the standard error.

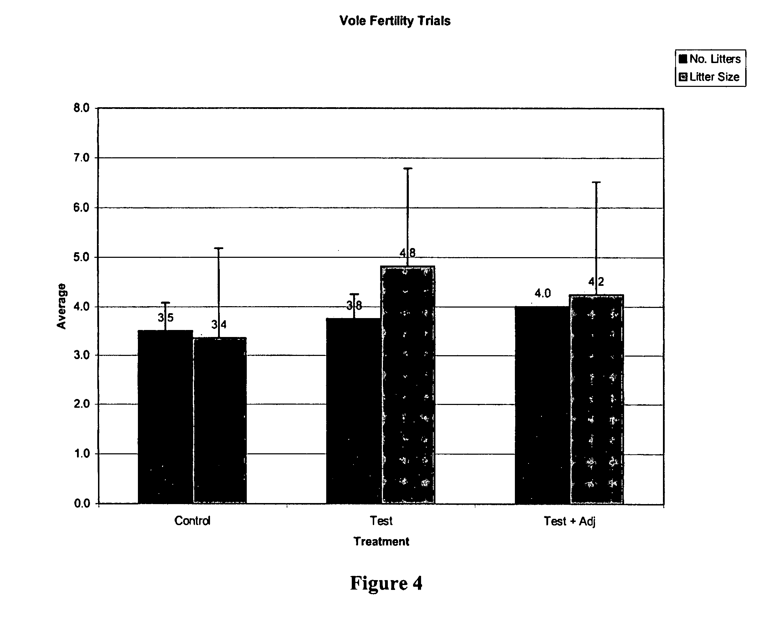

Figure 4 The effect of oral delivery of tested materials on the fertility of voles. Each bar represents the average of the six animals in a particular treatment where the control represents results from animals fed non-transgenic tomato paste, test represents results from animals fed transgenic tomato paste and test + adj represents results from animals fed transgenic tomato paste mixed with 10 mg of Quillaja extract powder (Garuda). Error bars represent the standard error.

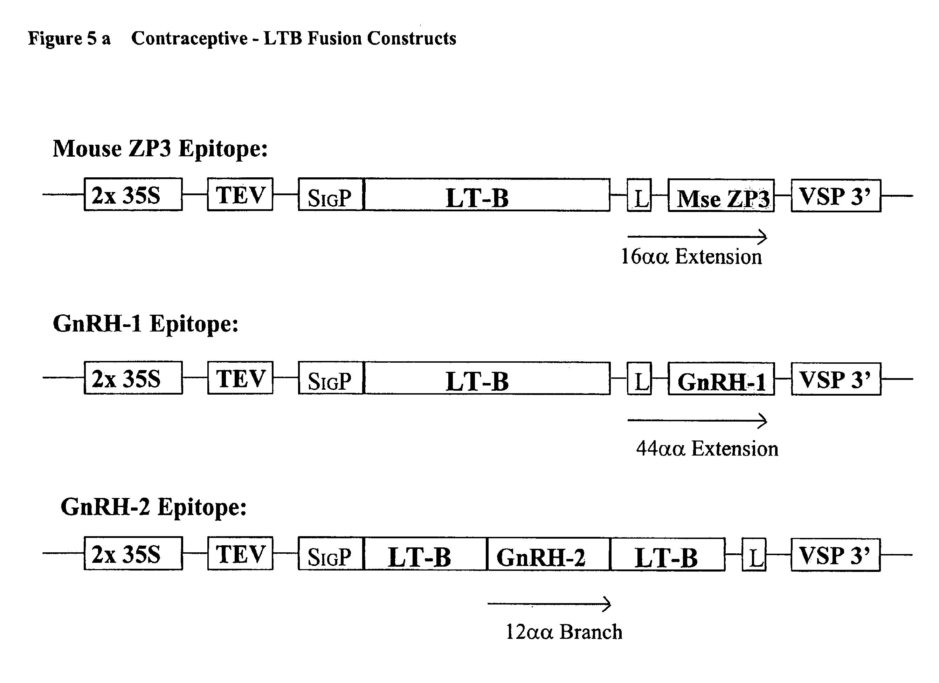

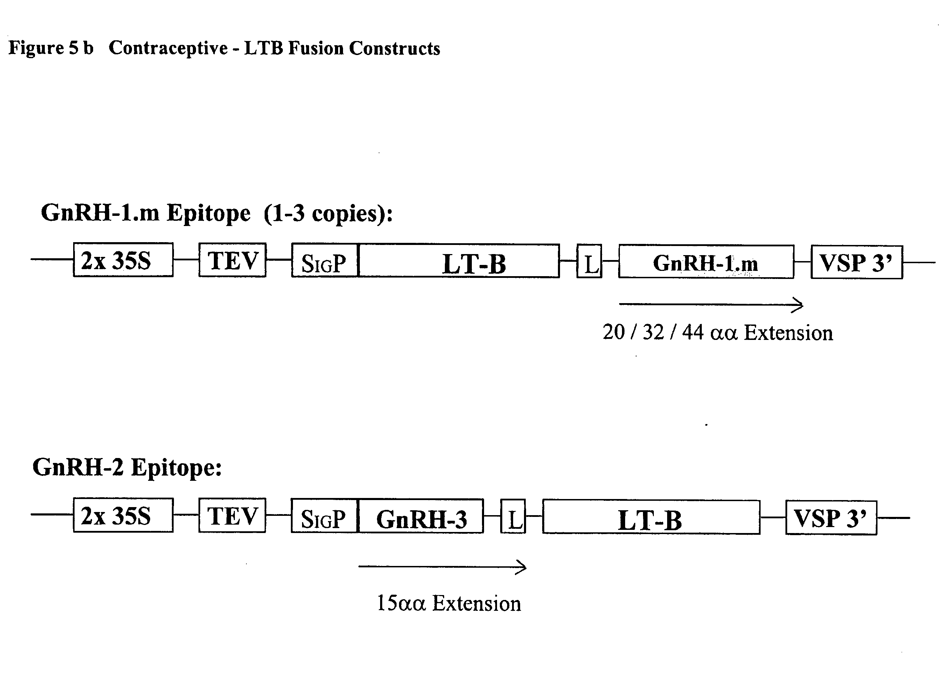

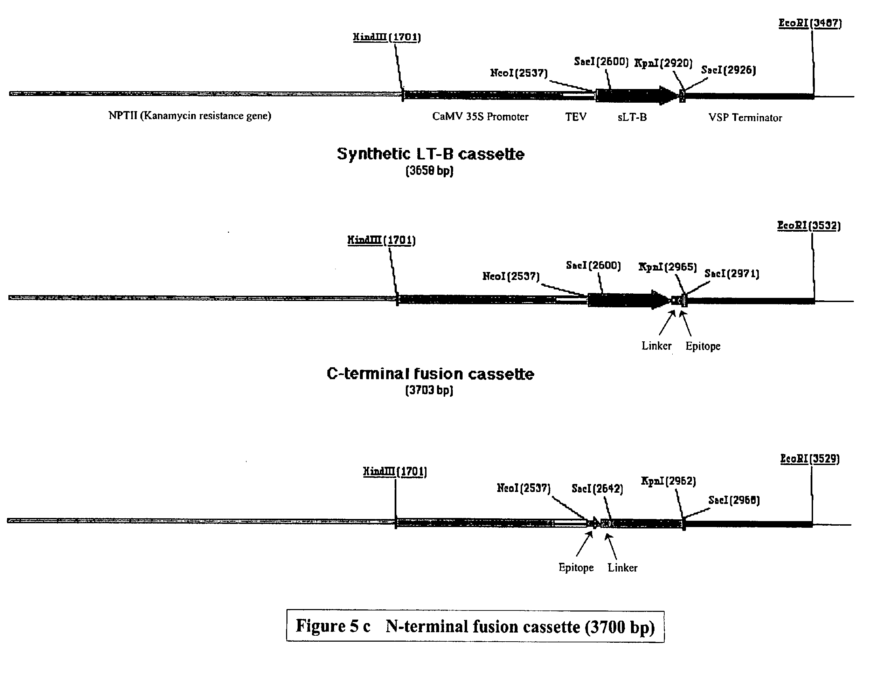

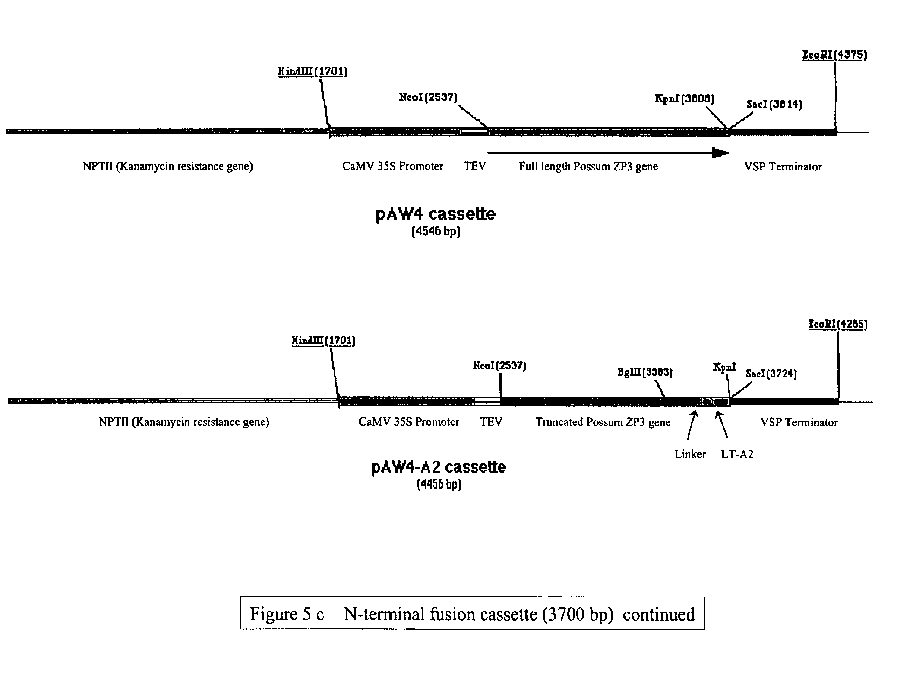

Figure 5 a LTB fusion constructs.
Figure 5 b Additional LTB fusion constructs.
Figure 5 c N-terminal fusion cassette.

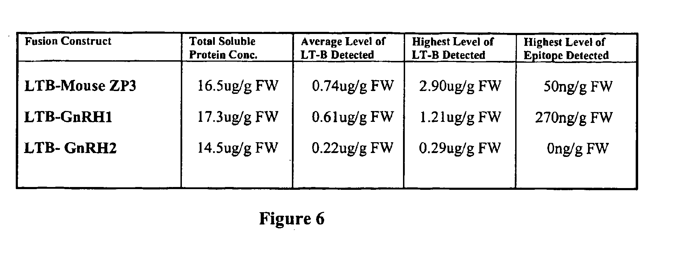

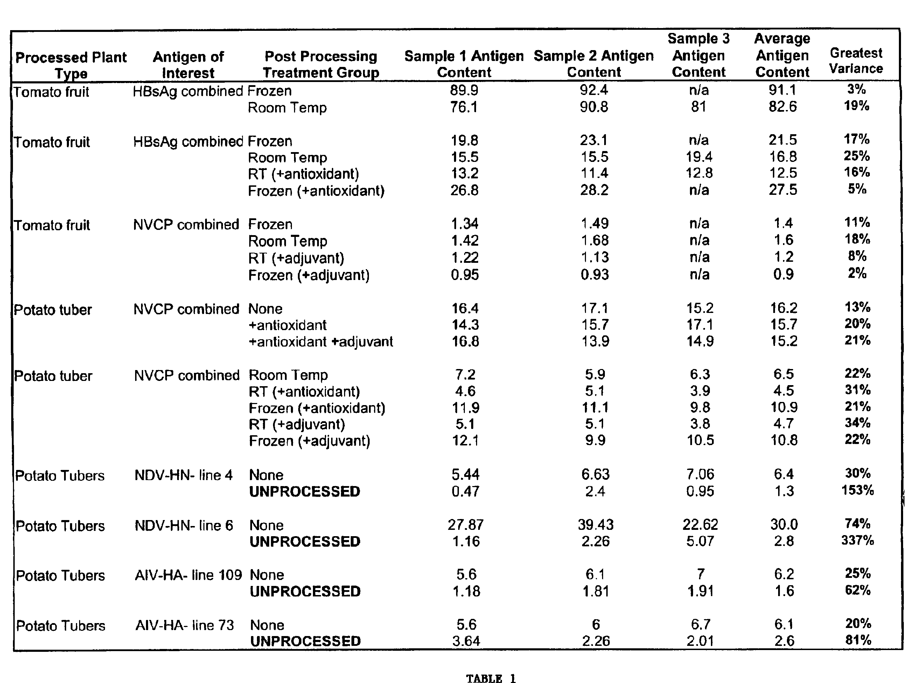

Figure 6 This figure illustrates average and "elite line" expression data for three immunocontraceptive fusions expressed in transgenic tobacco (NT-1) cell suspension cultures.
Epitope detection was based upon a sandwich ELISA, where primary capture was specific for LT-B, and secondary reaction was specific for the fused contraceptive epitope (either Mouse ZP3 epitope, or GnRH decapeptide according to the construct used). The data for LTB-GnRH2 suggests that the structure of the fusion protein interfered with the natural oligomerization, hence detection of fully formed pentamers was significantly reduced, as was the ability to detect the fused epitope by sandwhich ELISA.
Table 1. Improved homogeneity of processed materials. Antigen content of processed or unprocessed transgenic plant material present in "batches" of plants pooled together, or in unmixed samples from specific plant lines.

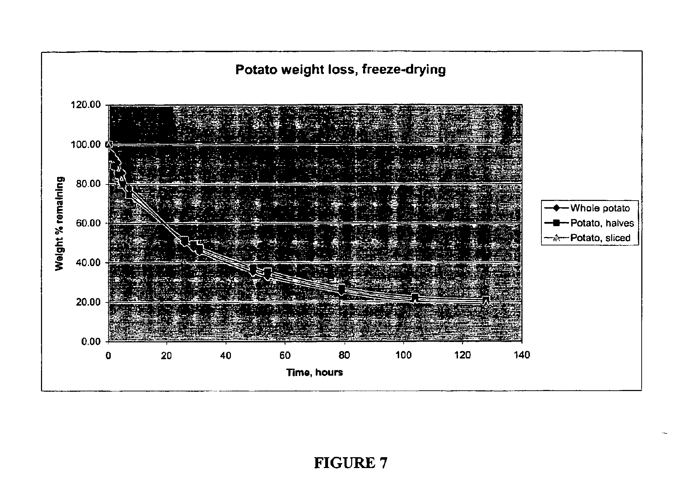

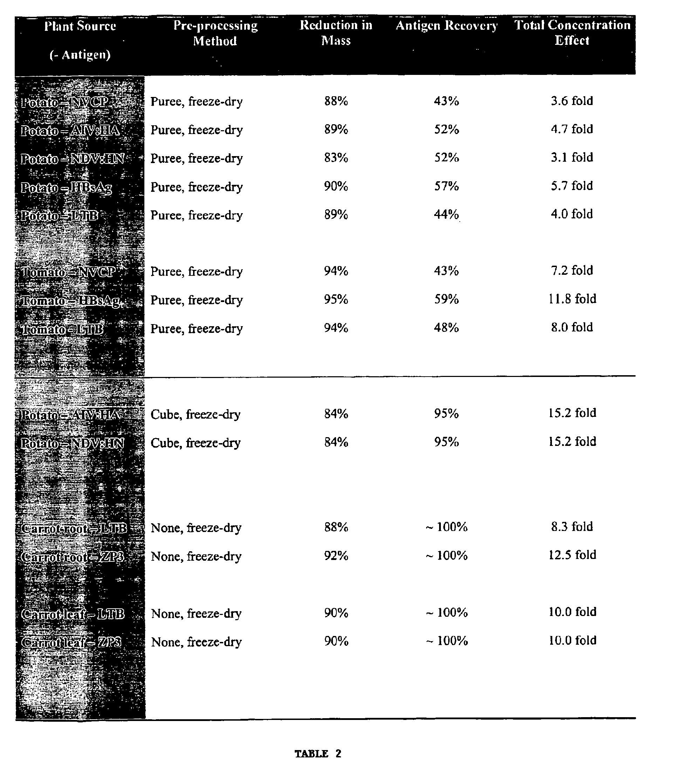

Figure 7 Graph showing effect of drying time by different incision methods
Table 2 Recovery of antigen in different tissues and concentration of antigen obtained by different methods. Processing of potato biopharmaceuticals by puree and freeze-drying allows concentration of detectable antigen by an average of 4.2 fold. When the same materials are processed by cubing or quartering followed by freeze-drying, the concentration of detectable antigen is increased to an average of 15.2 fold. Other materials such as transgenic tomato show a similar destructive effect on the antigen when pureeing is used as a method for material homogenization prior to freezing. When transgenic carrot materials are processed by freeze drying (no puree or slicing), the concentration of antigen comparible to raw material is directly proportional to the reduction in mass, further indicating conservation of the detectable antigen by this process.

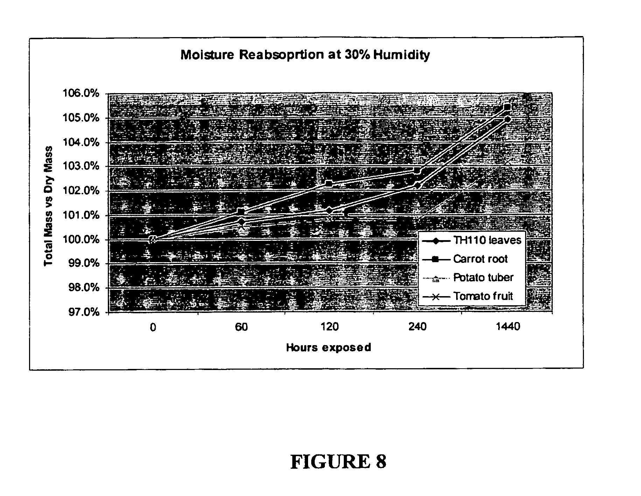

Figure 8 Graph showing reabsorbance of moisture over time in assorted materials.

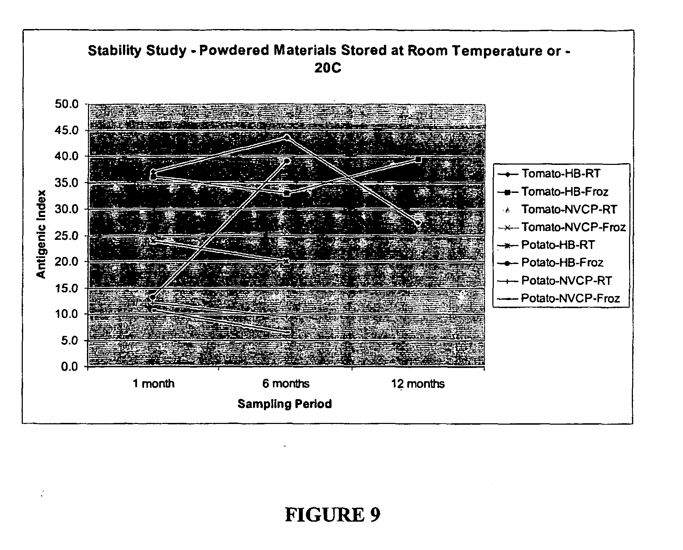

Figure 9 Graph showing antigen stability.

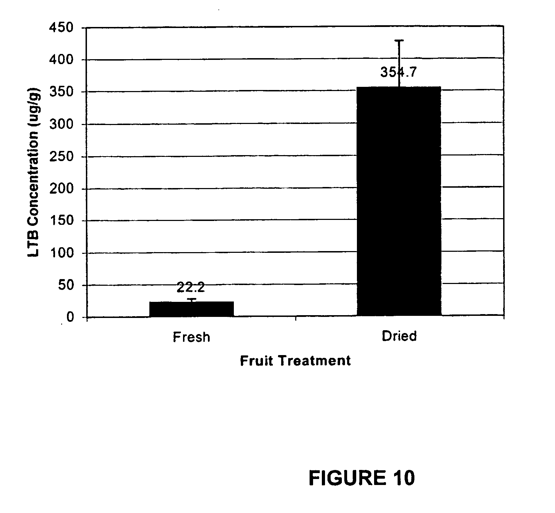

Figure 10 Effect of processing on LTB content within T2 fruit material. Solid bars represent the average LTB concentration of pooled fruit and the error bars represent the standard error of the mean.

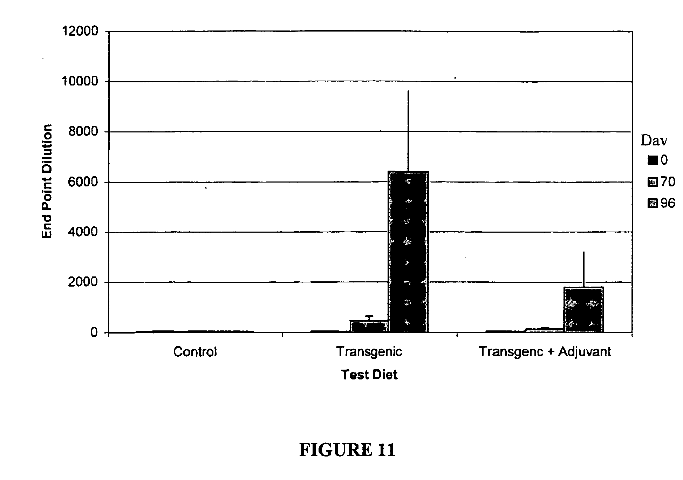

Figure 11 Anti-LTB end point dilutions of adult serum samples. Where the Control bar represents the average end point serum dilution of mice fed the wild type tomato powder, the Transgenic bar represents the average end point serum dilution of mice fed the transgenic tomato powder and the Transgenic + Adjuvant bar represents the average end point serum dilution of mice fed the transgenic tomato powder with Quillaja extract powder. The end point dilution was determined to be the first dilution of serum that received an OD 450nm reading of 0.1. Control n = 3, Transgenic n = 4 and Transgenic + Adj n = 2. Error bars represent the standard error of the mean.

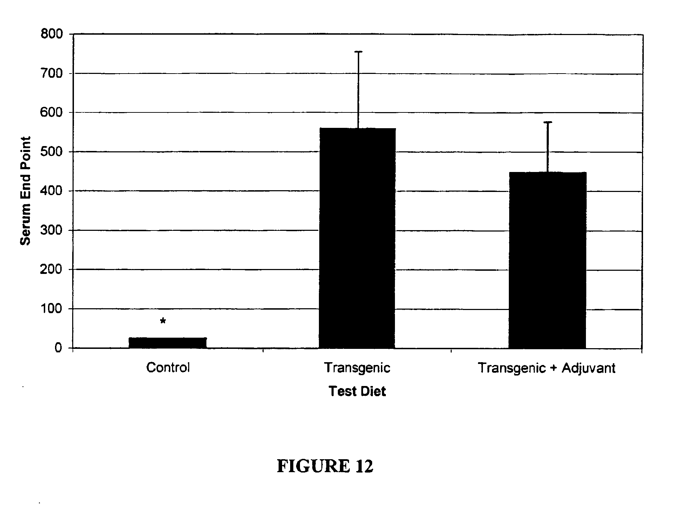

Figure 12 Anti-LTB end point dilutions of pup serum samples. Where the Control bar represents the average end point serum dilution of pups from mice fed the wild type tomato powder, the Transgenic bar represents the average end point serum dilution of pups from mice fed the transgenic tomato powder and the Transgenic + Adjuvant bar represents the average end point serum dilution of pups from mice fed the transgenic tomato powder with Quillaja extract powder. The end point dilution was determined to be the first dilution of serum that received an OD 450nm reading of 0.1. Control n = 18, Transgenic n = 17 and Transgenic + Adjuvent n = 13. *Contains serum samples that received an OD of less than 0.1 with the minimum dilution used (1 in 25). Error bars represent the standard error of the mean.

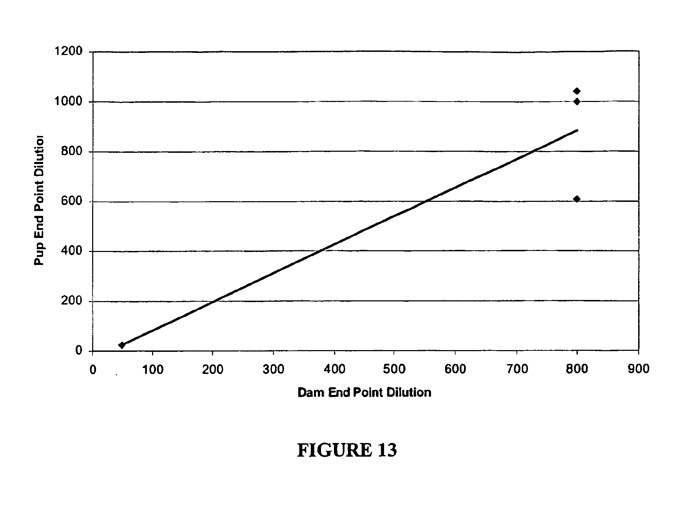

Figure 13 Relationship between dam and offspring titers. The dam end point dilution is the end point of the serum sample taken closest to the birth of the corresponding litter and the pup end point dilution represents the average end point of a litter. The end point dilution was determined to be the first dilution of serum that received an OD 450 nm reading of 0.1. Dam n = 7, litter n = 9 and pup n = 41.

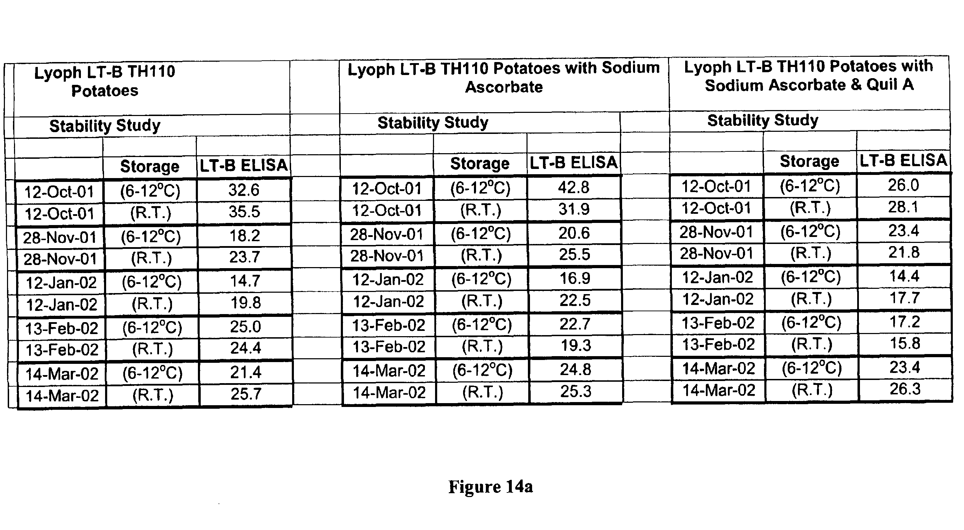

Figure 14a Stability study of Lyophilized LT-B Potato chart.

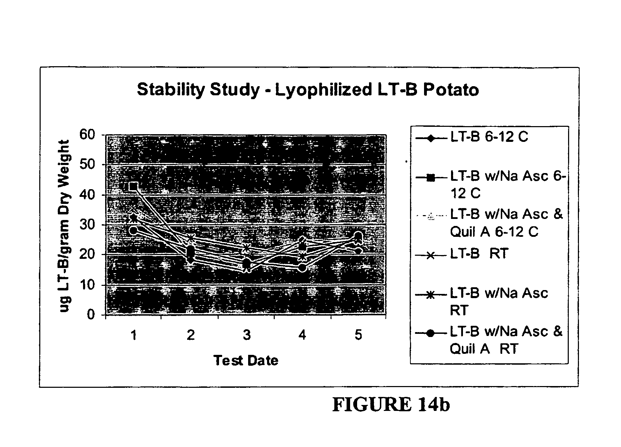

Figure 14b Stability study of Lyophilized LT-B Potato graph.

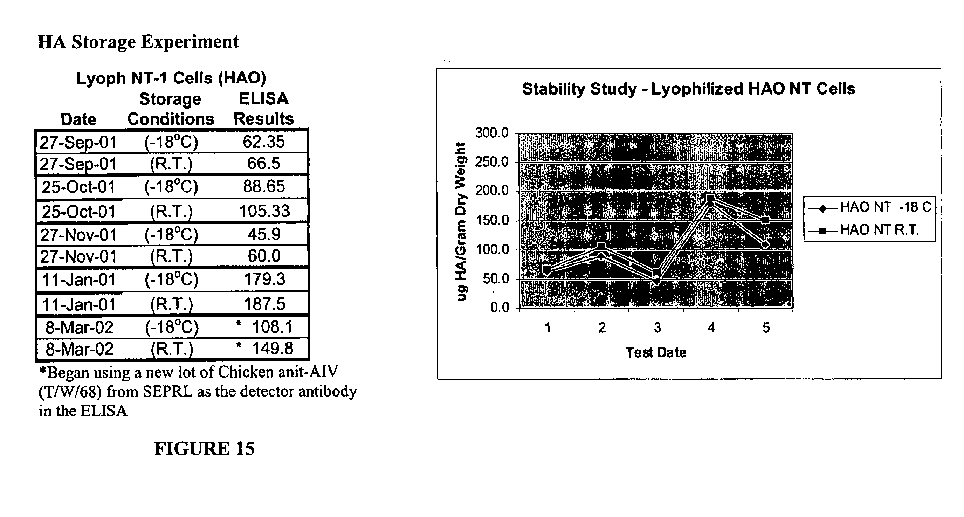

Figure 15 Stability study of Lyophilized HAO NT cells.

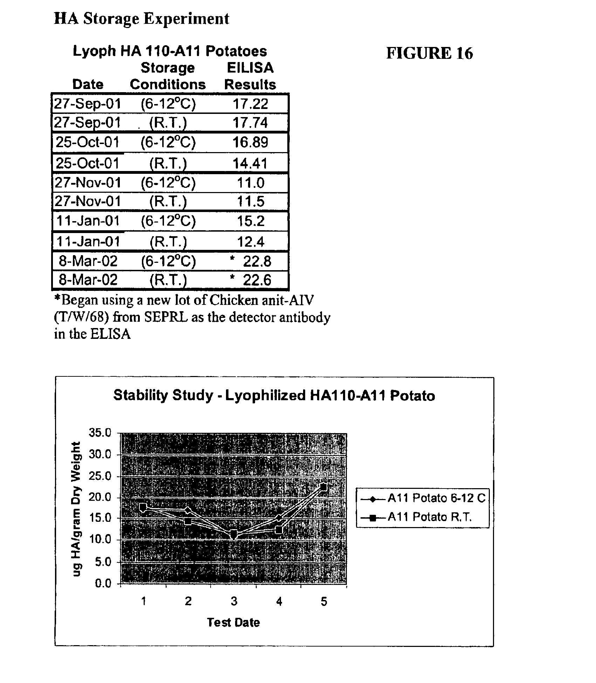

Figure 16 Stability study of Lyophilized HA110-A11 Potato.

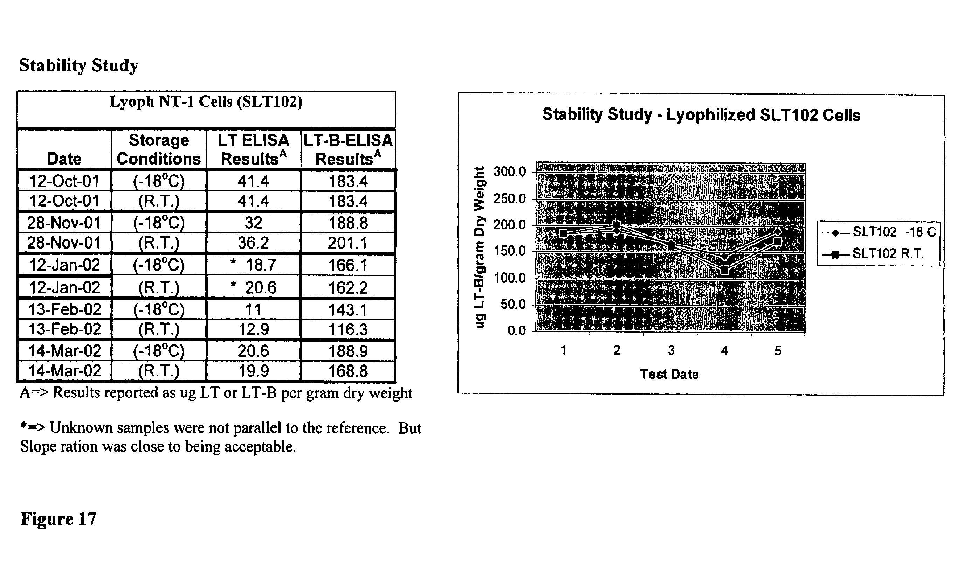

Figure 17 Stability study of Lyophilized SLT 102 cells.

### DETAILED DESCRIPTION

The present invention provides methods for the production of transgenic plant biopharmaceuticals. The methods described herein allow for the production of dry transgenic plant material that is stable at room temperature, homogeneous, and pharmaceutically potent. In general, an intact or partitioned transgenic plant that produces a biopharmaceutical is dried or freeze/dried through food processing techniques known in the art. The dry homogenate product can then be used therapeutically without the need to further extract, purify, or precipitate the pharmaceutical protein.

The resultant transgenic plant materials comprise formulated powders that can be easily handled, and that are stable at ambient temperatures when stored up to 12 months. Furthermore, batch processing described herein significantly increase the consistency in detectable antigen between plants or plant tissues, and concentrate the antigen for improved potency.

### Transgenic plant species

Plants that can be used for the present invention include both dicotyledon and monocotyledon types. These include, but are not limited to, carrot, spinach, pepper, potato, tomato, apple, wheat, rye, soybean, rice, maize, corn, berries such as strawberries, raspberries, alfalfa and banana. Since edible plants used by humans for food or as components of animal feed are dicotyledenous plants, dicotyledons are typically employed, although monocotyledon plants are useful for animal feed. Cells and seeds derived from these plant vaccines are also useful according to the invention.

The transgenic plant can be produced by any means known in the art. In one embodiment, a range of transgenic plant species including tomato, potato and carrot are grown under biosafety level 1 greenhouse containment conditions without any pesticide or herbicide applications, and allowed to grow to maturity.

The transgenic plants useful in this invention express heterologous proteins. Examples of suitable heterologous proteins include, but are not limited to, Norwalk virus capsid protein (NVCP), Avian Influenza hemagglutination antigen (AIV-HA), Newcastle Disease Virus huraminidase (NDV-HN), zona pellucida glycoprotein 3 (ZP3), and Hepatitis B surface Antigen (HBsAg), antibodies made in plants (e.g. "plantibodies"), and other therapeutic proteins (e.g. endostatin and angiostatin).

### Harvesting

The transgenic plant material can be harvested by hand or machine, or by any means known in the art. The harvesting method may vary per plant species. In one embodiment, transgenic potato or carrot, may be harvested as an entire batch, and stored at 4°C until processing. In another embodiment, transgenic tomato fruit deemed to be mid-ripe or ripe is harvested biweekly over a period of 6 weeks and processed on the day of harvest. In a further embodiment, the leaf or stem materials are removed tomato fruit, potato tubers, or carrot root by hand prior to washing tomato fruit, potato tubers, or carrot root in a 0.1 % chlorine solution (1% Clorox) to remove soil and bioburden. For purposes of record keeping, each batch is counted and weighed, and accompanied by Harvest and Processing Record sheets.

### Homogenation

Homogenation of transgenic plant material can be preformed either pre- or post-drying. In one embodiment, homogenation is performed prior to drying and involves the crude pulping and mixing of freshly harvested plant materials, followed by freezing and subsequent freeze-drying of the blended materials. The first process of slicing, blending or grinding, reduces raw plant tissues (fruit, tubers, leaf, seed, etc) to a pulp mixture. This mixture is assumed to contain both intact plant cells and ground cell debris. The advantage of this process is the reduction of larger plant organs, such as fruit or tubers, to a manageable mixture whereby variance in homologous protein content between tissues is combined into a more uniform batch of material. The ability to provide a uniformed batch without fully disrupting all plant cell integrity provides the stable environment for homologous protein storage during the subsequent processing stages. In another embodiment, homogenization requires minimal processing to the plant materials prior to processing. Homogination is performed after Freeze-drying of sliced, quartered or intact materials. The resultant materials are crushed to a fine powder, combined into a single batch container, and mixed thoroughly by mechanical means such as shaking or dry blending to obtain a homogenous mixture.

### Partitioning

For partitioning, whole plant or intact plant parts may be reduced in size by means known in the art. Means for partioning include, but are not limited to, cubing, quartering, mincing slicing, dicing, pureeing, pulping, milling, crushing, pressing, or cracking.

If pureeing is preferred, freshly harvested fruit, tubers or roots are pureed using a Stephan Industrial Vertical Mixer/Slicer for approximately 1 minute. It is preferred that a maximum volume of approximately 20 lbs of material be pureed at a single time. When pureeing potato, a quantity of water (up to 500 ml) may be added to assist in the pureeing process. It is a preferred that the temperature of the pureed materials does not exceed 2 °C. In one embodiment, the homogenate is poured off into clearly labeled freezer trays and stored at -40°C. To avoid loss of any genetically modified pulp or seed, the Mixer/Slicer may be wiped down with paper towel.

Materials may also be quartered, peeled, or sliced by any means known in the art.

### Dehydration

In one embodiment, the quartered, peeled, or sliced transgenic plant material is transferred to freezer trays and placed within an industrial lyophilizer and freeze-dried for 2-6 days, at a temperature of ⁻30 °C with a maximum shelf temperature of 25 °C. Resultant materials may then be combined into labeled containers and returned with completed Harvest and Processing Record sheets. In another embodiment, the transgenic plant material is sliced in a 1-gallon stainless steel Waring blender and samples are transferred to 500 ml or 1 liter flasks and freeze dried using a laboratory freeze dryer. In this case, materials were dried for 1-6 days at a temperature of ⁻30°C.

### Determination of Antigen Concentration

The analysis of plant materials to determine the concentration of antigens of interest can be achieved using enzyme linked immunosorbant assays (ELISA) or agglutination assays appropriate to the antigen contained in the plant tissues. The assay method chosen for a specific antigen should be applied consistently when evaluating both raw and processed materials.

### Stability and Excipient Use

To evaluate the stability of processed materials at ambient temperatures, samples of 10-50 grams of material were removed immediately following freeze drying, and placed in airtight bags and in secondary containers before storage on a laboratory shelf. Ambient temperature was maintained at 23°C ⁺/⁻ 2°C. The remainder of the processed materials were stored at either 4°C or at ⁻20°C. Samples were periodically taken from materials stored under each condition and antigen content compared by appropriate assay to determine the relative stability of materials stored at ambient temperature.

### Contraception

The present invention is also based on the discovery that transgenic plants are generated which express proteins capable of inducing contraception, and that administration of the transgenic plant material to an animal is sufficient to induce a mucosal immune response to the contraceptive protein such that effective contraception is achieved. The present invention provides a method for immunocontraception comprising administering to an animal a transgenic plant which expresses a contraceptive protein, wherein the administration of the transgenic plant induces a mucosal immune response in the animal to which it is administered. The present invention further provides a method for making a transgenic plant which expresses a contraceptive protein useful in the present invention for administration to an animal, and further provides a transgenic plant which, upon administration to an animal is capable of inducing contraception in that animal.

### Contraceptive Proteins

The present invention provides transgenic plant material, the cells of which, express a protein or polypeptide which is capable of inducing contraception in an animal to which the plant material is administered. Contraceptive proteins, useful in the present invention, include, but are not limited to zona pellucida glycoproteins, gonadotrophin releasing hormone (GnRH), leutinizing hormone releasing hormone (LHRH), Leutinizing hormone (LH), Lactate Dehydrogenase (LDH), and anti-sperm antigens.

### Zona Pellucida Glycoproteins

The zona pellucida is a tough, refractile, extracellular glycoprotein matrix enveloping the oocyte. Its characteristic striated appearance results from the numerous fine canals with which it is pierced. Cytoplasmic processes from the follicular cells extend through the ZP and occasionally invaginate the plasma membrane of the oocyte. The cytoplasmic processes of the follicular cells also transport nutritive material to the surface of the oocyte. In addition, microvilli of the oocyte extend into the ZP and increase the absorptive capacity at the surface of the oocyte. [See, generally, W. J. Hamilton, ed., Hamilton, Boyd and Mossman's Human Embryology, pp. 27-32 and 54-64 (1972); J. B. Warshaw, ed., The Biological Basis Of Reproduction And Developmental Medicine (1973).] Zone pellucida (ZP) glycoproteins, useful as contraceptive proteins in the present invention, include all forms of the glycoprotein matrix which are capable of eliciting antibodies to ZP, including ZP glycoproteins 1, 2, 3 and 4. These encompass ZP epitopes which appear late in an oocyte's development, during the antral and preovulatory phases, as well as epitopes which are expressed after ovulation.

In a preferred embodiment, the contraceptive protein of the present invention is the ZP3 glycoprotein, or an epitope thereof. An "epitope" of a contraceptive protein of the present invention is at least 6, 8, 10, 12, 14, 20, and up to 30 amino acids in length. As used herein, an "eptiope" of a contraceptive protein useful in the present invention is a portion of the contraceptive protein that can stimulate a mucosal immune response. The ability of an epitope of a contraceptive protein to stimulate a mucosal immune response may be determined by measuring the amount of secretory IgA present in an animal to which the contraceptive epitope has been administered using a technique known to those of skill in the art. For example, a measurement of the amount of secretory IgA present in an animal to which a contraceptive epitope has been administered can be measured by ELISA using anti-IgA antibodies and compared to sIgA levels in an animal to which the epitope has not been admininstered. A statistically significant difference between the sIgA levels in the two animals, measured using a statistical test known in the art, is indicative of the stimulation of a mucosal immune response by the epitope. The P value for statistical tests useful in the present invention is at least <0.1, <0.05, <0.01, <0.005, 0.001, 0.0005, and preferably <0.0001.

Despite the common morphological properties of the ZP of different species, the ZPs of different animal species have immunologically distinct regions or "epitopes" (Timmons et al., In: Perspectives in Immunoreproduction: Conception and Contraception (Mathur et al., eds.) Hemisphere Publ., pp. 242-260 (1988)). A number of ZP proteins have been isolated from distinct animal species, and are useful as contraceptive proteins in the present invention.

Zona pellucida proteins isolated from pig include: PZI, a 40-110 kD protein isolated by Dunbar et al., Biol. Reprod. 24:1111 (1981); PZII, a 70-110 kD protein, PZIII, a 95-118 kD protein, and PZIV, an 18-25 kD protein, all isolated by Dunbar et al. (Biol. Reprod. 32:619 (1985)); 90K, a 89-119 kD protein, 65K, a 61-83 kD protein, 55K, a 47-66 kD protein, and 25K, an 18-26 kD protein, all isolated by Hedrick, J. L. and Wardrip, N. J. (Biochem. 157: 63 (1986)); ZPA1, an 82-118 kD protein, ZP2, a 58-96 kD protein, ZP3 (PPZA), a 40-74 kD protein, and ZP4, a 21 kD protein, all isolated by Subramanian et al. (Biol. Reprod. 24:933 (1981)); 87K (ZP1/ZP2), a 77-97 kD protein, 58K, a 40-70 kD protein both isolated by Yurewicz et al. (Biol. Reprod. 29: 511 (1983)); deglycosylated PZI, a 35 kD protein; PZII, a 55 kD protein; and PZIII, an 80 kD protein all isolated by Skinner and Dunbar as described in Immunological Approaches to Contraception and the Promotion of Fertility, G. P. Talwar (ed.) New York: Plenum pp. 251-268 (1986); and deglycosylated ZP3 having a molecular weight of 45 kD isolated by Sacco et al. (J. Reprod. Fertil. 76:575 (1986)).

Isolated rabbit zona pellucida proteins include: RZI, RZII, and RZIII, having molecular weights of 68-125 kD, 80-100.5 kD, and 100-132 kD respectively, all isolated by Dunbar et al. (Biol. Reprod. 24:1111 (1986)); ZP1, ZP2, and ZP3 having molecular weights of 100-118 kD, 83-110 kD, and 80-92 kD respectively, all isolated by Sacco et al. (Proc. Soc. Exp. Biol. Med. 167:318 (1981)); deglycosylated RZI, and RZII having molecular weights of 65 kD, and 80 kD respectively, both isolated by Skinner and Dunbar and described in Immunological Approaches to Contraception and Promotion of Fertility. G. P. Talwar (ed.). New York: Plenum, pp. 251-268 (1986); and deglycosylated RZIII, a 90 kD protein isolated by Timmons and Dunbar (Biol. Reprod. 36: 1275 (1987)).

A number of mouse zona pellucida proteins have been isolated including: ZP I, ZP2, and ZP3 having molecular weights of 200 kD, 120 kD, and 83 kD respectively, all isolated by Bleil and Wassarman (Dev. Biol. 76:185 (1980)); and ZP1 and ZP2 having molecular weights of 166-122 kD and 90-92 kD respectively, isolated by Sacco et al. (Proc. Soc. Exp. Biol. Med. 167: 318 (1981)). The differences in the molecular weights of mouse ZP1 and ZP2 as reported by Bleil et al. and Sacco et al. may be due to the fact that Bleil used 2D-PAGE under non-reducing conditions while Sacco used 2D-PAGE under reducing conditions.

The cat zona pellucida proteins CZI and CZII were isolated by Maresh and Dunbar J. (Exp. Zool. 244:299 (1987)) and have molecular weights of 50-110 kD and 90-110 kD respectively.

Maresh and Dunbar (J. Exp. Zool. 244:299 (1987)), have also isolated the dog zona pellucida proteins DZI, DZII, and DZIII which have molecular weights of 50-110 kD, 70-95 kD, and 90-100 kD respectively.

Sacco et al. (Proc. Soc. Exp. Biol. Med. 167:318 (1981)) described squirrel monkey ZP1, ZP2, ZP3, and ZP4 having molecular weights of 63-78 kD, 63-70 kD, 47-51 kD, and 43-47 kD respectively. In the same publication Sacco et al. described human ZP1, ZP2, and ZP3 having molecular weights of 80-120 kD, 73 kD, and 59-65 kD respectively.

In addition to the ZP peptides described above, the nucleotide sequence encoding ZP glycoproteins of a number of species have been elucidated and are thus, useful in the present invention as they encode contraceptive proteins. For example, Ringuette et al. (Dev. Biol., (1988) 127:287-295) and Liang et al. (Mol. Cell. Biol., (1990) 10:1507-1515 ), reported cloning of mouse DNA encoding zona pellucida proteins ZP3 and ZP2, respectively. The clones were obtained by screening mouse cDNA libraries with anti-ZP3 and anti-ZP2 antibodies. No sequence homology was found between mouse ZP3 and ZP2.

Ringuette et al. (Proc. Natl. Acad. Sci. USA, (1986) 83:4341-4345), reported isolation of a partial cDNA clone for mouse ZP3, which clone hybridized with total genomic DNA of mouse, rat, dog, cow, and human, but not with pig or rabbit genomic DNA unless the hybridization was performed at very low stringency. The full length ZP3 cDNA characterized by Ringuette (Dev. Biol. (1988) 127:287-295) represents a germ-line specific mRNA having relatively short 5' and 3' untranslated regions and an open reading frame of about 1317 nucleotides with an additional 200-300 nucleotide poly-A tail. Ringuette also found that rat, rabbit, dog, and cow ovary transcribes mRNA which hybridized to the mouse ZP3 cDNA and that the ZP3 transcripts had similar molecular weights. Liang et al. (Mol. Cell. Biol., (1990) 10:1507-1515), showed that the nucleic acid and deduced amino acid sequence of ZP2 is distinctly different from that of ZP3 although it had the same short motif of 5' and 3' untranslated regions. The ZP2 mRNA is reported to have single open reading frame of 2,139 nucleotides which codes for a polypeptide of 80,217 Daltons representing 713 amino acids.

Chamberlin and Dean, (Dev. Biol. (1989) 131:207-214) and Kinloch, R. A. et al. (Proc. Nat. Acad. Sci. USA, (1988) 85:6409-6413) have reported the cloning of the mouse ZP3 gene. The mouse ZP3 gene is reported to have 8 exons and 7 introns in a transcription unit of 8.6 kbp.

Kinloch et al. (Dev. Biol. (1990) 142:414-421), reported cloning of hamster genomic ZP3 DNA from a hamster genomic DNA library screened with mouse ZP3 DNA as a probe. The hamster ZP3 gene has a transcription unit of 7900 nucleotides and was found to contain 7 introns and 8 exons. The hamster ZP3 protein is approximately 81 % homologous to mouse ZP3 protein. The hamster transcript contained 1266 nucleotides, six less than mouse ZP3 mRNA.

Chamberlain and Dean (Proc. Natl. Acad. Sci. USA (1990) 87:6014-6018), reported the cloning of human ZP3 from a human genomic DNA library using mouse ZP3 cDNA as a probe. The human ZP3 gene is composed of 8 exons in a transcription unit of 18.3 kbp. The exons are almost identical in size to the eight exons of mouse ZP3 and the nucleotide sequence of the coding region is 74% homologous. The human ZP3 transcript is very similar to mouse ZP3 mRNA. Both have short 5' and 3' untranslated regions, and both have a single open reading frame of 1272 nucleotides that encodes a 424-amino acid protein.

U.S. Pat. No. 4,996,297, to Dunbar, reported the isolation of three rabbit zona pellucida clones encoding rabbit ZP1 and ZP2 proteins, using anti-ZP1 and anti-ZP2 antibodies as screening probes. The sequences designated as P2 and P3 in FIG. 4 of the Dunbar patent represent rabbit ZP cDNAs of 812 and 1705 nucleotides respectively.

Schwoebel et al. (J. Biol. Chem. (1991) 266:7214-7219), isolated and characterized a full length cDNA (designated rc 55) encoding the 55-kD rabbit zona pellucida protein using cross-species affinity purified antisera. The protein encoded by this cDNA has some similarity to the mouse ZP2 protein described by Liang. However, comparisons of rc 55 with the mouse ZP3 protein revealed no homology.

Other ZP glycoprotein nucleotide sequences, useful in the present invention include, but are not limited to, Human lutenizing hormone-releasing hormone (LHRH, Genbank accession No. X01059), Human ZP3A glycoprotein (GenBank Accession No. XM004616), human ZPB glycoprotein (GenBank Accession No. XM001779), Bovine ZPA and B glycoproteins (GenBank Accession Nos. AB042653; AB042652), Chicken ZP1 and 3 glycoprotein (GenBank Accession Nos. AJ289697; AB031033), mouse ZP1, 2, and 3 glycoprotiens (GenBank Accession Nos. NM011776; NM011775; NM009580), vole ZP3 glycoprotein (GenBank Accession No. AF304487), dunnart ZPA (GenBank Accession No. AF263025), brushtail possum ZPA and B

(GenBank Accession Nos. AF263014; AF263013), brushtail possum ZP2 and 3 (GenBank Accession Nos. AF079525; AF079524) swine ZP1, 2, and 3 glycoprotein (GenBank Accession Nos. S74651; E07737; D45064), rat ZP1, 2, and 3 glycoprotein (GenBank Accession Nos. D78482; AB000929; AB000928), marmoset ZP1 and 2 glycoprotein (GenBank Accession Nos. Y10822; Y10767), feline ZP2 and 3 (GenBank Accession Nos. E07930; E06506), dog ZP2 and 3 (GenBank Accession Nos. D45064; D45070), and wallaby ZP3 glycoprotein (Australian Patent No. AU78554/98).

The term "zona pellucida glycoprotein", as used herein, includes a complete ZP protein, an enzymatically digested or chemically denatured form of ZP, or a combinations thereof. In addition, zona pellucida glycoprotein includes isolated ZP antigenic determinants which are not inherently immunogenic but which can be utilized as an immunogen when they are chemically linked to immunogenic carrier molecules (e.g., LT-B) as described herein below. A ZP extract includes a whole extract or one or more of the three individual glycoprotein fractions which comprise ZP,

### Other Contraceptive Proteins

In addition to the ZP glycoproteins described above, or fragments thereof, other proteins are useful as contraceptive proteins in the present invention. For example, in one embodiment a contraceptive protein may be selected from the group including, but by no means limited to gonadotrophin releasing hormone (GnRH), leutinizing hormone (LH), lactate dehydrogenase (LDH) and anti-sperm antigens. In addition to these contraceptive proteins, the nucleotide sequences which encode contraceptive proteins useful in the present invention, are also useful in the generation of transgenic plants which express the encoded contraceptive proteins. For example, nucleotide sequences encoding GnRH, useful in the present invention include, but are not limited to human GnRH1 (GenBank Accession No. XM005041), rat GnRH1 (GenBank Accession No. M31670), brushtail possum GnRH (GenBank Accession No. AF193516), and shrew GnRH (GenBank Accession No. AF107315). Nucleotide sequences encoding LH, useful in the present invention include, but are not limited to rat LH (GenBank Accession No. D00576), pig LH (GenBank Accession No. D00579), rhinoceros LH (GenBank Accession No. AF024521), tapir LH (GenBank Accession No. AF047606), horse LH (GenBank Accession Nos. Y16326; Y16265), bovine LH (GenBank Accession No. M11506), and canine LH (GenBank Accession No. Y00518).

In one embodiment, contraceptive proteins of the present invention include anti-sperm antigens. Immunization of male and female animals with extracts of whole sperm has been shown previously to cause infertility (Tung et al., (1979) J. Reprod. Immunol. 20:931). Accordingly, contraceptive proteins of the present invention may comprise a sperm-associated protein or nucleic acid sequence encoding such proteins including, but not limited to PH30 (U.S. Pat. No. 5,935,578), PH34 (U.S. Pat. No. 5,723,305), SP17 (U.S. Pat. No. 5,814,456), SP22 (U.S. Pat. No. 6,197,940), SP56 (mouse SP56: GenBank Accession No. U17108), LDHC₄ (human LDHC₄: GenBank Accession No. J02938; U.S. Pat. No. 5,891,992; Goldburg "LDH-X as a sperm-specific antigen", in T. Wegmann and T.J. Gill (eds.), Reproductive Immunology, Oxford University Press, 1981), fertilin (mouse fertilin: GenBank Accession No. AF167406; human fertilin: GenBank Accession No. AJ133005; tree shrew fertilin: GenBank Accession No. Y15965; baboon fertilin: GenBank Accession No. Y15520; gorilla fertilin: GenBank Accession No. Y15492; tamarin fertilin: GenBank Accession No. Y15512; bovine fertilin: GenBank Accession No. AF086808; and rat fertilin: GenBank Accession No. Y08616),

### Mucosal Targeting Proteins

In one embodiment of the present invention, a transgenic plant is administered to an animal, wherein the plant expresses a portion, or epitope of one or more of the contraceptive proteins described above. It is well recognized in the art that mucosal targeting of immunogenic substances is highly dependent upon particle size or natural binding affinity of a sufficient degree to ensure M-cell recognition. In addition, it has been found that the mucosal immune system may be stimulated by feeding low doses of certain classes of proteins. In particular, this may achieved with proteins which share the property of being able to bind specifically to various glycolipids and glycoproteins located on the surface of the cells on the mucosal membrane. Accordingly, the present invention provides a fusion protein comprising an auxilary immunogenic proteins which is fused to a complex immuno-contraceptive protein, or if desired to a smaller soluble contraceptive antigen thereof, such as a ZP3 epitope, or GnRH, to enhance the immunogenicity of the contraceptive eptiope.

Auxilary proteins, useful according to the invention for stimulating a mucosal immune response include antigenic proteins such as shigatoxin B (StxB) (Genbank Accession No. AJ132761), staphylococcus enterotoxin B (SEB)(GenBank Accession No. M11118), *E. coli* labile toxin B (LT-B)(GenBank Accession No. AB011677), *E. coli* labile toxin A subunit (LT-A) (GenBank Accession No. AB011677), Norwalk virus capsid protein (NVCP)(GenBank Accession No. AF093797), and hepatitis B surface antigen (HBsAg)(GenBank Accession No. AF090842). It is preferred that such antigenic proteins associate as antigenic particles and/or complexes when such association is necessary to impart immunogenicity thereto. Construction of fusion proteins comprising contraceptive proteins useful in the present invention and proteins which enhance mucosal immunogenicity will be described below.

### Hepatitis B Surface Antigen (HBsAg)

The feasibility of expressing HBsAg as virus-like particles (VLPs) in plants has been demonstrated (Mason *et al*., 1992). The VLPs are similar to the recombinant yeast-derived vaccine, which is licensed for parenteral immunization. The formation of HBsAg particles requires insertion of the peptide in the endoplasmic reticulum (ER) membrane with four transmembrane domains, followed by budding of particles into the ER lumen. The plant-derived HBsAg retains both B- and T-cell epitopes when studied in a mouse model (Thanavala *et al*., 1995). The finding that plant cells can produce an immunogenic HBsAg VLP indicates that plants are a feasible expression system for animal, viral, or bacterial proteins that assemble into complex structures. Accordingly, the necessary genetic elements for generating immunogenic HBsAg particles have been identified. These elements, e.g., regulatory and coding regions, can be incorporated into a vector encoding a contraceptive protein as described herein to afford a means of amplifying expression of this antigen in plants.

### Norwalk Capsid Protein (NVCP)

The expression and VLP assembly in plants of NVCP, and its oral immunogenicity in mice have been reported (Mason *et al*., 1996). The NVCP accumulated to 0.3% of the total protein, and assembled into VLPs with about 60% efficiency in tobacco leaf and potato tuber cells. When viewed by negative staining electron microscopy, the empty capsids were virtually indistinguishable from those produced in an insect cell system. Further, the material was orally immunogenic in mice when given by gavage in 4 doses as low as 10 µg each, or when given by direct feeding of potato tuber slices in 4 doses as low as 50 µg each. Both serum IgG and gut mucosal IgA were stimulated by the plant vaccine. Thus, the means for generating immunogenic amounts of VLPs of NVCP antigens have been identified. These can be used with the present invention to generate yet higher levels of immunogen in plants according to the gene amplification methods described herein.

### E. coli heat-labile enterotoxin (LT)

LT is a potent mucosal immunogen and adjuvant that stimulates immune responses against co-administered antigens (Clements *et al*., 1988). The B-subunit of LT (LT-B) expressed in plants assembles into active oligomers that possess ganglioside G_{M1} binding capacity (Haq *et al*., 1995). It is found that addition of a microsomal retention sequence (SEKDEL) at the carboxyl-terminus of LT-B increases its accumulation in plant tissue, while still allowing G_{M1} binding and immunogenicity. In oral tests with plant-derived LT-B given to mice, either tobacco leaf extracts administered by gavage or potato tubers fed without preparation (other than slicing) stimulated serum and gut mucosal antibodies against LT-B (Haq *et al*., 1995). The serum anti-LT-B from these animals showed inhibition of LT activity, indicating its potential value as a protective vaccine.

### Vectors for Contraceptive Protein Expression

Central to the use of the present invention is the creation and/or use of a nucleic acid construct comprising a nucleic acid sequence encoding a contraceptive protein and optionally a nucleic acid sequence encoding a mucosal targeting protein, wherein the construct can be introduced into a plant cell of interest to produce a transgenic plant that expresses the contraceptive protein. In preferred embodiments, the nucleic acid construct is a plant expression vector, a plasmid that can be prepared and grown in plant cells. The nucleic acid sequences of the construct can contain DNA, RNA, a synthetic nucleic acid, or a combination thereof, as known in the art. In embodiments where the nucleic acid construct comprises both a contraceptive protein and a mucosal targeting protein, the sequences encoding each of the components may be interspersed with other sequences (e.g., regulatory sequences) as needed.

Nucleic acid constructs, useful in the present invention may be generated using a plasmid backbone known to those of skill in the art which may be used to mediate expression of the contraceptive proteins in plant cells according to the invention. Appropriate vectors which can be utilized as starting materials are known in the art. Suitable vectors for transforming plant tissue have been described by deFramond et al. (Biotechnology 1983, 263), An et al. (EMBO 1985, 277), and Rothstein et al. (Gene 1987, 53), and include, but are not limited to pBluescript (Stratagene, La Jolla, CA), pIBT210 (Haq et al., (1995) Science 268:714), pGEM (Promega, Medison, WI) pGPTV.kan (Becker et al., (1992) Plant Mol. Biol. 20:1195-1197.), Agrobacterium-Ti plasmid (White et al., Plant Biotechnology Kung and Arntzen eds. Butterworth Pub., Boston, MA, 1989) In addition to these vectors, ma others have been produced in the art which are suitable for use in the present invention.

Vector constructs, useful in the present invention, preferably contain DNA sequences encoding contraceptive proteins. A DNA sequence encoding a contraceptive protein is obtained by conventional means and inserted into a vector suitable for the transformation of plants. For example, the DNA sequence can be isolated from a gene bank of genomic clones. Alternatively, the DNA sequence can be prepared by reverse transcription. The vectors are then introduced into plant cells by a variety of known techniques, described below, which give rise to transformed cells, tissues, and plants.

The DNA sequence can by chemically synthesized in the amino acid sequence of the contraceptive protein, or portion thereof is known. Several prior art methods can be utilized to determine the amino acid sequence of a contraceptive protein (see, for example Ausubel et al., Short Protocols in Molecular Biology 3rd Ed. John Wiley & Sons, Inc. 1995).

The DNA sequence encoding a contraceptive protein or part thereof is inserted into an appropriate vector in such a manner that the contraceptive protein is correctly expressed. In other words, the DNA sequence is positioned in the proper orientation and reading frame so that the correct amino acid sequence is produced upon expression of the DNA sequence in plant tissue. In accordance with conventional techniques, a chimeric DNA sequence is generally constructed which contains a promoter operably in plant tissue and the DNA sequence encoding a contraceptive protein. The DNA sequence may further contain 3' non-coding sequence operable in plant tissue. In one embodiment, the chimeric DNA sequence may further contain a coding sequence for a polypeptide which can enhance the mucosal targeting of the contraceptive protein, such as LT-B, such that a fusion protein is produced upon expression. The chimeric DNA sequence can be prepared *in situ* within a suitable vector by inserting the DNA sequence coding for a contraceptive protein or portion thereof in to a restriction site of a known plant transformation vector. Alternatively, the chimeric gene may be first constructed and then inserted into a vector to produce a plant transformation vector. Vectors, useful in the invention, are generally inserted into a prokaryotic host cell, such as *E. coli,* wherein the number of vectors is amplified by replication. Vectors may subsequently be isolated from the prokaryotic host cell, using a technique known in the art (see, for example Ausubel et al., *supra*), and subsequently used to transform a host plant cell of interest.

Promoter sequences of the present invention include, but are not limited to a promoter that is operably in a selected plant host cell according to the invention. The DNA construct according to the invention preferably has a plant-functional promoter operably linked to the 5' end of a nucleotide sequence of interest. A preferred promoter is selected from among CVMV, Gamma Sein, Gelvin promoter, CaMV 35S, tomato E8, patatin, ubiquitin, mannopine synthase (*mas*)*,* rice *actin 1*, soybean seed protein glycinin (Gy1), soybean vegetative storage protein (*vsp*), and granule-bound starch synthase (*gbss*). A construct of the present invention can also include a translational enhancer region, such as tobacco etch virus (TEV) enhancer, which has been described elsewhere (Carrington, *et al.* (1990)). Optionally, a construct of the invention can comprise at least one vegetative storage protein (VSP) signal peptide encoding sequence, such as an αS or αL sequence (Mason *et al*. 1988), operably linked to the 5' end of a nucleotide sequence encoding a protein of interest.

Preferably, the sequences of the DNA construct encoding the contraceptive protein and, optionally, the mucosal targeting protein are all under the control of a single promoter sequence, resulting in the expression of a fusion protein containing each of the three elements. This assures that the contraceptive protein and the mucosal targeting protein with be synthesized in stiochiometric proportion, and results in similar levels of expression of the contraceptive protein and the mucosal targeting protein. Alternatively, the expression of the mucosal targeting protein can be driven from a second promoter inserted in a bidirectional manner, or into the construct 3' of the sequence encoding the contraceptive protein, but 5' of the sequence encoding the mucosal targeting protein.

Vectors, useful in the present invention, in addition to carrying the DNA constructs described above, may additionally comprise a selectable marker gene. Marker genes useful according to the invention may include a gene encoding a selectable marker, e.g., an antibiotic resistance gene such as the bacterial tetracycline resistance gene. Incorporation of the tetracycline resistance gene permits the use of tetracycline as a selective agent in the plasmid preparation procedure according to the invention. One advantage to the use of a tetracycline resistance gene is that tetracycline is not degraded in *E. coli,* and therefore more tetracycline does not have to be added during fermentation. In addition, the tetracycline resistance gene is preferred over a gene encoding ampicillin resistance because tetracycline is prescribed less often as an antibiotic in a clinical setting, and therefore read through from the plasmid resistance gene will be less likely to interfere with the use of an antibiotic in a clinical setting.

Additional marker genes useful according to the invention include resistance to biocide, particularly an antibiotic, such as kanamycin, G418, bleomycin, hygromycin, chloramphenicol or the like. Additional marker genes useful according to the invention include resistance to herbicides such as organophosphates and bialaphosphates. Additional marker genes useful according to the invention also include those encoding for visual marker proteins such as beta glucorinidase (GUS), green flourescent protein (GFP), or the like. The particular marker employed will be one which allows for selection of transformed cells as compared to cells lacking the nucleic acid which has been introduced.

A vector can also have an *A. tumefaciens* origin of replication, such as when it is desired to maintain the vector in *A. tumefaciens* for later transformation with this system. In this event, the nucleotide sequence encoding a protein of interest (i.e., a contraceptive protein) is flanked by the left and right T-DNA border regions to effect its transfer to a host plant cell.

A strain of bacteria, such as *E. coli,* can be transfected with an expression vector of the present invention in order to grow/amplify an instant expression cassette according to methods well known in the art (Ausubel, supra). The purified expression cassette can be added to *A. tumefaciens* and subjected to electric pulse or heat shock treatment to introduce the vector therein, where it can reside intact as a shuttle vector. A helper Ti plasmid in the *A. tumefaciens* can provide the vir genes necessary to transfer the T-DNA directly from the shuttle vector to the plant cell. Alternatively, the vector can undergo homologous recombination with a tumor-inducing (Ti) plasmid and exchange the instant cassette for the T-DNA of the Ti plasmid. In preferred embodiments, the invention provides for methods of stably transforming plant cells wherein a DNA construct that is introduced into a plant cell is stably integrated into a chromosome.

### Transgenic Plants

The present invention provides transgenic plants and plant material (e.g., leaves, fruit, roots, etc.) comprising plant cells which express one or more contraceptive proteins useful in the present invention. In a preferred embodiment, a plant cell of interest in transformed with one or more of the DNA constructs described above, wherein expression of the construct within the transformed plant cell results in the production of a contraceptive protein by the plant cell.

### Methods of Introducing Nucleic Acid into Plant Cells

Methods of gene transfer into plants include, but are not limited to use of the *A. tumefaciens* --Ti plasmid system. The tumor-inducing (Ti) plasmids of *A. tumefaciens* contain a segment of plasmid DNA called transfer DNA (T-DNA), which integrates into the plant host genome. First, a plasmid vector is constructed that replicates in *E. coli.* This plasmid contains the DNA encoding the protein of interest (i.e., a contraceptive protein) and this DNA is flanked by T-DNA border sequences, which define the limits to the segment of DNA which is transferred into the plant cell and integrated into the plant genome. Usually a gene encoding a selectable marker (such as a gene encoding resistance to an antibiotic such as kanamycin) is also inserted between the left border (LB) and right border (RB) sequences. The expression of this gene in transformed plant cells gives a positive selection method to identify those plants or plant cells having an integrated T-DNA region. Second, the plasmid is transferred to *Agrobacterium.* This can be accomplished via a direct uptake of plasmid DNA by the *Agrobacterium.* For subsequent transfer of the T-DNA to plants, the *Agrobacterium* strain utilized must contain a set of inducible virulence (*vir*) genes, which are essential for T-DNA transfer to plant cells.

The *A. tumefaciens* gene transfer system mentioned above is the etiologic agent of crown gall, a disease of a wide range of dicotyledons and gymnosperms [DeCleene, M. et. al., Bot. Rev. 42, 389 (1976)], that results in the formation of tumors or galls in plant tissue at the site the infection. The *Agrobacterium* system has been developed to permit routine transformation of a variety of plant tissue [see, e.g., Schell, J. et al., Bio/Technology 1, 175 (1983); Chilton, M-D, Scientific American 248, 50 (1983)]. Representative tissues transformed in this manner include tobacco [Barton, K. et al., Cell 32, 1033 (1983)]; tomato [Fillatti, J. et al., BiolTechnology 5, 726 (1987)]; sunflower [Everett, N. et al.,. BiolTechnology 5, 1201 (1987)]; cotton [Umbeck, P. et al., Bio/Technology 5, 263 (1987)]; rapeseed [Pua, E. et al., BiolTechnology 5, 815 (1987)]; potato [Facciotti D. et al., BiolTechnology 3, 241 (1985); poplar [Pythoud, F. et al., Bio/Technology 5, 1323 (1987); and soybean [Hinchee, M. et al., Bio/Technology 6, 915 (1988)]. Other plants can be transformed by routine extensions or modifications of these methods.

Multiple choices of *Agrobacterium* strains and plasmid construction strategies can be used to optimize genetic transformation of plants. For instance, *A. tumefaciens* may not be the only *Agrobacterium* strain used. Other *Agrobacterium* strains such as *A. rhizogenes* may be more suitable in some applications. *A. rhizogenes,* which incites root hair formation in ma dicotyledonous plant species, carries a large extra-chromosomal element called an Ri (root-including) plasmid, which functions in a manner analogous to the Ti plasmid of *A. tumefaciens.* Transformation using *A. rhizogenes* has developed analogously to that of *A. tumefaciens* and has been successfully utilized to transform, for example, alfalfa, [Sukhapinda, K. et al., Plant Mol. Biol. 8, 209 (1987)].

Methods of inoculation of the plant tissue vary depending upon the plant species and the *Agrobacterium* delivery system. A convenient approach is the leaf disc procedure for transforming potato, however *Agrobacterium*-mediated transformation can be performed with a tissue explant that provides a good source for initiation of whole plant differentiation and regeneration. The addition of nurse tissue may be desirable under certain conditions. Other procedures such as *in vitro* transformation of regenerating protoplasts with *A. tumefaciens* may be followed to obtain transformed plant cells as well.

Several so-called "direct" gene transfer procedures have been developed to transform plants and plant tissues without the use of an *Agrobacterium* intermediate, for example plant regeneration from protoplasts [Evans, D. A. et al., Handbook of Plant Cell Culture 1, 124 (1983)]. When a plant species can be regenerated from protoplasts, direct gene transfer procedures can be utilized and transformation is not dependent on the use of *A. tumefaciens.* In the direct transformation of protoplasts the uptake of exogenous genetic material into a protoplast may be enhanced by use of a chemical agent or electric field. The exogenous material may then be integrated into the nuclear genome.

Early work has been conducted in the dicot *Nicotiana tabacum* (tobacco) where it was shown that the foreign DNA was incorporated and transmitted to progeny plants [Paszkowski, J. et al., EMBO J. 3: 2717 (1984); Potrykus, I. et al., Mol. Gen. Genet. 199: 169 (1985)]. Monocot protoplasts have also been transformed by this procedure: for example, *Triticum monococum* [Lorz H. et al., Mol. Gen. Genet. 199: 178 (1985)]; *Lolium multiflorum* (Italian ryegrass), Potrykus, I. et. al., Mol. Gen. Genet 199, 183 (1985); maize [Rhodes, C., et al., Bio/Technology 5, 56 (1988)]; and Black Mexican sweet corn [Fromm, M. et al., Nature 319, 719 (1986)]. Other plants that have been regenerated from protoplasts include rice [Abdulah, R. et al., BiolTechnology 4, 1987 (1987)]; rapeseed [Kansha, et al., Plant Cell Reports 5, 101 (1986)]; potato [Tavazza, R. et al., Plant Cell Reports 5, 243 (1986)]; eggplant, Sihachaki, D. et al., Plant Cell, Tissue, Organ Culture 11, 179 (1987); and cucumber [Jia, S-R., et al., J. Plant Physiol. 124, 393 (1986)]. Methods for directly transforming protoplasts of other varieties are evident.

Introduction of DNA into protoplasts of a plant can be effected by treatment of the protoplasts with an electric pulse in the presence of the appropriate DNA in a process called electroporation. In this method, the protoplasts are isolated and suspended in a mannitol solution. Supercoiled or circular plasmid DNA is added. The solution is mixed and subjected to a pulse of about 400 V/cm at room temperature for less than 10 to 100 microseconds. A reversible physical breakdown of the membrane occurs to permit DNA uptake into the protoplasts.

Liposome fusion has also been shown to be a method for transformation of plant cells. In this method, protoplasts are brought together with liposomes carrying the desired gene. As membranes merge, the foreign gene is transferred to the protoplasts [Dehayes, A. et al., EMBO J. 4, 2731 (1985)].

Polyethylene glycol (PEG) mediated transformation has been carried out in *N. tabacum* (a dicot) and *Lolium multiflorum* (a monocot). It is a chemical procedure of direct gene transfer based on synergistic interaction between Mg²⁺, PEG, and possibly Ca²⁺ [Negrutiu, R. et al., Plant Mol. Biol. 8, 363 (1987)]. Alternatively, exogenous DNA can be introduced into cells or protoplasts by microinjection. A solution of plasmid DNA is injected directly into the cell with a finely pulled glass needle.

Another developed procedure for direct gene transfer involves bombardment of cells by microprojectiles carrying DNA [Klein, T. M. et al., Nature 327, 70 (1987)]. In this "biolistic" procedure, tungsten or gold particles coated with the exogenous DNA are accelerated toward the target cells. At least transient expression has been achieved in onion. This procedure has been utilized to introduce DNA into Black Mexican sweet corn cells in suspension culture and maize immature embryos and also into soybean protoplasts [Klein, T. M. et al., Bio/Technology 6, 559 (1988)]. Stably transformed cultures of maize, tobacco, barley, oats, wheat, rice, carrot, banana and soybean have been obtained by microprojectile bombardment. Stably transformed plants can also be regenerated and recovered by this procedure [McCabe, D. E. et al., Bio/Technology 6, 923 (1988)].

To produce transformed seeds, flowers of Arabidopsis are transformed according to the following method. The Agrobacterium is vacuum-infiltrated into developing flowers, and the resulting seed are then screened for marker resistance and foreign gene expression. Presumably, stamens/pollen, ovary/egg, or even the developing zygote if fertilization has already occurred, are transformed. This method (described in Clough & Bent, 1998, Plant J., 16:735) is used to transform Arabidopsis with a construct comprising a rep gene under the transcriptional regulation of the At2S-2 seed promoter.

Following transformation, the transformed cell or plant tissue is selected or screened by conventional techniques. The transformed cell of plant tissue containing the chimeric DNA sequence discussed above is then regenerated using known procedures, including those described in the referenced discussed above. The species of plant which can most easily be regenerated by these techniques, and are thus, preferred in the present invention include, but are not limited to maize, sunflower, rapeseed, clover, tobacco, cotton, alfalfa, rice, potato, eggplant, cucumber and soybean. The regenerated plants are screened for transformation by standard methods described below. Progeny of the regenerated plants are screened and selected for the continued presence of the integrated DNA sequence in order to develop improved plant and seed lines. The DNA sequence can be moved into other genetic lines by a variety of techniques, including classical breeding, protoplast fusion, nuclear transfer and chromosome transfer.

### Plants, Cells and Seeds Useful According to the Invention

Plants that can be used for practice of the present invention include a dicotyledon and monocotyledon. These include, but are not limited to, tobacco, carrot, spinach, pepper, potato, tomato, apple, wheat, rye, soybean, rice, maize, corn, berries such as strawberries, raspberries, alfalfa and banana. Since ma edible plants used by humans for food or as components of animal feed are dicotyledenous plants, dicotyledons are typically employed, although monocotyledon transformation is also applicable especially in the production of certain grains useful for animal feed. It is particularly advantageous in human immunocontraception to produce a contraceptive protein in a juice for ease of administration to humans such as juice of tomato, soybean, and carrot, or milk. Cells and seeds derived from these plant vaccines are also useful according to the invention.

A transgenic plant transformed with a vector described hereinabove is another aspect of the present invention. Particularly preferred plant hosts for the vector include banana, tomato, potato, carrot, alfalfa, medicago, maize, and tobacco.

Potato varieties FL 1607 ("Frito Lay 1607") and Desiree, and tomato variety Tanksley TA234TM2R are particularly preferred varieties, which have been transformed with binary vectors using the methods described herein. Of these transformed varieties, Desiree is the only commercial variety; the other varieties can be obtained from Frito-Lay (Rhinelander, WI) and Steve Tanksley (Dept. of Plant Breeding, Cornell Univ.). Tomato is preferred as a model system for expression of foreign proteins because of its ease of genetic transformation, and because fruit-specific, ripening dependent promoters are available for regulated expression (Giovannoni *et al*., 1989). The E8 promoter has been used to mediate high level production of polygalacturonase protein in mutant tomato fruit (Giovannoni *et al*., 1989) and monellin in wild-type tomato fruit (Penarrubia *et al*., 1992).

Plant cell suspension cultures are widely known and available to those in the art. Plant cell culture is preferred as a model system for expression of foreign proteins because they allow transformation, growth and protein production more rapidly than whole plant systems. Plant cell suspension cultures can be grown in liquid, or can be transferred to solid media for callus production. Common examples of cell suspension cultures which could be used for the purposes described in the invention include but are not limited to carrot, tobacco (eg. NT-1 or BY-1 cell lines), maize.

### Detection of Transformed Plant Material

The invention provides for methods of detecting nucleic acid sequences encoding contraceptive proteins and the contraceptive proteins themselves including but not limited to Southern and northern blot analysis, PCR-based methods of detection, as well as immunological methods of detecting a protein of interest according to the invention. These techniques may be used according to the invention, to confirm the presence of nucleic acid sequences encoding contraceptive proteins, and optionally mucosal targeting proteins, and to confirm the expression of the contraceptive and mucosal targeting proteins themselves.

### Detection of a Nucleotide Sequence of Interest

### 1. Southern Blot Analysis

Southern blot analysis can be used to detect a nucleotide sequence of interest from a PCR amplified product or from a total genomic DNA test sample via a non-PCR based assay. The method of Southern blot analysis is well known in the art (Ausubel et al., supra, Sambrook et al., 1989, Molecular Cloning. A Laboratory Manual., 2nd Edition, Cold Spring Harbor Laboratory Press, Cold Spring Harbor, NY). This technique involves the transfer of DNA fragments from an electrophoresis gel to a membrane support resulting in the immobilization of the DNA fragments. The resulting membrane carries a semi-permanent reproduction of the banding pattern of the gel.

Southern blot analysis is performed according to the following method. Genomic DNA (5-20µg) obtained from a plant transformed with a nucleic acid vector according to the methods of the present invention is digested with the appropriate restriction enzyme and separated on a 0.6-1.0% agarose gel in TAE buffer. The DNA is transferred to a commercially available nylon or nitrocellulose membrane (e.g. Hybond-N membrane, Amersham, Arlington Heights, IL) by methods well known in the art (Ausubel et al., supra, Sambrook et al., supra). Following transfer and UV cross linking, the membrane is hybridized with a labeled probe in hybridization solution (e.g. under stringent conditions in 5X SSC, 5X Denhardt solution, 1% SDS) at 65°C. Alternatively, high stringency hybridization can be performed at 68°C or in a hybridization buffer containing a decreased concentration of salt, for example 0.1X SSC. The hybridization conditions can be varied as necessary according to the parameters known in the art. Following hybridization, the membrane is washed at room temperature in 2X SSC/0.1% SDS and at 65°C in 0.2X SSC/0.1% SDS, and exposed to film. The stringency of the wash buffers can also be varied depending on the amount of the background signal (Ausubel et al., supra).

Detection of a nucleic acid probe-target nucleic acid hybrid will include the step of hybridizing a nucleic acid probe to the DNA target, wherein the probe is complementary to a portion of the nucleic acid sequence of the DNA construct described above. This probe may be radioactively labeled or covalently linked to an enzyme such that the covalent linkage does not interfere with the specificity of the hybridization. A resulting hybrid between plant DNA and the probe can be detected. Methods for labeling a probe include random oligonucleotide primed synthesis, nick translation, kinase reactions, or polymerase chain reaction (see Ausubel et al., supra). Alternatively, a hybrid can be detected via non-isotopic methods. Non-isotopically labeled probes can be produced by the addition of biotin or digoxigenin, fluorescent groups, chemiluminescent groups (e.g. dioxetanes, particularly triggered dioxetanes), enzymes or antibodies. Typically, non-isotopic probes are detected by fluorescence or enzymatic methods. Detection of a radiolabeled probe-target nucleic acid complex can be accomplished by separating the complex from free probe and measuring the level of complex by autoradiography or scintillation counting. If the probe is covalently linked to an enzyme, the enzyme-probe-conjugate-target nucleic acid complex will be isolated away from the free probe enzyme conjugate and a substrate will be added for enzyme detection. Enzymatic activity will be observed as a change in color development or luminescent output resulting in a 10³-10⁶ increase in sensitivity. An example of the preparation and use of nucleic acid probe-enzyme conjugates as hybridization probes (wherein the enzyme is alkaline phosphatase) is described in (Jablonski et al., 1986, Nuc. Acids Res., 14:6115)

Two-step label amplification methodologies are known in the art. These assays are based on the principle that a small ligand (such as digoxigenin, biotin, or the like) is attached to a nucleic acid probe capable of specifically binding to a gene of interest (i.e., a gene encoding a contraceptive protein or portion thereof).

According to the method of two-step label amplification, the small ligand attached to the nucleic acid probe will be specifically recognized by an antibody-enzyme conjugate. For example, digoxigenin will be attached to the nucleic acid probe and hybridization will be detected by an antibody-alkaline phosphatase conjugate wherein the alkaline phosphatase reacts with a chemiluminescent substrate. For methods of preparing nucleic acid probe-small ligand conjugates, see (Martin et al., 1990, BioTechniques, 9:762). Alternatively, the small ligand will be recognized by a second ligand-enzyme conjugate that is capable of specifically complexing to the first ligand. A well known example of this manner of small ligand interaction is the biotin avidin interaction. Methods for labeling nucleic acid probes and their use in biotin-avidin based assays are described in Rigby et al., 1977, J. Mol. Biol., 113:237 and Nguyen et al., 1992, BioTechniques, 13:116).

Variations of the basic hybrid detection protocol are known in the art, and include modifications that facilitate separation of the hybrids to be detected from extraneous materials and/or that employ the signal from the labeled moiety. A number of these modifications are reviewed in, e.g., Matthews & Kricka, 1988, Anal. Biochem., 169:1; Landegren et al., 1988, Science, 242:229; Mittlin, 1989, Clinical Chem. 35:1819; U.S. Pat. No. 4,868,105, and in EPO Publication No. 225,807.

### 2. Northern Blot Analysis

The method of Northern blotting is well known in the art. This technique involves the transfer of RNA from an electrophoresis gel to a membrane support to allow the detection of specific sequences in RNA preparations.

Northern blot analysis is performed according to the following method. An RNA sample obtained from plant tissue transformed with the DNA constructs described above (prepared by the addition of MOPS buffer, formaldehyde and formamide) is separated on an agarose/formaldehyde gel in 1X MOPS buffer. Following staining with ethidium bromide and visualization under ultra violet light to determine the integrity of the RNA, the RNA is hydrolyzed by treatment with 0.05M NaOH/1.5MNaCl followed by incubation with 0.5M Tris-Cl (pH 7.4)/1.5M NaCl. The RNA is transferred to a commercially available nylon or nitrocellulose membrane (e.g. Hybond-N membrane, Amersham, Arlington Heights, IL) by methods well known in the art (Ausubel et al., supra, Sambrook et al., supra). Following transfer and UV cross linking, the membrane is hybridized with a labeled probe in hybridization solution (e.g. in 50% formamide/2.5% Denhardt's/100-200mg denatured salmon sperm DNA/0.1% SDS/5X SSPE) at 42°C. The hybridization conditions can be varied as necessary as described in Ausubel et al., supra and Sambrook et al., supra. Following hybridization, the membrane is washed at room temperature in 2X SSC/0.1% SDS, at 42°C in 1X SSC/0.1% SDS, at 65°C in 0.2X SSC/0.1% SDS, and exposed to film. The stringency of the wash buffers can also be varied depending on the amount of background signal (Ausubel et al., supra).

### 3. PCR

Nucleic acid sequences of interest (i.e., those encoding a contraceptive protein) of the invention are amplified from genomic DNA or other natural sources by the polymerase chain reaction (PCR). PCR methods are well-known to those skilled in the art.

PCR provides a method for rapidly amplifying a particular DNA sequence by using multiple cycles of DNA replication catalyzed by a thermostable, DNA-dependent DNA polymerase to amplify the target sequence of interest. PCR requires the presence of a nucleic acid to be amplified, two single stranded oligonucleotide primers flanking the sequence to be amplified, a DNA polymerase, deoxyribonucleoside triphosphates, a buffer and salts.

The method of PCR is well known in the art. PCR, is performed as described in Mullis and Faloona, 1987, Methods Enzymol., 155: 335.

PCR is performed using template DNA (at least 1fg; more usefully, 1-1000 ng) and at least 25 pmol of oligonucleotide primers. A typical reaction mixture includes: 2µl of DNA, 25 pmol of oligonucleotide primer, 2.5 µl of 10X PCR buffer 1 (Perkin-Elmer, Foster City, CA), 0.4 µl of 1.25 µM dNTP, 0.15 µl (or 2.5 units) of Taq DNA polymerase (Perkin Elmer, Foster City, CA) and deionized water to a total volume of 25 µl. PCR is performed using a programmable thermal cycler.

The length and temperature of each step of a PCR cycle, as well as the number of cycles, are adjusted according to the stringency requirements in effect. Annealing temperature and timing are determined both by the efficiency with which a primer is expected to anneal to a template and the degree of mismatch that is to be tolerated. The ability to optimize the stringency of primer annealing conditions is well within the knowledge of one of moderate skill in the art. An annealing temperature of between 30°C and 72°C is used. Initial denaturation of the template molecules normally occurs at between 92°C and 99°C for 4 minutes, followed by 20-40 cycles consisting of denaturation (94-99°C for 15 seconds to 1 minute), annealing (temperature determined as discussed above; 1-2 minutes), and extension (72°C for 1 minute). The final extension step is generally carried out for 4 minutes at 72°C, and may be followed by an indefinite (0-24 hour) step at 4°C.

Several techniques for detecting PCR products quantitatively without electrophoresis may be useful according to the invention. One of these techniques, for which there are commercially available kits such as Taqman^{™} (Perkin Elmer, Foster City, CA), is performed with a transcript-specific antisense probe. This probe is specific for the PCR product (e.g. a nucleic acid fragment derived from a gene of interest) and is prepared with a quencher and fluorescent reporter probe complexed to the 5' end of the oligonucleotide. Different fluorescent markers can be attached to different reporters, allowing for measurement of two products in one reaction. When Taq DNA polymerase is activated, it cleaves off the fluorescent reporters of the probe bound to the template by virtue of its 5'-to-3' nucleolytic activity. In the absence of the quenchers, the reporters now fluoresce. The color change in the reporters is proportional to the amount of each specific product and is measured by a fluorometer; therefore, the amount of each color can be measured and the PCR product can be quantified. The PCR reactions can be performed in 96 well plates so that multiple samples can be processed and measured simultaneously. The Taqman^{™} system has the additional advantage of not requiring gel electrophoresis and allows for quantification when used with a standard curve.

### Detection of a Protein Sequence of Interest

### 1. Preparation of Antibodies

Antibodies specific for the contraceptive proteins of the present invention are useful for protein purification and detection. By antibody, we include constructions using the binding (variable) region of such an antibody, and other antibody modifications. Thus, an antibody useful in the invention may comprise a whole antibody, an antibody fragment, a polyfunctional antibody aggregate, or in general a substance comprising one or more specific binding sites from an antibody. The antibody fragment may be a fragment such as an Fv, Fab or F(ab')₂ fragment or a derivative thereof, such as a single chain Fv fragment. The antibody or antibody fragment may be non-recombinant, recombinant or humanized. The antibody may be of an immunoglobulin isotype, e.g., IgG, IgM, and so forth. In addition, an aggregate, polymer, derivative and conjugate of an immunoglobulin or a fragment thereof can be used where appropriate.

Although a protein product (or fragment or oligopeptide thereof) of a gene of interest of the invention that is useful for the production of antibodies does not require biological activity, it must be antigenic. Peptides used to induce specific antibodies may have an amino acid sequence consisting of at least five amino acids and preferably at least 10 amino acids. Preferably, they should be identical to a region of the natural protein and may contain the entire amino acid sequence of a small, naturally occurring molecule. Short stretches of amino acids corresponding to species specific epitopes of the contraceptive proteins of the invention may be fused with amino acids from another protein (i.e., a mucosal targeting protein) such as LT-B, and antibody will be produced against the chimeric molecule. Procedures well known in the art can be used for the production of antibodies to the proteins of interest of the invention.

For the production of antibodies, various hosts including goats, rabbits, rats, mice etc... may be immunized by injection with the protein products (or a portion, fragment, or oligonucleotide thereof which retains immunogenic properties) of the genes of interest of the invention. Depending on the host species, various adjuvants may be used to increase the immunological response. Such adjuvants include but are not limited to Freund's, mineral gels such as aluminum hydroxide, and surface active substances such as lysolecithin, pluronic polyols, polyanions, peptides, oil emulsions, keyhole limpet hemocyanin, and dinitrophenol. BCG (bacilli Calmette-Guerin) and Corynebacterium parvum are potentially useful human adjuvants.

### a. Polyclonal antibodies.

The antigen protein may be conjugated to a conventional carrier in order to increase its immunogenicity, and an antiserum to the peptide-carrier conjugate will be raised. Coupling of a peptide to a carrier protein and immunizations may be performed as described (Dymecki et al., 1992, J. Biol. Chem., 267: 4815). The serum can be titered against protein antigen by ELISA (below) or alternatively by dot or spot blotting (Boersma and Van Leeuwen, 1994, J. Neurosci. Methods, 51: 317). At the same time, the antiserum may be used in tissue sections prepared as described. A useful serum will react strongly with the appropriate peptides by ELISA, for example, following the procedures of Green et al., 1982, Cell, 28: 477.

### b. Monoclonal antibodies.

Techniques for preparing monoclonal antibodies are well known, and monoclonal antibodies may be prepared using a candidate antigen whose level is to be measured or which is to be either inactivated or affinity-purified, preferably bound to a carrier, as described by Arnheiter et al., 1981, Nature, 294;278.

Monoclonal antibodies are typically obtained from hybridoma tissue cultures or from ascites fluid obtained from animals into which the hybridoma tissue was introduced.

Monoclonal antibody-producing hybridomas (or polyclonal sera) can be screened for antibody binding to the target protein.

### 2. Antibody Detection Methods

Particularly preferred immunological tests rely on the use of either monoclonal or polyclonal antibodies and include enzyme-linked immunoassays (ELISA), immunoblotting, immunohistochemistry and immunoprecipitation (see Humason, G.L., 1979, Animal Tissue Techniques, 4th ed. W.H. Freeman & Co., San Francisco, CA; Voller, 1978, Diagnostic Horizons, 2:1, Microbiological Associates Quarterly Publication, Walkersville, MD; Voller et al., 1978, J. Clin. Pathol., 31: 507; U.S. Reissue Pat. No. 31,006; UK Patent 2,019,408; Butler, 1981, Methods Enzymol., 73: 482; Maggio, E. (ed.), 1980, Enzyme Immunoassay, CRC Press, Boca Raton, FL) or radioimmunoassays (RIA) (Weintraub, B., Principles of radioimmunoassays, Seventh Training Course on Radioligand Assay Techniques, The Endocrine Society, March 1986, pp. 1-5, 46-49 and 68-78). For analyzing plants for the presence or absence of a protein of interest according to the present invention, immunohistochemistry techniques may be used. It will be apparent to one skilled in the art that the antibody molecule may have to be labeled to facilitate easy detection of a target protein. Techniques for labeling antibody molecules are well known to those skilled in the art (see Harlow and Lane, 1989, Antibodies, Cold Spring Harbor Laboratory).

### Dosage and Administration of Transgenic Plant Material

The present invention provides a method of inducing contraception in an animal by administering to an animal a transgenic plant material which expresses a contraceptive protein, wherein the administration of the transgenic plant material induces a mucosal immune response to the contraceptive protein. Without being bound to a one particular theory, the present invention relies on administration of transgenic plant material to an animal, such that, the contraceptive proteins expressed by the transgenic plant cells contact one or more mucosal surfaces of the animal to which it is administered. When the contraceptive protein (antigen) is taken up by M cells overlying the GALT and BALT, a generalized mucosal immunity results with secretory IgA against the contraceptive protein being produced by all secretory tissues in the body (Cebra et al., supra; Bienenstock et al., supra; Weinz-Carrington et al., supra; McCaughan et al., supra). Oral administration, therefore, is a preferred route to stimulate a generalized mucosal immune response and, in addition, lead to local stimulation of a secretory immune response in the oral cavity and in the gastrointestinal tract.

In a preferred embodiment, the contraceptive protein produced by the methods of the present invention is administered by the oral consumption of the foodstuff which has been produced from the transgenic plant or plant cell culture producing the contraceptive protein. In one embodiment, the edible portion of a transgenic plant of the present invention is administered as a dietary component while the contraceptive protein is administered in the process.

In one embodiment, the transgenic plant material is administered to an animal in raw form, although the plant material may be processed by cutting, chopping, mincing, dicing, pureeing, or otherwise reducing the transgenic plant material to smaller pieces so to facilitate ingestion by an animal, such as by the reduction of transgenic grasses to silage for ingestion by livestock. Transgenic plant material which may be administered in raw form include, but is not limited to fruit, leaves, stems, roots, tubers, and seeds. In a further embodiment, wherein the transgenic plant material is in the form of grasses or grains, the plant may be administered to an animal by permitting an animal to which the contraceptive protein is to be administered to orally consume the plant material in the environment in which it is growing (e.g., transgenic grass is grown in a field in which cows are permitted to graze, thus administering the contraceptive protein to the cow).

In an alternative embodiment, the transgenic plant material may undergo additional processing (i.e., other than chopping or cutting) prior to administration to an animal. Transgenic plant material may be processed by a technique known to those of skill in the art by which the transgenic plant material of the present invention is refined for oral, nasal, or inhaled application. The plant material may be processed by emulsification with excipients which are pharmaceutically acceptable and compatible with the antigen. Suitable excipients include, but are not limited to, adjuvants, buffers, sugars, antioxidents, stabalizers, or antigenic powders; for example, sodium ascorbate, Quillaja extract, water, saline, dextrose, glucose, sucrose, glycerol, ethanol, or the like and combinations thereof. In addition, if desired, the vaccine may contain amounts of auxiliary substances such as wetting or emulsifying agents, pH buffering agents, or adjuvants which enhance the effectiveness of the vaccines. Further, the plant material may be mixed with non-transgenic plant or non-plant material to facilitate ingestion by an animal. For example, transgenic corn may be mixed with other vegetables, or transgenic soy plants may be mixed with other ingredients such as spices, or other flavorings to produce, for example soy burgers, soy shakes, or soy milk. For further example, transgenic tobacco cultures may be filtered and added to regular feed materials by means such as but not limited to mixing, spraying, or top-coating. In addition, the transgenic plant material may be dried and administered in powdered form either alone, or as a suspension in an acceptable liquid such as juice, or water.

In a further embodiment, the plant material may be processed so as to incorporate the plant material and/or cells in pharmaceutically acceptable creams, ointments, salves, or suppositories. Accordingly, transgenic plant material carried with a pharmaceutical means may be administered to an animal by contacting the pharmaceutically carried plant material to a mucosal surface including, but not limited to oral, nasal, rectal or vaginal. Additional mucosal surfaces which may be contacted with transgenic plant material of the present invention include the inner coat of the bronchi, the mucous layer of the tympanic cavity, the inner mucous coat of the colon, the inner layer of the ductus deferens, the inner coat of the esophagus, the mucous coat of the small intestine, the mucous coat of the larynx, the mucous membrane of the tongue, the pituitary membrane, the mucous membrane of the oral cavity, the mucous membrane of the pharynx, the inner mucous layer of the trachea, the lining of the auditory tube, the mucous layer of the uterine tube, the inner layer of the ureter, the inner layer of the urethra, the endometrium, the mucous membrane of the vagina, the mucous layer of the stomach, the inner coat of the urinary bladder, and the mucous membrane of the seminal vesicle.

### Dosage

Transgenic plant material of the present invention may be administered to an animal in a number of different forms as described above (e.g., raw, powdered, etc.). In preferred embodiments, transgenic plant material useful in the present invention will express at least 8 µg of contraceptive protein, or a contraceptive protein/mucosal targeting protein fusion per gram of dry plant material, preferably at least 10 µg, and most preferably at least 20 µg of contraceptive protein per gram of dry plant material.

In one embodiment, animals housed in a controlled environment, such as a laboratory, kennel, or private home, and animals housed outdoors, but whose food intake is regulated (e.g., livestock) are fed transgenic plant material at weekly intervals for at least 12 weeks (i.e., days 0, 7, 14, 21, 28, 35, 42, 49, 56, 63, 70, 77,). At each feeding, animals receive between 4 and 100,000 grams of transgenic plant material depending on the size and average food intake of the animal to be treated (e.g., the average cow consumes approximately 90 pounds of food per day).

In an alternate embodiment, captive animal populations as described above are fed transgenic plant material on a daily basis (eg. days 0,1,2,3,4,5,6) to achieve primary contraceptive vaccination, and then fed transgenic plant material on a regular boosting schedule (eg. monthly) to maintain a sufficient contraceptive immune state.

In an alternate embodiment, delivery of trangenic plant material is used to augment parenteral delivery of contraceptive preparations. For example, a contraceptive preparation (produced through systems such as plants, bacterial yeast, or mammalian cells) may be delivered by veterinarian to a companion animal at the onset of puberty, and boosted on a regular schedule (eg. monthly) with a prescribed contraceptive feed derived from transgenic plant materials described herein.

In an alternate embodiment for human application, transgenic plant materials may be formulated into capsules or tablets and incorporated into a self-administered regime, whereby contraceptive and non-contraceptive tablets or capsules are packaged such that dosing and non-dosing occurs on alternate days.

In an alternate embodiment, animals in the wild may be administered transgenic plant material in the form of food baits which are placed in the animals natural environment. Best methods for delivery of baits, and modelling of population effects per application, are available to those skilled in the art of wild population control, and will determine the best specific baiting regime for each target species. Alternatively, transgenic plants which express the contraceptive proteins of the present invention may be allowed to grow wild in areas which are inhabited by animals to which the contraceptive protein is to be administered. Plants may be examined using the techniques described herein, to measure the amount of contraceptive protein expressed by the plant. In preferred embodiments, transgenic plant growing in the wild will express at least 8 µg of contraceptive protein, or a contraceptive protein/mucosal targeting protein fusion per gram of dry plant material, preferably at least 10 µg, and most preferably at least 20 µg of contraceptive protein per gram of dry plant material. Under these conditions, wild animals would be permitted to feed *ad libitum* on the transgenic plant material.

### Adjuvant

It is well known in the art that adjuvants can be added with an immunological antigen to increase the antigenic response (Coligan LG et al. eds. Current Protocols in Immunology, New York: John Wiley & Sons, 1995). Freund's Complete Adjuvant has been the mainstay of immunological adjuvants for decades. While usually effective, the adjuvant may induce undesirable side effects which has lead to refinement of its use and to development of alternatives.

Other alternative adjuvants include, but are not limited to, Ribi Adjuvant System (RAS) which is a oil-in-water emulsion containing detoxified endotoxin (MPL) and mycobacterial cell wall components (TDW, CWS) in 2% squalene (Ribi Immunochem Research, Inc. Hamilton, Montana); TiterMax, a stable, metabolizable water-in-oil adjuvant (CytRx Corporation, Atlanta Norcross, Georgia); Syntex Adjuvant Formulation (SAF)³, a performed oil-in-water emulsion stabilized by Tween 80 and pluronic polyoxyethlene/polyoxypropylene block copolymer L121 (Chiron Corporation, Emeryville, California); Freund's Incomplete Adjuvant (FIA), a less inflammatory alternative of Freund's complete adjuvant (Novavax Inc., Columbia, Maryland); ALUM - aluminum hydroxide, a widely used adjuvant, especially in commercial products such as vaccines (commercially available as Alhydrogel, Accurate Chemical & Scientific Co, Westbury, New York); SuperCarrier (Syntex Research, Palo Alto, CA); Elvax 40W (DuPont Chemical Co. Wilmington, DE); Montanide, a manide-oleate compound (ISA Seppic Fairfield, NJ); Nitrocellulose-absorbed protein (Nilsson BO, Larsson A. Inert carriers for immunization. Res. Immunol. 143:553-557, 1992); Gerbu adjuvant, (C-C Biotech, Poway, California); Immune-stimulating complexes (ISCOMS) (Coligan LG et al. eds. Current Protocols in Immunology, New York: John Wiley & Sons, 1995).

Saponin adjuvants have been used for many years for parenteral vaccination (products include "Quil A" or "QS-21"). Saponins are glycosidic compounds that are produced as secondary metabolites. They are widely distributed among higher plants and in some marine invertebrates of the phylum Echinodermata (ApSimon et al., Stud. Org. Chem. 17:273-286 (1984)). Because of their antimicrobial activity, plant saponins are effective chemical defenses against microorganisms, particularly fungi (Price et al., CRC Crit. Rev. Food Sci. Nutr. 26:27-135 (1987)). Saponins are responsible for the toxic properties of many marine invertebrates (ApSimon et al., Stud. Org. Chem. 17:273-286 (1984)). The chemical structure of saponins imparts a wide range of pharmacological and biological activities, including some potent and efficacious immunological activity. In addition, members of this family of compounds have foaming properties (an identifying characteristic), surfactant properties (which are responsible for their hemolytic activity), cholesterol-binding, fungitoxic, molluscicidal, contraceptive, growth-retarding, expectorant, antiinflammatory, analgesic, antiviral, cardiovascular, enzyme-inhibitory, and antitumor activities [Hostettmann, K., et al., Methods Plant Biochem. 7:435-471(1991); Lacaille-Dubois, M. A. & Wagner, H., Phytomedicine 2:363-386 (1996); Price, K. R., et al., CRC Crit. Rev. Food Sci. Nutr. 26:27-135 (1987)].

In addition, solutions of saponin have been employed as detergents to increase the delivery of polypeptides across mucosal membranes. For example, Pillion, D. J. et al., Invest. Opthal. Vis. Sci. 32:3021-27 (1991) disclose that administration of insulin in eyedrops containing unpurified saponin from Gypsophilla as a 1% solution causes a rapid and reproducible reduction in the blood levels of D-glucose in rats. Insulin eyedrops lacking saponin were ineffective. Japanese Abstract No. JP 62126135 (1987) discloses the nasal administration of growth hormone releasing factor employing a saponin having a steroidal or triterpene structure. See also, Chiou, G. C. Y. et al., J. Pharm. Sci. 78:815-818 (1989); and Chiou G. C. Y. et al., J. Ocul. Pharm. 5:81-91 (1989) who disclose the systemic delivery of insulin by its administration as a component of eyedrops containing saponin (obtained from Sigma Chemical Company). This detergent property of Saponin may also facilitate the permeability of biomembrane, therefore increase the immune response.

Saponin adjuvants from the bark of the Quillaja saponaria Molina tree (Quillajasaponins) are chemically and immunologically well-characterized products (Dalsgaard, K. Arch. Gesamte Virusforsch. 44:243 (1974); Dalsgaard, K., Acta Vet. Scand. 19 (Suppl. 69):1 (1978); Higuchi, R. et al., Phytochemistry 26:229 (1987); ibid. 26:2357 (1987); ibid. 27:1168 (1988); Kensil, C. et al., J. Immunol. 146:431 (1991); Kensil et al., U.S. Pat. No. 5,057,540 (1991); Kensil et al., Vaccines 92:35 (1992); Bomford, R. et al., Vaccine 10:572 (1992); and Kensil, C. et al., U.S. Pat. No. 5,273,965 (1993)).

Saponin also exists in other plant such as soy bean (Andrzejewska E., 1984, Rocz Panstw Zakl Hig, 35:135-8).

Quillaja saponins are found as a mixture of about twenty structurally closely related triterpenoid glycosides with minimal differences between them (Higuchi, R. et al., Phytochemistry 26:229 (1987); ibid., 26:2357 (1987); ibid., 27:1169 (1988); Kensil et al., U.S. Pat. No. 5,057,540 (1991); Kensil et al., Vaccines 92:35 (1992)), making their separation difficult. Quilliaja Saponaria extracts are commercially available (e.g., Garuda International, Inc. Lemon Cove, CA 93244). U.S. Patent Nos 5,273,965 and 5,650,398 disclose crude Saponin extracts and U.S. Patent No. 5,057,540 discloses a substantially pure Saponin extract. The extraction processes for both crude and pure Saponin include purifying the compound through chromatography.

A useful saponin adjuvant, according to the invention, includes crude and pure saponin extract (Garuda International, Inc. Lemon Cove, CA 93244). Useful saponin adjuvant also includes part of (e.g., leaf, root, fruit, or other parts) or processed plant containing saponin. By "processed", it refers to the cutting, chopping, mincing, dicing, or an otherwise reduction to smaller pieces of the saponin-containing plant, so to facilitate ingestion by an animal.

In a preferred embodiment, saponin adjuvant is orally administered with the transgenic plant material.

### Detecting Immunization

The present invention provides a means of inducing contraception in an animal by administering a contraceptive protein to which a mucosal immune response is raised. In a preferred embodiment, following administration of transgenic plant material bearing contraceptive proteins of the present invention, animals are examined to determine whether an immune response to the contraceptive protein has been raised. Animals may be examined for an immune response to the contraceptive protein at a time following administration of the plant material.

The following describes the assessment of ZP antigens, however, the techniques described may be used to an animal's immune status with respect to a of the contraceptive proteins described herein. In one embodiment, one of skill in the art can determine whether an animal has been immunized is by determining the animal's immune status with respect to ZP antigens. This evaluation can be done by assaying the titer of ZP-binding antibodies in a sample of, for example, blood, serum, urine, saliva, teardrop, etc. Most preferably, an enzyme-linked immunosorbent assay ("ELISA") that is capable of detecting antibodies to the ZP immunogen or its equivalent may be used for this purpose (Drell, et al., Biol. Reprod. 30:445 (1984); ELISA and Other Solid Phase Immunoassays (Kemeny, D. M. et al., Eds.), John Wiley & Sons, N.Y. (1988)).

As will be understood from the well-known principles of immunoassays, alternative formats, such as immunometric assays (also known as a "two-site" or "sandwich" assays), including both "forward," "simultaneous" and "reverse" assays may be employed (Fackrell, J. Clin. Immunoassay 8:213-219 (1985); Yolken, R. H., Rev. Infect. Dis. 4:35 (1982); Collins, W. P., In: Alternative Immunoassays. John Wiley & Sons, N.Y. (1985); Ngo, T. T. et al., In: Enzyme Mediated Immunoassay, Plenum Press, N.Y. (1985)).

For example, in a "forward" assay, ZP antigens are bound to a solid support (such as a microtiter plate, test tube, dipstick, etc.), and then first contacted with the sample being evaluated for the presence of anti-ZP antibody under conditions that permit the formation of a binary solid phase ZP-antibody complex. After incubation and washing, the support is placed in contact with a quantity of labeled ZP antigen (which functions as a "reporter molecule"). After a second incubation period to permit the labeled ZP antigen to complex with the immobilized ZP through the unlabeled antibody, the solid support is washed a second time to remove the unreacted labeled ZP antigen. This type of forward sandwich assay may be a simple "yes/no" assay to determine whether anti-ZP antibodies are present or may be made quantitative by comparing the amount of retained labeled ZP antigen with that obtained for a standard containing known quantities of anti-ZP antibody. "Two-site" or "sandwich" assays are described by Wide, In: Radioimmune Assay Method, (Kirkham et al., Ed.), E. & S. Livingstone, Edinburgh, pp. 199-206 (1970)).

In a preferred embodiment, the forward ELISA assay may be used to measure a mucosal immune response. The assay is conducted as described above, with the modification that following incubation with the sample being evaluated for musosal immunoglobulins, the support is place in contact with labeled anti-IgA antibodies, which function as reporters of the presence of IgA in the sample obtained from the animal.

In a "simultaneous" assay, a single incubations step is employed in which the bound ZP and the labeled ZP are both added to the sample being tested at the same time. After the incubation is completed, the solid support is washed to remove the residue of fluid sample and uncomplexed labeled antibody. The presence of labeled antibody associated with the solid support is then determined as it would be in a conventional "forward" sandwich assay.

In a "reverse" assay, a solution of labeled ZP is incubated with the sample, and the reaction is then placed in contact with a solid support to which unlabeled ZP has been previously bound. After a second incubation, the solid phase is washed in a conventional fashion to free it from the residue of the sample being tested and the solution of unreacted labeled ZP. The determination of antibody titer is determined as in the "simultaneous" and "forward" assays.

In its most preferred embodiment, the ELISA of the present invention employs a monoclonal antibody. Most preferably, such antibodies are generated, as described above, by immunizing a heterologous animal (such as a mouse, rat, rabbit, etc.) with a ZP antigen, and then harvesting the splenic leukocytes of the animal, and fusing them with a suitable myeloma cell, in the manner described above.

Although the immunoassay has been described with respect to a particular preferred solid support, a of a variety of alternative solid supports may be employed. Suitable solid supports may be composed, for example, of materials such as glass, paper, polystyrene, polypropylene, polyethylene, dextran, nylon, amylases, natural and modified celluloses, polyacrylamides, agaroses, or magnetite. The nature of the support can be either soluble to some extent or insoluble for the purposes of the present invention. The support material may have virtually a possible structural configuration so long as the ZP molecules are capable of binding to antibody. Thus, the support configuration may be spherical, as in a bead, or cylindrical, as in the inside surface of a test tube, or the external surface of a rod. Alternatively, the surface may be flat such as sheet, test strip, etc. Those skilled in the art will note ma other suitable carriers for binding monoclonal antibody, or will be able to ascertain the same by use of routine experimentation.

In a further embodiment, the immune status of an animal to which transgenic plant material of the present invention has been administered may be evaluated by immunohistochemical techniques. Briefly, following administration of transgenic plant material by a of the routes of administration described above, female animals may be sacrificed and the ovaries removed. As fertilization occurs in the fallopian tube following release of the ovum from the ovary, it is preferred that the presence of antibodies against contraceptive proteins of the invention be detected on ovarian ova. Following removal from the animal, the ovaries are frozen an cryosectioned. Sections are fixed in a fixative known in the art including, but not limited to formaldehyde, acetone, paraformaldehyde, and glutaraldehyde. Sections are subsequently washed and incubated with antibodies which bind to the antibodies raised by the animal in response to the contraceptive protein. The sections are further incubated with a secondary antibody comprising a reporter molecule (e.g., a fluorescent dye), and examined under a microscope. Identification of antibodies directed against the contraceptive protein expressed by the administered transgenic plant is indicative of an immune response having been raised by the animal against the contraceptive protein. For example, if transgenic plant material of the present invention is administered to a mouse, a mucosal immune response to the contraceptive protein expressed by the plant may be measured by obtaining tissue sections of the mouse ovary, and treating the tissue with antibodies directed against the kappa chain of the mouse immunoglobulin. The anti-murine kappa chain antibody localization may be visualized by treating the tissue with a secondary antibody conjugated to a reporter, such as a fluorescent dye. The tissue may then be examined under a microscope to visually localize the labeled antibodies, and the image of the antibody labeling quantitated using a morphometric computer software known to those of skill in the art, such as NIH Image (National Institutes of Health, Bethesda, MD). The quantitative data is then compared statistically to similar antibody labeling data obtained from a mouse that has not been treated with a contraceptive protein of the invention. A statistically significant difference between the animals is indicative of immunization of the mouse with the contraceptive protein, wherein the P value for the statistical test is at least <0.1, <0.05, <0.01, <0.005, 0.001, 0.0005, and preferably <0.0001.

### Determining Contraceptive Effect

In the most preferred embodiment, the contraceptive effect of the administered contraceptive proteins of the present invention is determined by evaluating the number of offspring produced per breeding cycle by a female animal to which was admininstered transgenic plant material according to the methods of the present invention. For example, following 5 to 6 weeks of administration of transgenic plant material to, for example, a mouse, individual female mice are placed in cages with a male mouse. Male mice are then removed after between 10 and 20 days. Female mice are then observed daily for birth of pups. The contraceptive effect of the administered contraceptive protein is determined by the reduction in litter size following administration. For example, for animal species which produce average litters of 3 offspring or more (e.g., mice), the contraceptive protein can be considered effective if there is a reduction in litter size by 10% to 100%, 30% to 90%, or 60% to 80%. For animal species which produce average litters of 1 or 2 offspring (e.g., white tailed deer) a contraceptive protein is considered effective if there is a reduction in litter size by at least 50%, preferably 100%.

Alternatively, the effectiveness of the contraceptive proteins of the present invention may be determined by measuring the levels of antibodies against the contraceptive protein in the blood, serum, mucosal secretions, excrement, etc., as described above. The effectiveness of the contraceptive proteins may also be evaluated by visualizing antibodies against the contraceptive protein bound to the ovum by immunohistochemical techniques as described above. However, to accurately determine the effectiveness of a contraceptive protein according to the invention, the measurement or visualization of antibodies against the contraceptive protein must be evaluated in conjunction with an observed reduction in litter size. For example, a contraceptive protein may be considered not effective regardless of the detection of antibodies against the protein in an animal's serum if there is no reduction in fecundity.

### EXAMPLES

### Example 1. Fusion of a plant-optimized mouse ZP3 epitope to a plant-optimized synthetic LT-B gene

An EcoRV and KpnI fragment from TH210 (Mason at al., (1998) Vaccine 16: 1336) containing a synthetic LTB sequence was inserted into the corresponding sites of pBluescript (Stratagene, La Jola, CA, USA) to make pBlueLTB. The plasmid pLTB-L was created by using a unique BbsI site to fuse the coding region of a 6 amino acid linker (translational) to the 3' end of the LTB coding region within pBlueLTB. The linker was constructed using two commercially synthesized oligomers 5' AAC TCT GAT CCA CAT GTT CCT (SEQ ID NO: 1) and 5' AGT TAG GAA CAT GTG GAT CAG (SEQ ID NO: 2) that had been purified by electrophoresis on a denaturing polyacrylamide gel. The oligomers were phosphorylized with T4 kinase in separate reactions, mixed in equimolar amounts and annealed by heating to 90°C for 5 minutes before being gradually cooled to 23°C over an hour. The plasmid pLTB-L was prepared for insertion of the mouse ZP3 epitope by serial digestion with BbsI and KpnI and dephosphorylation with calf intestinal phosphatase (CIP).

The coding sequence of the mouse ZP3 (336-342) epitope was analyzed for its codon usage compared with plant genes (Wada et al., 1990 Nucleic Acid Research 18: 2367). A plant-optimized epitope was designed maintaining the amino acid sequence of the native epitope with additional nucleotides at the 5' and 3' ends that, upon assembly of the oligos, make a BbsI and KpnI compatible fragment. The oligonucleotides MZepA 5' AAC TTC CAA ATT CAT GGA CCA AGA AAC TAA GTC TTC GGT AC (SEQ ID NO: 3) and MzepB 5' CGA AGA CTT AGT TTC TTG GTC CAT GAA TTT GGA (SEQ ID NO: 4) were synthesized and purified by electrophoresis on a denaturing polyacrylamide gel. The epitope was assembled as previously described in the construction of pLTB-L. The epitope was placed on ice before ligation into a BbsI, KpnI fragment pLTB-L making pLTBL7. The plasmids pLTB-L and pLTB7 were sequenced by the dideoxy chain termination method. The authors obtained an NcoI and KpnI fragment of pLTB7 and inserted it into pIBT210 (Haq at al., 1995 Science 268: 714) digested with NcoI and KpnI to make pAW7. The expression cassette was purified from pAW7 after digestion with HindIII and EcoRI and ligated with pGPTV.kan (Becker et al., Plant Molecular Biology 20: 1195) digested with HindIII and EcoRI to give pAWBin7.

### Example 2. Tomato Transformation

pAWBin7 prepared from cultures of E. coli DH5∝ was electroporated into *Agrobacterium tumefaciens* EHA105. *Agrobacterium*-mediated transformation of tomato cotyledons (variety Tanksley TA234TM2R) was performed as per Frary and Earle (Frary and Earle, 1996 Plant Cell Reports 16: 235) except seeds were sterilized by soaking in 70% ethanol for two minutes before rinsing in sterile water and washing in a mixture of 10% domestos and 1% Tween-20 for two hours. The seeds were rinsed three times in sterile distilled water before plating on half strength Murashige and Skoog medium (half strength MS salts (Murashige and Skoog, 1962 Physiologia Plantarum 15: 473), 50 mg/l myo-inositol, 2 mg/l thiamine HCl, 0.5 mg/l pyridoxine HCL, 0.5 mg/l nicotinic acid, 10 g/l sucrose and 8 g/l difco bacto agar, pH 5.8). Plantlets were regenerated on medium containing kanamycin at 300 mg/l. Individual lines were screened by ganglioside-dependent ELISA for LTB expression in leaves and fruit. The lines with best fruit expression were self-pollinated and the resulting seeds germinated on MS medium supplemented with 300 mg/l kanamycin. Surviving seedlings were transferred to soil and the glasshouse and self-pollinated. Fruit from each T1 plant were freeze-dried, powdered, pooled, and stored in a dry environment.

### Example 3. Protein Extraction and ELISA

Leaf samples and fresh fruit from greenhouse grown plants or dried fruit samples were homogenized in 2 (fresh samples) or 50 (dried fruit) ml/l (respectively) of ice cold extraction buffer (50 mM sodium phosphate, pH 6.6, 100 mM NaCl, 1 mM EDTA, 0.1% Trition X-100, 10 µg/ml leupeptin, 1 mM PMSF) in a Bio 101 Fast prep machine. Insoluble materials were pelleted by centrifugation at 14 000 rpm in an Eppendorf 5415C microcentrifuge at 4°C for 5 minutes. The supernatant was kept on ice during analysis and stored at -80°C. Total protein concentration of the plant samples was determined using Coomassie dye binding assay (Bio Rad), using bovine serum albumin (BSA) as a standard. Ganglioside-dependent ELISA was performed on two samples of 1 in 100 dilutions of each extract as described (Haq et al., *supra*) (Figure 1 a, b,c).

### Example 4. Fusion protein purification and detection

Dried fruit samples from T1 plants expressing over 8 µg LTB per gram dry weight were pooled. An extract from the dried samples was made as previously described except the material was homogenized in 20 ml/g ice cold extraction buffer. The resulting supernatant was incubated with PBS equilibrated sephadex on a platform rocker at 4°C overnight. The sephadex solution was allowed to settle and a 1 ml sample collected and stored at 4°C. The volume of the sephadex solution was reduced using a sintered glass funnel and flushed with 60 ml ice cold PBS. A sephadex slurry was made with 5 ml PBS and the air removed via a vacuum dessicator before being poured into a Bio Rad X column. The column was washed with 50 ml PBS with 1 ml samples collected from the first and fifth milliliter run through. The fusion protein was eluted using 0.2 M D-(+)-galactose in PBS and stored at 4°C. Samples were later analyzed using SDS-PAGE and comassie blue staining. A band corresponding to the fusion protein was detected on SDS-PAGE gel. The presence of the fusion protein was also confirmed by ELISA as described in Example 3.

### Example 5. Isolation of Genomic Nucleic Acid

Genomic nucleic acid was isolated from young leaf tissue of T1 plants by grinding 200-250 mg of material in a 1.5 ml microcentrifuge tube using liquid nitrogen and a nail. The powder was resuspended in 500 µl of extraction buffer (420 g/l urea, 312.5 mM NaCl, 50 mM Tris.HCl, pH 8.0, 20 mM EDTA, 1% sarcocine) and incubated for 30 minutes with 500 µl of phenol:chloroform:isoamyl alcohol (25:24:1) on a sample rotator. The resulting slurry was centrifuged for 15 minutes at 14 000 rpm, 4°C. The upper aqueous phase was collected and RNA precipitated through addition of equal volume of 4 M LiCl and incubation at -20°C for at least 8 hours. Samples were centrifuged at 15000 rpm for 15 minutes at 4°C and the supernatant removed to separate tubes. The RNA pellet was resuspended in 50 µl Rnase free sterile water. Genomic DNA was precipitated from the saved supernatant through addition of 10% volume 7.5 M ammonium acetate, 1 volume ice-cold isopropanol and incubation at -20°C for at least 8 hours. The DNA was washed with 70% ice cold ethanol, allowed to air dry for 5 minutes and resuspended in 50 µl sterile water + RNase (50 µg/ml).

### Example 6. Southern Analysis

Southern blot analysis was performed to confirm the chromosomal integration of the fusion DNA.

Samples containing 15 µg DNA, were digested with 3.4 units of HindIII per µg DNA in an overnight reaction at 37°C. Digested samples were run overnight in a 1% TAE agarose gel. The gel was depurinated (0.25 M HCl) and transferred to zeta probe membrane (Bio Rad) following the manufacturer's recommendations for alkaline transfer. The DNA was fixed by UV cross-linkage.

A PCR labeled probe was made using the primer set, TEV (5'-GCA TTC TAC TTC TAT TGC AGC) (SEQ ID NO: 5) and VSP (5'-GTC CAT ATC AGC ATA C) (SEQ ID NO: 6) on a pAW7 template. DIG labeled dCTP was incorporated into the 588 bp amplicon as per manufacturer's instructions (PCR DIG Probe Synthesis kit, Boehringer Mannheim). The amplifications were performed over 32 cycles using a Hybaid PCR Express Thermo-Cycler. The template was initially melted at 94°C for 4 minutes followed by 30 cycles of 94°C for 15 seconds, 55°C for 30 seconds, and 72°C for 90 seconds. A final extension step was performed at 72°C for 5 minutes before soaking at 4°C.

Hybridization bottles and 10 ml DIG Easy Hyb (Boehringer Mannheim) per membrane were pre-warmed to 45°C in a hybridization oven. Membranes were pre-hybridized for at least 90 minutes and hybridized overnight with a probe concentration of 5 µl/ml DIG Easy Hyb. Post hybridization washes and detection were performed as per manufacturer's instructions (Boehringer Mannheim - DIG wash and block buffer set and DIG Luminescent Detection Kit). Labeled membranes were visualized after exposure to film. The integration of fusion DNA into plant host chromosome was confirmed.

### Example 7. Northern Analysis

Northern blot analysis was used to confirm the expression of the fusion protein at mRNA level.

Northern analysis was performed as described in Molecular Cloning [Sambrook et al. *supra*]. Briefly, genomic RNA samples were denatured with formaldehyde / formamide and run for two hours in a 1% agarose, MOPS-acetate-EDTA gel. RNA was then transferred to zeta probe membrane (Bio Rad) by upward capillary action and fixed by UV cross-linkage. Membrane hybridization and detection were performed as with Southern analysis except hybridization steps were performed at 42°C. The presence of fusion protein mRNA was confirmed.

### Example 8. Mouse feeding and sample collection

The efficacy of contraception effect of transgenic plant was tested in vivo using laboratory mice.

Mice were housed as male / female pairs while mice were caged three females to one male. The number of pups per female was noted throughout the experiment.

Freeze-dried, powdered fruit from tomato plants expressing over 8 µg LTB per gram of dry mass were pooled and mixed. For each feeding, the tomato powder was mixed 1:2 (weight per volume) with apple cider. Feedings were performed at fortnightly intervals for 12 weeks (days 0, 3, 14, 17, 28, 31, 42, 45, 56, 59, 70, 73, 84, 87). Animals were divided into 3 groups, 4 - 6 received 4 g of wild-type tomato plus apple cider; 4 - 5 received 4 g of transgenic tomato plus apple cider; 4 - 5 received 4 g of transgenic tomato plus apple cider plus 10 mg of Quillaja extract powder (Garuda). Starting at day 31, female mice were subjected to a fasting protocol in which at noon, they were removed to individual cages with water and bedding only. The test diet was given at 4pm and left overnight. At 8 am the following morning, females were returned to their regular cage.

On days 0 and 70, serum samples from tail bleeds were collected from each female and stored at -80°C for future antibody analysis. Final serum samples were collected on day 96 through cardiac puncture and stored as previously described. Fecal samples were collected on days 70 and 87 and frozen overnight at -80°C. The frozen pellet samples were lyophilized, resuspended in 0.8 ml phosphate buffered saline, pH 7.2 (PBS), centrifuged at 14 000 rpm for 5 minutes, and the supernatant stored at -20°C until assayed.

### Example 9. ELISAs for anti-LTB and anti-ZP3 epitope antibodies and fertility of experimental mice

Samples were assayed for anti-LTB and anti-ZP3 epitope IgG in serum and anti-LTB and anti-ZP3 epitope IgA in faecal pellets as described (Dickenson and Clements, 1995 Infect. Immun. 63: 1617). The results are shown in Figure 2 a and 2 b. Samples were serially diluted in PBS containing 0.05% Tween-20 (PBST). For detection of anti-LTB antibodies, microtitire plates were coated with mixed gangliosides (Type III), Sigma, St Louis, MO, USA, blocked with 0.25% BSA and incubated with purified recombinant LTB for one hour. For detection of anti-ZP3 epitope, microtitre plates were coated with recombinant epitope conjugated to BSA and blocked with 0.25% BSA. Serum anti-LTB or anti-ZP3 epitope was detected using rabbit antiserum against mouse or vole IgG and deer anti-rabbit antiserum conjugated with alkaline phosphatase (Sigma). Levels of fecal anti-LTB and anti-ZP3 epitope antibodies were determined with goat antiserum against mouse or vole immunoglobulins and rabbit anti-goat antiserum conjugated to alkaline phosphatase.

The effect of oral delivery of tested material were examined by comparing the number and size of litters from control and experimental group (Figure 3). Six mice were used for each group. The experimental groups of mice were fed with transgenic tomato paste. The effect of the test material on a non-target species (Voles) was also examined (Figure 4).

### Example 10. Production of Contraceptive Tobacco Suspension Cultures

The coding regions for a mouse ZP3 epitope, a possum ZP3 epitope and a general GnRH epitope were fused to LTB (constructs described below, Figure 5 a, b, c). The mouse and possum epitopes were individually fused to LTB so that they would be translated off the carboxyl terminus of LTB. Three fusions were made with the GnRH epitope. The GnRH epitope was fused to LTB so that it would be translated off the amino terminus, the carboy terminus or within the LTB monomer in a position predicted to sit on the exterior of an LTB pentamer. The coding region of these fusion proteins were then placed into the plant expression cassette of pIBT210.1 and inserted into the binary vector pGPTV.kan for *Agrobacterium*-mediated transformation. NT1 cells (tobacco cell suspension) were transformed with the mouse ZP3-LTB fusion, possum ZP3-LTB fusion, the GnRH carboxyl fusion and the GnRH internal fusion.

LTB specific and GnRH specific ELISA analysis of the mouse and GnRH fusion transformants was performed (Figure 6). Detection was confirmed for the carboxyl terminus fusion and LTB levels were low for the internal fusion. We concluded that the internal fusion was interfering with pentamer formation and hence detection. Although expression was low, the GnRH epitope could be expressed by plant cells.

### Example 11. Production of Transgenic Carrot

All transformations are using the carrot cultivar Nanco (Daucus carota L. cv. Nanco). Nanco was one of 3 cultivars examined by Gilbert et.al in 1996 (references below), and was identified as superior for frequency of transformation and speed of regeneration. The seed was obtained from a commercial seed supplier in Hemel Hempstead, England, and imported to the Boyce Thompson Institute for Plant Research (BTI). All seed were surface sterilised with bleach for 20 minutes, followed by 70% Ethanol for 20 minutes, prior to germination. A materials contaminated with additional bacterial or fungal infection during the germination, transformation or regeneration stages were destroyed. Carrot hypocotyls were excised 7-15 days following aseptic germination. Explants were transferred to liquid growth media, and co-cultivated with *Agrobacterium* strain EHA105 (containing the appropriate expression vectors, as described below) for 36-48 hours at room temperature, in darkness and under slow shaking conditions. Explants were then transferred to solid growth media, which contained additional plant auxin 2,4-D (to stimulate embryogenic callus production), the antibiotic Timentin (to cure all remaining traces of *Agrobacterium*), and stored in darkness at room temperature.

After five (5) weeks, the explants had developed significant callus and were transferred to freshly prepared solid growth media containing Kanamyacin to induce selective pressure against non-transformed cells. All tissues remained at room temperature and stored in darkness. After an additional 5 weeks, healthy callus was transferred to fresh solid media in the absence of 2,4-D and placed in well-lit conditions to induce plantlet regeneration. In addition, some callus was transferred to fresh media (containing 2,4-D) and returned to darkness to stimulate further callus production for regeneration at a later stage. All tissues which did not grow appropriately on Kanamycin selection (assumed non-transgenic), or showed signs of bacterial or fungal contamination were destroyed.

After approximately 10-14 weeks on regenerative/selective media, with ample lighting, carrot plantlets with initial roots and shoots could be transferred to controlled greenhouse growth conditions. Harvest of transgenic root materials was conducted 8-10 weeks after planting.

### Example 12. Production of Transgenic Carrot Suspension Cultures

2-5grams of healthy carrot callus selected from Kanamcin media (described above) was placed into 50 mls of liquid growth media. The standard liquid media contained 400ml water, 400uL Murashige & Skoog Vitamins, 1.7g Murashige & Skoog Salts, 12g sucrose, and was sterilized by autoclaving prior to adding final concentrations of 2,4-D (2.2mg/L) and the antibiotic Timentin to control microbial contamination (300mg/L). The mixture was then placed on an orbital shaker at ambient temperature, to shake at 100-120 rpm. The callus gradually broke apart into smaller individual cells or cell clumps. Every 7-10 days, 5ml of suspension culture was added to 45mls of fresh liquid growth media, to maintain healthy growth of cells.

### Example 13. Production of Powdered Carrot Cells

After 7-10 days growth, liquid suspension cultures were filtered onto filter paper, and frozen at -80°C for a minimum of 24 hours. Materials collected and frozen over a period of several weeks, were placed into lyophilization tubes, and attached to freeze drying apparatus for a minimum of 48 hours. Resultant material was weighed, crushed by hand, and stored at -20°C in air-tight plastic bags in alliquots of approximately 350g. The powdered material was highly anhydrous, such that it would stick to human skin upon contact. The powdered material had been reduced to less than 10% of fresh harvest weight. Samples of the powdered material were assayed by ELISA and PCR specific for the construct in use. Measurable antigen content was found to be within the range of 50-1 00ug per gram dry weight, an approximate 10-15 fold increase in the detectable antigen concentration.

### Example 14. Specific Constructs:

**pTH110** This construct encodes for the expression of the B subunit from the *E. coli* heat labile enterotoxin (LT-B). This construct has previously been expressed in potatoes for oral delivery to mice (Mason et.al., *supra*) and for human clinical trial in humans (Tacket et.al. 1998 Nature Med. 4: 607). This construct was expressed in potatoes, which were the subject of MAF Import Permit number 1999007035, and the subsequent oral delivery to possums at Landcare Research facilities in Lincoln, NZ. Carrots expressing LT-B will be used as negative control materials for immunological and fertility trials of contraceptive materials specified below.

The LT-B molecule forms a pentamer in native form (and in plant cells), providing a binding affinity for epithelial cells which line the mucosal surfaces of reproductive, respiratory and digestive tracts. This affinity has provided a strategy for targeting delivery of smaller proteins such as contraceptive epitopes. Ma of the following constructs encode genetic fusions between LT-B and immunocontraceptive peptides. Analysis of leaf and root materials by ELISA has shown that the resultant molecule is a pentamer, with co-display of the candidate peptides.

**pGPTV - MuZP3** In contrast to pTH110, this construct has an additional sequence inserted at the carboxyl terminus of the LT-B gene, prior to the VSP terminator. The additional sequence includes a linker peptide (Asn-Ser-Asp-Pro-His-Val-Pro) (SEQ ID NO: 7) and the minimal B-cell epitope, amino acid residues 335-342 (Asn-Phe-Gln-Ile-His-Gly-Pro-Arg) (SEQ ID NO:8), from the murine zona pellucida glycoprotein 3 (MuZP3).

**pGPTV - GNRH1** In contrast to pGPTV-MuZP3, this construct has sequence encoding the decapeptide for porcine gonadotrophin releasing hormone (GnRH), in place of the MuZP3 epitope. The linker is conserved. The sequence for this decapeptide (Gln-His-Trp-Ser-Tyr-Gly-Leu-Arg-Pro-Gly) (SEQ ID NO: 9) is common to most mammals.

**pGPTV - GNRH3** In contrast to pTH110, this construct has the GnRH decapeptide fused (via a linker) to the amino- terminus of the LT-B gene. A signal peptide is contained within the LT-B gene, and is cleaved during post-translational processing to leave the mature monomeric protein. This cleavage occurs between Tyrosine and Glycine (residues 20 & 21). The GnRH epitope has been inserted immediately following the Glycine residue. A short linker (Tyr-Ala-His-Gly) (SEQ ID NO: 10) is used to link the GnRH epitope to the amino-terminus of LT-B, maintaining the Glycine-Alanine starting sequence of the mature protein.

**pGPTV - pAW6** In contrast to pGPTV-GnRH1, this construct has sequence encoding a predicted B-cell epitope from Australian Brushtail Possum zona pellucida glycoprotein 3 (PossZP3), in place of the GnRH decapeptide. The epitope consists of 27 amino acids (334-361 from native sequence) and the linker is conserved.

**pGPTV - pAW4** In contrast to pTH110, the LT-B gene has been replaced by the full-length gene encoding for Brushtail Possum ZP3.

**pGPTV - pAW4-A2** In contrast to pGPTV-pAW4, this construct contains a truncated version of the PossZP3, where a putative cleavage peptide has been removed. Additional sequence has been inserted at the carboxyl terminus of the Brushtail Possum ZP3 gene, which encodes for a short linker (Gly-Pro-Gly-Pro) (SEQ ID NO: 11) and the A2 region from the *E. coli* heat labile enterotoxin subunit A (LT-A). Colonisation by enterotoxigenic *E. coli* results in the secretion of LT-A and LT-B. In their native state (and also in plant cells), they bind to each other to form a stable holotoxin. Whilst LT-B targets the molecule to epithelial linings, it is the toxic A subunit which causes ADP-ribosylation and subsequently diarrhoea. The LT-A subunit consists of two regions, A1 and A2. The region designated as A2 forms an alpha helix which extends from the A1 region downwards into the pentamer formed by LT-B, and forms the bond between the two subunits. The A1 protein is then anchored above the pentamer. In the case of pGPTV-pAW4-A2, when the plant cells are co-transformed with pTH110 (or the derivatives listed above) the A2 helix provides the anchor between the PossZP3 protein and an LT-B pentamer.

### Example 15. Mouse contraception experiment with carrot samples

Transgenic carrots comprising one of the constructs in Example 14 were produced as descibed in Example 11 and processed as in Example 12 and 13. Mice were housed as male / female pairs while mice were caged three females to one male. The number of pups per female was noted throughout the experiment.

Powdered carrot expressing over 50 µg LTB per gram of dry mass were pooled and mixed. For each feeding, the carrot powder was mixed 1:2 (weight per volume) with apple cider. Feedings were performed at fortnightly intervals for 12 weeks (days 0, 3, 14, 17, 28, 31, 42, 45, 56, 59, 70, 73, 84, 87). Animals were divided into 3 groups, 4 - 6 received 4 g of wild-type carrot plus apple cider; 4 - 5 received 4 g of transgenic tcarrot plus apple cider; 4 - 5 received 4 g of transgenic tomato plus apple cider plus 10 mg of Quillaja extract powder (Garuda).

Starting at day 31, female mice were subjected to a fasting protocol in which at noon, they were removed to individual cages with water and bedding only. The test diet was given at 4pm and left overnight. At 8 am the following morning, females were returned to their regular cage.

On days 0 and 70, serum samples from tail bleeds were collected from each female and stored at -80°C for future antibody analysis. Final serum samples were collected on day 96 through cardiac puncture and stored as previously described. Fecal samples were collected on days 70 and 87 and frozen overnight at -80°C. The frozen pellet samples were lyophilized, resuspended in 0.8 ml phosphate buffered saline, pH 7.2 (PBS), centrifuged at 14 000 rpm for 5 minutes, and the supernatant stored at -20°C until assayed.

### Example 16. Feeding mice with fresh plant materials

Fresh plants were also used to feed the experimental mice. Both tomato and carrot plant leaves or fresh tomato and carrot were used.

Fresh leaves or tomato and carrot corresponding to their dry powder form expressing over 50 µg LTB per gram of dry mass were pooled and mixed. In some cases, the fresh materials were chopped into small pieces. Feedings were performed at fortnightly intervals for 12 weeks (days 0, 3, 14, 17, 28, 31, 42, 45, 56, 59, 70, 73, 84, 87). Animals were divided into 3 groups, 4 - 6 received 4 g of wild-type tomato or carrot; 4 - 5 received 4 g of transgenic tomato or carrot; 4 - 5 received 4 g of transgenic tomato plus apple cider plus 10 mg of Quillaja extract powder (Garuda).

Starting at day 31, female mice were subjected to a fasting protocol in which at noon, they were removed to individual cages with water and bedding only. The test diet was given at 4pm and left overnight. At 8 am the following morning, females were returned to their regular cage.

On days 0 and 70, serum samples from tail bleeds were collected from each female and stored at -80°C for future antibody analysis. Final serum samples were collected on day 96 through cardiac puncture and stored as previously described. Fecal samples were collected on days 70 and 87 and frozen overnight at -80°C. The frozen pellet samples were lyophilized, resuspended in 0.8 ml phosphate buffered saline, pH 7.2 (PBS), centrifuged at 14 000 rpm for 5 minutes, and the supernatant stored at -20°C until assayed.

### Example 17. Reduction of antigen concentration variance in powder homogenates

The application of plant-derived vaccines is dependent on the ability to provide a consistent dose, and achievement of clinical data to support the minimal and maximal doses to be applied. Batch manufacturing and QC assurance can only be validated if the vaccine material provides consistent antigen concentration within an acceptable range. In three recent human clinical trials (Tacket et.al 1998, Tacket et.al 2000, Thanavala et.al., personal communication), human volunteers were fed raw, cubed, transgenic potato materials, expressing antigenic proteins of interest. The experimental doses in each of those trials were shown to vary by as much as 300%, as shown by assay results on randomized samples of consumed materials. Similar potato materials reduced to dehydrated powder by the method described herein resulted in equivalent in-vitro immunogenic reactivity (by appropriate assay detection) with up to a 10-fold reduction in variance across randomized samples. This result was duplicated in processed transgenic tomato material expressing the same antigen (HBsAg), and several other model plant species and antigens, as shown in Table 1.

In the examples, homogenization was achieved by either of two methods. The first method was applied prior to freeze drying and involves the crude pulping and mixing of freshly harvested plant materials, followed by freezing and subsequent freeze-drying of the blended materials. The first process of slicing, blending or grinding, reduced raw plant tissues (fruit, tubers, leaf, seed, etc) to a pulp mixture. This mixture was assumed to contain both intact plant cells and ground cell debris. The advantage of this process is the reduction of larger plant organs, such as fruit or tubers, to a manageable mixture whereby variance in homologous protein content between tissues is combined into a more uniform batch of material. The ability to provide a uniformed batch without fully disrupting all plant cell integrity provides the stable environment for homologous protein storage during the subsequent processing stages. The second method of homogenization required minimal processing to the plant materials prior to processing. Freeze-drying of sliced, quartered or intact materials was conducted as described above. The resultant materials were then hand-crushed to a fine powder, combined into a single batch container, and mixed thoroughly by mechanical means such as shaking or dry blending.

The removal of any pureeing step decreased labor intensity required for the overall process. The effect of pre-drying treatment (pureed, peeled, quartered, or sliced) was evaluated for its effect on overall drying time for potato. As displayed in Figure 7, reduction to full freeze dried state was limited only if potato materials were not peeled or cut in some manner (quartered, diced, sliced or pureed).

### Example 18. Potato tuber samples can be concentrated by up to 11 fold as a measure of dry weight versus wet weight by dehydration with minimized loss of transgenic protein using a non-pureeing protocol.

Previous feeding trials have utilized relatively large amounts of material to deliver sufficient doses of the desired antigen. For example, the three human clinical trials referenced above required volunteers to consume a range of 100g to 150g of raw potato at each session, taking some volunteers more than 30mins to consume a single dose. Oral administration of 100g or more of raw material is unlikely to be well accepted by vaccine administrators, or tolerated by vaccines on a broader scale. A major advantage of the process described herein, is the concentration of protein, which is achieved. As shown in Table 2, the material mass was reduced by as much as 94% (11% final weight for potato, 6% final weight for tomato). In comparison to the clinical trials reference above, such reduction in mass would theoretically provide an equivalent dose by consuming just 6 grams of powder, assuming no significant loss of antigen by processing. Additionally the physical qualities of this powdered material are convenient to allow formulation for oral intake by more popular methods, such as consuming several tablespoons of a paste or drink concoction.

The data described thus far provides examples for several antigens and across several plant species and tissues. Removal of more than 90% of the moisture, concentrated the plant samples by up to 11-fold as a measure of dry weight versus wet weight. However, despite the 11-fold reduction in material mass the antigen concentration was generally a 4-8-fold increase depending on the plant species evaluated, indicating significant loss of antigen (or at least a reduction in detection of antigen) as a result of the process. The examples used generally involved the puree of raw materials, addition of any excipients (antioxidants, stabilizers, adjuvants etc), followed by freezing, then freeze-drying, and reduction of the resultant material to a fine powder. As summarized in Table 2 the non-puree protocol described above was shown capable of achieving a material of equivalent characteristics whilst minimizing the loss of antigen in several plant species.

The practical outcome of this procedure is that formulation and oral dosing can be achieved with a significantly lower dose volume, with parallel increase in antigen concentration. Additionally, this method provides a convenient mechanism for stabilizing vaccines or therapeutics from a wider range of plant tissues such as leaf or stem materials which would not otherwise by amenable to homogenization by wet blending without significant disruption to cellular structure and the transgenic proteins of interest.

### Example 19. Ambient stability of homogenate formulations and the effect of excipients on antigen recovery and stability.

Previous studies dependant on extraction of plant-derived pharmaceuticals have evaluated the stability of the target proteins in crude plant extracts. Extraction of a model antibody produced in Alfalfa showed stability up to 2 hours in water extracts and up to 12 weeks in dried alfalfa (Khoudi et.al., 1999). For plant-derived therapeutics to gain most value, particularly as oral vaccine strategies, stability of the antigen in some non-perishable food form would need to extend to at least 6 months, and preferably 2-3 years.

Several formulations expressing model antigens of interest were evaluated for ambient stability of the desired protein at regular periods up to 12 months. As shown in Figure 9, the majority of materials suffered no significant degradation of antigen at ambient storage, or at lower temperatures. This indicates that efficient non-heat dehydration is an effective means of providing a protective state for antigens which are dried alongside plant material or remain encapsulated within plant cells and partly disrupted cell debri. Further examination should be completed, to determine whether the presence of plant enzymes or structural proteins, are sufficient to impart this protection in formulations where antigens are spiked into a non-transgenic homogenate.

Several excipients were added to these model formulations, for both immunogenic evaluation and to investigate their effect on plant material processing, antigen recovery, and antigen stability. Previous reports (Leopold, 1998) concluded that simple vitrified sugars were capable of stabilizing plant enzymes by protecting against natural protein denaturation. Food grade sucrose was added to transgenic tomato homogenate at the time of pureeing to evaluate its effect on the stability of an antigen encapsulated in plant material. In addition to sugars, experimental adjuvants were added to potato or tomato materials in liquid phase (post-puree) for clinical evaluation, and Ascorbic Acid (Aldrich) was added to some samples to evaluate the potential for antioxidants to minimize antigen loss in pureed potato. The effects of these excipients on antigen recovery and stability were inconsistent, although not severe in any single case. The addition of sucrose did significantly increase the drying time required to reach an adequately dehydrated product. The addition of Ascorbic Acid was required at concentrations not less than 1:150 (by mass) to effectively block visible oxidation (browning) of pureed potato, however this concentration had no consistent effect on recoverable antigen levels within the samples tested.

In general, all freeze dried materials displayed stability of the antigen at ambient temperatures for at least 6 months and in some cases up to 12 months. Whilst freeze-drying was the principal method of dehydration in these examples, crudely pulped materials could alternatively be stabilized by air-drying, or spray drying prior to storage. Ideally, by whatever drying method, the final material is dehydrated sufficiently to remove at least 90% of all water content by weight, and stored in airtight bags or containers. Once sealed, the homologous powder can be stored at room temperature for significant periods without any significant decrease in measurable levels of the target protein.

### Example 20. Animals fed dried immunocontraceptive transgenic tomato showed reduction in litter size compared to control animals.

Within this study an orally delivered, plant-derived, LTB-mouse ZP3 epitope fusion protein co-administered with a saponin adjuvant displayed species-specificity and reduced mice fertility by 45.3%

The transgenic tomato fruit used in this study expressed LTB fused to an epitope from the mouse ZP3 gene as described by Millar et al. Three test diets were used: wild-type tomato (control), transgenic tomato (transgenic), and transgenic tomato with Quillaja extract (transgenic + adjuvant). Freeze-dried, powdered, tomato fruit was mixed 1:2 (by weight) with pasteurized apple cider (Cornell Orchards). The concentration of LTB within tomato fruit was taken as an approximation as to the concentration of fusion protein. Tomato fruit expressing over 8 g LTB per gram of dry mass were used in the test diet for the two transgenic groups. After pooling the transgenic fruit the calculated average LTB or fusion protein was 37.8 g.g-1. We also added 10 mg of Quillaja extract powder (Garuda) per 4 g of feed for the trans+quill group. At each feeding, the female was offered 4 g of the test-feed in a petri dish. In treatments consisting of transgenic fruit, this amounted to a total of 100.8 g of the LTB fusion protein per feeding.

Both mice and voles were provided with the test-diet at fortnightly intervals for 12 weeks, on days 0, 3, 14, 17, 28, 31, 42, 45, 56, 59, 70, 73, 84, and 87.

For Mice, on the first five test diet feedings days (days 0 to 28) the females were taken from their home cages, placed into individual cages containing the test diet and left for four to six hours before being returned to their home cages. Starting at day 31, female mice were subjected to a fasting protocol in which at noon, they were removed to individual cages with water and bedding only. The test feed was given at 4pm and left overnight. At 8 am the following morning, females were returned to their home cage. With both feeding regimen, the final weight of test diet consumed was recorded.

For voles, immediately prior to offering a female the test-feed, the male and any pups were removed from the home cage and transferred to a temporary holding cage to prevent them consuming the test-diet. They were returned after a maximum of one hour to reduce any stress caused by the separation. If the female had not consumed all the tomato mix within this time, the remaining portion was removed and offered again four hours later, following the same feeding procedure. The final weight consumed was recorded.

The number of litters and pups produced per female was recorded throughout the experiment for both mice an voles.

With the mice, no significant difference was found between the diet treatments with respect to number of litters produced (∝= 0.05). However there was a 45.3% reduction in litter size from the females fed the control diet (average of 5.3 pups per litter) to the litter size of females fed the transgenic + adjuvant diet (average of 2.9 pups per litter). This proved a significant reduction using a confidence interval of 90% (α = 0.1).

With the Voles, no significant difference was found between the diet treatments with respect to number of litters produced (α = 0.05). There was also no significant difference between diet treatments with respect to litter size using a confidence interval of 95% (α = 0.05 and α= 0.1).

### Example 21. Effect of processing on antigen content

To determine the effect of freeze-drying on mass and antigen content, two fruit from five T₂ plants were weighed and assayed for LTB content before and after processing. Comparison of sample mass before and after freeze-drying revealed the process to decrease the sample to 6% of it's original mass (data not shown). ELISA analysis of LTB content of fresh and processed materials displayed the the antigen to show a similar 16-fold concentration, suggesting no appreciable loss of antigen by the processing method employed (Figure 10).

### Example 22. Oral imunization of mice with plant derived vaccine passively immunize offspring.

### Animals

Mice were maintained in cages provisioned with water and standard laboratory food except when fed the test diet. Each treatment contained 5-6 adult females. Mice were partnered in a ratio of either three females to one male or two females to one male per cage. Pups were removed when weaned and kept in single sex cages.

### Test Diet

Transgenic tomato lines expressing LTB were grown within approved greenhouse facilities. Harvested tomato fruit was processed into a powdered form and combined as a uniform vaccine batch. This batch was stored at 4°C prior to use. Vaccine formulation was achieved by mixing transgenic tomato powder with apple cider in a ration of 1:2 (by weight). An adjuvanted formulation was achieved by augmenting the basic vaccine formulation with 10mg of Quillaia extract for every 4g of transgenic powder.

### Vaccination Regime

Feedings were performed at fortnightly intervals for 12 weeks (days 0, 3, 14, 17, 28, 31, 42, 45, 56, 59, 70, 73, 84, 87). Starting at day 31, the females were subjected to a fasting protocol in which they were removed to individual cages with water and bedding only for four hours before adding the test diet. The tomato diet was left overnight with the females and removed at 8 am the following morning at which time females were returned to their regular cage. As described above, the animals received either non-transgenic formulation (n=4), transgenic formulation (n=5) or adjuvanted formulation (n=5).

### Serum Sampling

On days 0 and 70, serum samples were collected from each adult female by tail bleed, and stored at -80°C until analysis. Final serum samples were collected on day 96 through cardiac puncture and stored as previously described. After day 42 of the trial, pups born to each experimental group (n= minimum of 13 per group, from 2 or more mothers per group) were maintained in good health for later determination of passive anti-LTB titers. At a minimum age of 21 days serum was collected from these pups by cardiac puncture.

### ELISAs for anti-LTB antibodies

Samples were assayed for serum anti-LTB IgG as described [8]. Briefly, samples were serially diluted in PBS containing 0.05% Tween-20 (PBST). Microtiter plates were coated with mixed gangliosides (Type III), Sigma, St Louis, MO, USA, blocked with 0.25% BSA and incubated with purified recombinant LTB for one hour. The diluted serum was added to the wells and incubated for one hour before washing and detection using rabbit antiserum against mouse IgG and goat anti-rabbit antiserum conjugated with alkaline phosphatase (Sigma).

We investigated the relationship between dam and pup titers by comparing the end point dilutions of dams and corresponding litters. The dams' dilution was taken as the end point dilution of the serum sample that was taken closest to the birth date of the pups (either at 70 or 96 days). Only pups that were bled at 21 days of age were used for this analysis. The data set consisted of 7 dams, 9 litters and 41 pups.

### Treatment and Litters

Of the pups sampled for LTB titer determination, 18 pups from 4 litters were born to 3 different mothers from the control group; 17 pups from 5 litters were born to 4 different mothers from the transgenic tomato group and 13 pups from 3 litters were born to 2 different mothers from the transgenic tomato plus adjuvant group.

### ELISAs for anti-LTB antibodies

Parent mice within the transgenic tomato or transgenic tomato plus adjuvant diet groups received 14 oral inoculations of tomato averaging 75.6 µg of LTB per inoculation. The number of inoculations the mother had received before birthing the sampled pups varied, however at least 8 feedings of the diet had occurred. All of the mothers receiving transgenic tomato diet generated anti-LTB serum IgG titers significantly higher than the control group by day 70 of the trial (Figure 11 , α = 0.05). There was no significant difference in titers between the two transgenic material test diets, (transgenic tomato powder with and without the addition of Quillaja extract powder). Of the 18 pups born to mothers in the control group, none possessed an end point dilution larger than 25 (Figure 12). The average end point dilution of the 17 pups born to mothers in the transgenic tomato group was 558.8 whilst the average end point for the 13 pups born to the mothers in the transgenic tomato plus adjuvant groups was 448.1. Whilst no significant difference was found between the average end point dilutions of the pups from transgenic tomato and transgenic tomato plus adjuvant mothers (α= 0.05), the average dilution end points of pups from both transgenic diets were significantly higher than the control groups' (α = 0.05). A positive, linear relationship was found between dams and their corresponding litters with an r2 value of 0.9289 (Figure 13).

### Example 23. Stability Studies

A number of different transgenic plant materials were processed by means described herein. Materials included transgenic NT-1 cell culture expressing either AIV-HA (HAO NT) or a modified E.coli enterotoxin (SLT102), transgenic potato expressing either AIV-HA, NVCP, HBsAg or E.coli enterotoxin B subunit (LT-B), and transgenic tomato expressing either NVCP or HBsAg. Processed materials were stored under a variety of temperatures for periods beyond 6 months. At regular intervals during that period, randomized samples were evaluated for antigen content (by appropriate assay for the antigen of interest). Figures 14 to 17 describe the detectable antigen levels retained in those materials during the sampling period.

### References

Arakawa T, Yu J, Chong DK, Hough J, Engen PC, Langridge WH: "A plant-based cholera toxin B subunit-insulin fusion protein protects against the development of autoimmune diabetes"; Nat Biotechnol 16: 934-8 (1998).
Arikawa, J., K. Yoshimatsu, and K. H.: "Epidemiology and epizootiology of hantavirus infection in Japan."; Jpn. J. Infect. Dis., 54, no. 3: 95-102 (2001).
Bagdasarian MM, Nagai M, Frey J, Bagdasarian M: "Immunogenicity ofActinobacillus ApxIA toxin eptiopes fused to the E. coli heat-labile enterotoxin B subunit". Vaccine 17: 441-447. (1999).
Bandivdekar, A.H., V.J. Vemekar, S.B. Moodbidri et al.: "Characterization of 80 kDa human sperm antigen responsible for immunoinfertility. "Am. J Reprod. Immunol"; 45, no. 1: 28-34 (2001).
Becker D, Kemper E, Schell J, Masterson R: "New plant binary vectors with selectable markers located proximal to the left T-DNA border. Plant Molecular Biology"; 20: 1195-1197 (1992).
Berger, J., and S.L. Cain: "Reproductive synchrony in brucellosis-exposed bison in the southern Greater Yellowstone Ecosystem and in noninfected populations."; Conservation Biology, 13, no. 2: 357-366 (1999).
Bosshardt, S.C., et al.: "Brugia pahangi: circulating antibodies to adult worm antigens in uninfected progeny of homologously infected female jirds"; Exp Parasitol, 72(4): p. 440-449 (1991).
Cardenas L, Clements JD: "Development of mucosal protection against the heat-stable enterotoxin (ST) of Escherichia coli by oral immunization with a genetic fusion delivered by a bacterial vector"; Infect Immun 61: 4629-36 (1993).
Chavali, S.R. and J.B. Campbell: "Adjuvant effects of orally administered saponins on humoral and cellular immune responses in mice"; Immunobiology, 174(3): p. 347-359 (1987).
Cousins, D.V.: "Mycobacterium bovis infection and control in domestic livestock. " Rev. Sci. Tech."; 20, no. 1: 71-85 (2001).
Cox, J.C., and A.R. Coulter: "Adjuvants--a classification and review of their modes of action." Vaccine"; 15, no. 3: 248-256 (1997).
Dalsgaard, K., et al.: "Plant-derived vaccine protects target animals against a viral disease". Nat Biotechnol, 15(3): p. 248-52 (1997).
Dickinson, B.L. and J.D. Clements: "Dissociation of Escherichia coli heat-labile enterotoxin adjuvanticity from ADP-ribosyltransferase activity"; Infect Immun, 63(5): p. 1617-23 (1995).
Dunbar, B.S., and S.V. Prasad: "Contraceptive vaccine comprising a glycosylated 55KD zona pellucida protein immunogen and method of use of the same in contraception." United States, 1997.
Edelman, R. "An update on vaccine adjuvants in clinical trial"; AIDS Res. Hum. Retroviruses; 8, no. 8: 1409-1411 (1992).
Fitchen J, Beachy RN, Hein MB: "Plant virus expressing hybrid coat protein with added murine epitope elicits autoantibody response"; Vaccine 13: 1051-7 (1995).
Frary A, Earle ED: "An examination of factors affecting the efficiency of Agrobacterium-mediated transformation of tomato"; Plant Cell Reports 16: 235-240 (1996).
Haq TA, Mason HS, Clements JD, Arntzen CJ.: "Oral immunization with a recombinant bacterial antigen produced in transgenic plants"; Science 268:714-716 (1995).
Hiatt AC, Cafferkey R, Bowdish K.: "Production of antibodies in transgenic plants"; Nature 342: 76-78 (1989).
Hoshi, S., A. Uchino, N. Saito et al.: "Comparison of adjuvants with respect to serum IgG antibody response in orally immunized chickens"; Comp. Immunol. Microbiol. Infect. Dis., 22, no. 1: 63-69 (1999).
Ingardia, C.J., et al., "Correlation of maternal and fetal hepatitis B antibody titers following maternal vaccination in pregnancy"; Am. J. Perinatology, 16(3): p. 129-132 (1999).
Kennelly, J.J., and K.A. Converse: "Surgical sterilization: an underutilized research procedure for wildlife damage control"; In Proceeding of symposium: Contraception in wildlife management. Washington, D.C.: USDA/APHIS.
Khoudi H, Laberge S, Ferullo J-M, Bazin R, Darveau A, Castonguay Y, Allard G, Lemieux R, Vezina L-P: "Production of a diagnostic monoclonal antibody in perennial alfalfa plants. Biotechnology and Bioengineering"; 64(2): 135-143 (1999).
Koo, M., et al.: "Protective immunity against murine hepatitis virus (MHV) induced by intranasal or subcutaneous administration of hybrids of tobacco mosaic virus that carries an MHV epitope"; Proc Natl Acad Sci U S A, 96(14): p. 7774-9 (1999).
Kuby, J. "Immunolgy"; Third ed., ed. Allen, D. New York: W. W. Freeman and Company, 1997.
Leopold C.: "A sweet way to keep proteins safe"; Science. 281: 179 (1998)
Ma J.K.-C, Hein M.B. "Plant antibodies for immunotherapy"; Plant Physiol. 109: 341-346 (1995).
Marciani, D.J., J.B. Press, R.C. Reynolds et al.: "Development of semisynthetic triterpenoid saponin derivatives with immune stimulating activity"; Vaccine, 18, no. 27: 3141-3151 (2000).
Martinez, M.L., and J.D. Harris: "Effectiveness of zona pellucida protein ZPB as an immunocontraceptive antigen"; J. Reprod. Fertil., 120, no. 1: 19-32 (2000).
Mason H, Lam D, Arntzen CJ: "Expression of hepatitis B surface antigen in transgenic plants"; Proc. Natl. Acad. Sci. USA 89:11745-11749 (1992).
Mason HS, Ball J, Shi JJ, Jiang X, Estes MK, Arntzen CJ: "Expression and immunogenicity of Norwalk virus capsid protein from transgenic tobacco and potato"; Proc. Natl. Acad. Sci. USA 93: 5335-5340 (1996).
Mason HS, Haq TA, Clements JD, Arntzen CJ: "Edible vaccine protects mice against Escherichia coli heat-labile enterotoxin (LT): potatoes expressing a synthetic LT-B gene"; Vaccine 16: 1336-43 (1998).
Millar, S.E., S.M. Chamow, A.W. Baur et al.: "Vaccination with a synthetic zona pellucida peptide produces long-term contraception in female mice"; Science 246: 935-938 (1989).
Miller, L.A., B.E. Johns, and G.J. Killian: "Immunocontraception of white-tailed deer with GnRH vaccine"; Am. J. Reprod. Immunol. 44, no. 5: 266-274 (2000).
Miller, L.A., B.E. Johns, D.J. Elias et al.: "Comparative efficacy of two immunocontraceptive vaccines"; Vaccine 15, no. 17-18: 1858-1862 (1997).
Modelska A, Dietzschold B, Sleysh N, Fu ZF, Steplewski K, Hooper DC, Koprowski H, Yusibov V: "Immunization against rabies with a plant-derived antigen"; Proc Natl Acad Sci U S A 95: 2481-5 (1998).
Murashige T, Skoog F: "A revised medium for rapid growth and bioassays with tobacco tissue cultures"; Physiologia Plantarum 15: 473-497 (1962).
O'Dowd AM, Botting CH, Precious B, Shawcross R, Randall RE: "Novel modifications to the C-terminus of LTB that facilitate site-directed chemical coupling of antigens and the development of LTB as a carrier for mucosal vaccines"; Vaccine 17: 1442-1453 (1999).
O'Hern, P.A., Z.G. Liang, C.S. Bambra et al.: "Colinear synthesis of an antigen-specific B-cell epitope with a 'promiscuous' tetanus toxin T-cell epitope: a synthetic peptide immunocontraceptive"; Vaccine 15, no. 16: 1761-1766 (1997).
Pal, R., and O. Singh: "Absence of corpus luteum rescue by chorionic gonadotropin in women immunized with a contraceptive vaccine"; Fertil. Steril., 76 no. 2: 332-336 (2001).
Pillai D, Dixit A, Alok D, Garg LC: "Translational fusion of heat labile enterotoxin chain B and beta- subunit of human chorionic gonadotropin: periplasmic expression in Escherichia coli and its immunogenicity"; FEBS Lett 387: 23-6 (1996).
Richmond, M., and C.H. Conaway: "Management breeding and reproductive performance of the vole, Microtus ochrogaster, in a laboratory colony"; Laboratory Animal Care 19: 80-87 (1969).
Richter L, Thanavala Y, Arntzen CJ, Mason HS: "Production of hepatitis B surface antigen in transgenic plants for oral immunization"; Nature Biotechnol. 18:1167-1171(2000).
Sambrook J, Fritsch EF, Maniatis T: "Molecular Cloning - A Laboratory Manual"; Cold Spring Harbor Laboratory Press, Plainview, New York (1989).
Schodel F, Will H, Johansson S, Sanchez J, Holmgren J: "Synthesis in Vibrio cholerae and secretion of hepatitis B virus antigens fused to Escherichia coli heat-labile enterotoxin subunit B"; Gene 99: 255-9 (1991).
Sewani CR, Bagdasarian MM, Ireland JJ, Bagdasarian M: "Display of an inhibin epitope in a surface-exposed loop of the E. coli heat-labile enterotoxin B subunit"; Vaccine 16: 1611-9 (1998).
Sixma TK, Kalk KH, van Zanten BAM, Dauter Z, Kingma J, Witholt B, Hol WGJ: "Refined structure of Escherichia coli heat-labile enterotoxin, a close relative of cholera toxin"; J. Mol. Biol. 230: 890-918 (1993).
Sixma TK, Pronk SE, Kalk KH, van Zanten BA, Berghuis AM, Hol WG: "Lactose binding to heat-labile enterotoxin revealed by X-ray crystallography"; Nature 355: 561-4 (1992).
Skinner, S.M., G.J. Killian, L.A. Miller et al.: "Characterization of antigenicity and immunogenicity patterns of native and recombinant zona pellucida proteins in the white-tailed deer (Oidocoileus virginianus)"; Journal of Reproduction and Fertility 101: 295-303 (1994).
Soltysik, S., J.Y. Wu, J. Recchia et al.: "Structure/function studies of QS-21 adjuvant: assessment of triterpene aldehyde and glucuronic acid roles in adjuvant function"; Vaccine 13, no. 15: 1403-1410 (1995).
Tacket CO, Mason HS, Losonsky G, Clements JD, Levine MM, Arntzen CJ: "Immunogenicity in humans of a recombinant bacterial antigen delivered in transgenic potato" Nature Medicine 4:607-609 (1998).
Tacket CO, Mason HS, Losonsky G, Estes MK, Levine MM, Arntzen CJ: "Human immune responses to a novel Norwalk virus vaccine delivered in transgenic potatoes"; J. Infect. Dis. 182: 302-305 (2000).
Takahashi, H., T. Takeshita, B. Morein et al.: "Induction of CD8+ cytotoxic T cells by immunization with purified HIV-1 envelope protein in ISCOMs"; Nature 344, no. 6269: 873-875 (1990).
Takikawa, M., M. Kamada, M. Maegawa et al.: "Evaluation of two sperm antigens, rSMP-B and YWK-II, as targets for immunocontraception"Zygote 9, no. 2: 145-151 (2001).
Tung, K.S.K., J. Ang, and Y. Lou.: "ZP3 peptide vaccine that induces antibody and reversible infertility without autoimmune oophoritis"; American Journal of Reproductive Immunology 35: 181-183 (1996).
VandeVoort, C.A., E.D. Schwoebel, and B.S. Dunbar: "Immunization of monkeys with recombinant complimentary deoxyribonucleic acid expressed zona pellucida proteins"; Fertil. Steril. 64, no. 4: 838-847 (1995).
Wada K, Aota S, Tsuchiya R, Ishibashi F, Gojobori T, Ikemura T: "Codon usage tabulated from the GeneBank genetic sequence data"; Nucleic Acid Research 18: 2367-2411 (1990).
Walmsley AM, Arntzen CJ: "Plants for delivery of edible vaccines. Current Opinion in Biotechnology 11"; 126-129 (2000).
Wigdorovitz A, Perez Filgueira DM, Robertson N, Carrillo C, Sadir AM, Morris TJ, Borca MV: "Protection of mice against challenge with foot and mouth disease virus (FMDV) by immunization with foliar extracts from plants infected with recombinant tobacco mosaic virus expressing the FMDV structural protein VP1"; Virology 264: 85-91 (1999).
Wigdorovitz, A., C. Carrillo, M.J. Dus Santos et al.: "Induction of a protective antibody response to foot and mouth disease virus in mice following oral or parenteral immunization with alfalfa transgenic plants expressing the viral structural protein VP1"; Virology 255, no. 2: 347-53 (1999). Wu, J.Y., B.H. Gardner, C.I. Murphy et al.: "Saponin adjuvant enhancement of antigen-specific immune responses to an experimental HIV-1 vaccine"; J. Immunol. 148, no. 5: 1519-1525 (1992).
Yu, J. and W.H. Langridge: "A plant-based multicomponent vaccine protects mice from enteric diseases. Nat Biotechnol"; 19(6): p. 548-552 (2001).

## Claims

1. A method for producing a stable dry homogenate of a transgenic plant expressing a heterologous contraceptive protein comprising:
a) obtaining either intact or partitioned transgenic plant material expressing a heterologous contraceptive protein fused to a mucosal targeting protein;
b) dehydrating said transgenic plant material;
c) mixing said transgenic plant material into a homogenate either before or after said dehydrating step; and
d) adding an adjuvant to said homogenate.

2. The method of claim 1 or claim 2, wherein the dehydration is accomplished by freeze drying, air drying, or spray drying said material.

3. The method of claim 1, further comprising the step of partitioning the dehydrated material before said mixing step.

4. A stable dry homogenate of a transgenic plant expressing a heterologous contraceptive protein fused to a mucosal targeting protein and comprising an adjuvant obtainable by the method according to any one of claims 1 to 3.

5. A stable dry homogenate comprising: (i) a dehydrated transgenic plant material containing a transgenic contraceptive protein fused to a mucosal targeting protein; and (ii) an adjuvant.

6. A pharmaceutical composition comprising the stable dry homogenate according to claim 4 or 5 mixed with a pharmaceutically acceptable carrier

7. A method of contraception comprising:
administering to a non-human animal: (i) a stable dry homogenate of a transgenic plant material comprising a nucleic acid molecule which encodes a contraceptive polypeptide fused to a mucosal targeting protein; and (ii) an adjuvant, and wherein said administering reduces the average number of offspring produced per breeding cycle by said animal by at least 50%,

8. The method according to claim 7, wherein said administering induces a mucosal immune response in said non-human animal to said contraceptive protein

9. The method according to claim 7 or claim 8 , wherein said nucleic acid is integrated into a plant chromosome

10. The method of any of claims 1 to 3 or 9, wherein said transgenic plant material is selected from the group consisting of: leaf, root, shoot, stem, fruit, tuber, flower, and seed.

11. The method according to any of claims 1 to 3, 9 or 10, wherein the transgenic plant material is edible.

12. The method of any of claims 1 to 3, wherein said transgenic plant material is selected from the group consisting of: leaf, root, shoot, stem, fruit, tuber, flower, and seed.

13. The method of any of claims 1 to 3 or 9 to 12, wherein said contraceptive polypeptide encoded by said nucleic acid is selected from the group consisting of a zona pellucida glycoprotein, GnRH, LHRH, LH, LDH and anti-sperm antigens.

14. The method of claim 13 wherein said zona pellucida glycoprotein is selected from the group consisting of ZP1, ZP2, ZP3, and ZP4.

15. The method of claim 14, wherein said zona pellucida glycoprotein is ZP3.

16. The method of any of claims 1 to 3 or 9 to 15, wherein said contraceptive protein is species-specific.

17. The method of any one of claims 7 to 16, wherein said mucosal targeting protein in the *E. coli* enterotoxin subunit B.

18. The method of any of claims 7 to 10, wherein said transgenic plant material is administered as a whole plant.

19. The method of any of claims 7 to 10, wherein said transgenic plant material is administered by mucosal delivery.

20. The method of claim 19, wherein said mucosal administration is oral, nasal, or ocular delivery.

21. A method of inducing a mucosal immune response comprising:
administering to a non-human animal: (i) a stable dry homogenate of a transgenic plant material comprising a nucleic acid sequence which encodes a contraceptive polypeptide fused a mucosal targeting protein; and (ii) an adjuvant, wherein said administering induces a mucosal immune response in said non-human animal to said contraceptive protein, and wherein said administering reduces the average number of offspring produced per breeding cycle by said non-human animal by at least 50%.

22. The method of any of claims 7 to 10, wherein said transgenic plant material is selected from the group consisting of: leaf, root, shoot, stem, fruit, tuber, flower, and seed.

23. The method of any of claims 7 to 10, wherein said transgenic plant material is in the form of an edible plant.

24. The method of any of claims 21 to 23, wherein said contraceptive polypeptide encoded by said nucleic is selected from the group consisting of a zona pellucida glycoprotein, GnRH, LHRH, LH, LDH and anti-sperm antigen.

25. The method of any of claims 21 to 23, wherein said zona pellucida glycoprotein is selected from the group consisting of ZP1, ZP2, ZP3 and ZP4.

26. The method of any of claims 21 to 23, wherein said zona pellucida glycoprotein is ZP3.

27. The method of any of claims 21 to 26, wherein said contraceptive protein is species-specific.

28. The method of any of claims 21 to 27, wherein said mucosal targeting protein is the *E.coli* enterotoxin subunit B.

29. A composition comprising a saponin adjuvant in admixture with a processed stable dry homogenate of a transgenic plant material expressing a heterologous contraceptive protein fused to a mucosal targeting protein for oral administration to a non-human animal, wherein said administration reduces the number of offspring produced by said non-human animal per breeding cycle by said non-human animal by at least 50%.

## Patentansprüche

1. Verfahren zur Herstellung eines stabilen trockenen Homogenats einer transgenen Pflanze, die ein heterologes kontrazeptives Protein exprimiert, umfassend:
a) Erhalten von intaktem oder abgetrenntem transgenem Pflanzenmaterial, das ein heterologes kontrazeptives Protein, fusioniert an ein mukosales Targeting-Protein, exprimiert;
b) Dehydratisieren des transgenen Pflanzenmaterials;
c) Mischen des transgenen Pflanzenmaterials zu einem Homogenat, entweder vor oder nach dem Schritt des Dehydratisierens, und
d) Zugeben eines Adjuvans zu dem Homogenat.

2. Verfahren nach Anspruch 1 oder Anspruch 2, wobei die Dehydratisierung durch Gefriertrocknung, Lufttrocknung oder Sprühtrocknung des Materials erreicht wird.

3. Verfahren nach Anspruch 1, das außerdem den Schritt des Abtrennens bzw. Aufteilens des dehydratisierten Materials vor dem Schritt des Mischens umfasst.

4. Stabiles trockenes Homogenat einer transgenen Pflanze, die ein heterologes kontrazeptives Protein, fusioniert an ein mukosales Targeting-Protein, exprimiert, und das ein Adjuvans umfasst, das durch das Verfahren nach einem der Ansprüche 1 bis 3 erhältlich ist.

5. Stabiles trockenes Homogenat, umfassend: (i) ein dehydratisiertes transgenes Pflanzenmaterial, das ein transgenes kontrazeptives Protein, fusioniert ein mukosales Targeting-Protein, enthält, und (ii) ein Adjuvans.

6. Pharmazeutische Zusammensetzung, die das stabile trockene Homogenat nach Anspruch 4 oder 5 vermischt mit einem pharmazeutisch verträglichen Träger umfasst.

7. Kontrazeptionsverfahren, umfassend:
Verabreichen an ein nicht-menschliches Tier: (i) ein stabiles trockenes Homogenat eines transgenen Pflanzenmaterials, das ein Nukleinsäuremolekül umfasst, welches ein kontrazeptives Polypeptid, fusioniert an ein mukosales Targeting-Protein, kodiert und (ii) ein Adjuvans, und wobei das Verabreichen die durchschnittliche Anzahl von Nachkommen, die pro Zuchtcyclus durch das Tier produziert werden, um wenigstens 50% verringert.

8. Verfahren nach Anspruch 7, wobei das Verabreichen eine mukosale Immunantwort in dem nicht-menschlichen Tier auf das kontrazeptive Protein produziert.

9. Verfahren nach Anspruch 7 oder Anspruch 8, wobei die Nukleinsäure in ein Pflanzenchromosom integriert ist.

10. Verfahren nach einem der Ansprüche 1 bis 3 oder 9, wobei das transgene Pflanzenmaterial ausgewählt ist aus der Gruppe, bestehend aus: Blatt, Wurzel, Schössling, Stängel, Frucht, Knolle, Blüte und Samen.

11. Verfahren nach einem der Ansprüche 1 bis 3, 9 oder 10, wobei das transgene Pflanzenmaterial essbar ist.

12. Verfahren nach einem der Ansprüche 1 bis 3, wobei das transgene Pflanzenmaterial ausgewählt ist aus der Gruppe, bestehend aus: Blatt, Wurzel, Schössling, Stängel, Frucht, Knolle, Blüte und Samen.

13. Verfahren nach einem der Ansprüche 1 bis 3 oder 9 bis 12, wobei das kontrazeptive Polypeptid, das durch die Nukleinsäure kodiert wird, aus der Gruppe, bestehend aus einem Zona pellucida-Glycoprotein, GnRH, LHRH, LH, LDH und Anti-Sperma-Antigenen, ausgewählt wird.

14. Verfahren nach Anspruch 13, wobei das Zona pellucida-Glycoprotein aus der Gruppe, bestehend aus ZP1, ZP2, ZP3 und ZP4, ausgewählt wird.

15. Verfahren nach Anspruch 14, wobei das Zona pellucida-Glycoprotein ZP3 ist.

16. Verfahren nach einem der Ansprüche 1 bis 3 oder 9 bis 15, wobei das kontrazeptive Protein Spezies-spezifisch ist.

17. Verfahren nach einem der Ansprüche 7 bis 16, wobei das mukosale Targeting-Protein in der E.coli-Enterotoxin-Untereinheit B ist.

18. Verfahren nach einem der Ansprüche 7 bis 10, wobei das transgene Pflanzenmaterial als ganze Pflanze verabreicht wird.

19. Verfahren nach einem der Ansprüche 7 bis 10, wobei das transgene Pflanzenmaterial durch mukosale Abgabe verabreicht wird.

20. Verfahren nach Anspruch 19, wobei die mukosale Verabreichung eine orale, nasale oder okulare Abgabe ist.

21. Verfahren zum Induzieren einer mukosalen Immunantwort, umfassend:
Verabreichen an ein nicht-menschliches Tier: (i) ein stabiles trockenes Homogenat eines transgenen Pflanzenmaterials, das eine Nukleinsäuresequenz, welche ein kontrazeptives Polypeptid, fusioniert an ein mukosales Targeting-Protein, kodiert, und (ii) ein Adjuvans umfasst, wobei das Verabreichen eine mukosale Immunantwort in dem nicht-menschlichen Tier auf das kontrazeptive Protein induziert und
wobei das Verabreichen die durchschnittliche Zahl von Nachkommen, die pro Zuchtcyclus durch das nicht-menschliche Tier produziert werden, um wenigstens 50% verringert.

22. Verfahren nach einem der Ansprüche 7 bis 10, wobei das transgene Pflanzenmaterial aus der Gruppe, bestehend aus:
Blatt, Wurzel, Schössling, Stängel, Frucht, Knolle, Blüte und Samen, ausgewählt wird.

23. Verfahren nach einem der Ansprüche 7 bis 10, wobei das transgene Pflanzenmaterial in der Form einer essbaren Pflanze ist.

24. Verfahren nach einem der Ansprüche 21 bis 23, wobei das kontrazeptive Polypeptid, das durch die Nukleinsäure kodiert wird, ausgewählt ist aus der Gruppe, bestehend aus einem Zona pellucida-Glycoprotein, GnRH, LHRH, LH, LDH und Anti-Sperma-Antigen.

25. Verfahren nach einem der Ansprüche 21 bis 23, wobei das Zona pellucida-Glycoprotein aus der Gruppe, bestehend aus ZP1, ZP2, ZP3 und ZP4, ausgewählt wird.

26. Verfahren nach einem der Ansprüche 21 bis 23, wobei das Zona pellucida-Glycoprotein ZP3 ist.

27. Verfahren nach einem der Ansprüche 21 bis 26, wobei das kontrazeptive Protein Spezies-spezifisch ist.

28. Verfahren nach einem der Ansprüche 21 bis 27, wobei das mukosale Targeting-Protein die E.coli-Enterotoxin-Untereinheit B ist.

29. Zusammensetzung, die ein Saponin-Adjuvans im Gemisch mit einem verarbeiteten stabilen trockenen Homogenat eines transgenen Pflanzenmaterials, das ein heterologes kontrazeptives Protein, fusioniert an ein mukosales Targeting-Protein, umfasst, zur oralen Verabreichung an ein nicht-menschliches Tier, wobei die Verabreichung die Anzahl an Nachkommen, die durch das nicht-humane Tier pro Zuchtcyclus durch das nicht-humane Tier produziert werden, um wenigstens 50% verringert.

## Revendications

1. Procédé de production d'un homogénat sec et stable de plante transgénique exprimant une protéine contraceptive hétérologue, lequel procédé comporte les étapes suivantes :
a) obtenir, à l'état entier ou divisé, un matériau de plante transgénique exprimant une protéine contraceptive hétérologue fusionnée avec une protéine mucosale de ciblage ;
b) déshydrater ledit matériau de plante transgénique ;
c) avant ou après cette étape de déshydratation, mixer ledit matériau de plante transgénique de manière à en faire un homogénat ;
d) et ajouter un adjuvant à cet homogénat.

2. Procédé conforme à la revendication 1, dans lequel la déshydratation est effectuée par lyophilisation, séchage à l'air ou séchage par pulvérisation dudit matériau.

3. Procédé conforme à la revendication 1 ou 2, qui comporte en outre, avant ladite étape de mixage, une étape consistant à diviser le matériau déshydraté.

4. Homogénat sec et stable d'une plante transgénique exprimant une protéine contraceptive hétérologue fusionnée avec une protéine mucosale de ciblage, comprenant un adjuvant, accessible par un procédé conforme à l'une des revendications 1 à 3.

5. Homogénat sec et stable, comprenant :
i) un matériau de plante transgénique déshydraté, contenant une protéine contraceptive transgénique fusionnée avec une protéine mucosale de ciblage,
ii) et un adjuvant.

6. Composition pharmaceutique comprenant un homogénat sec et stable, conforme à la revendication 4 ou 5, mélangé avec un véhicule pharmacologiquement admissible.

7. Procédé de contraception, comprenant le fait d'administrer à un animal non humain :
i) un homogénat sec et stable d'un matériau de plante transgénique comprenant une molécule d'acide nucléique qui code un polypeptide contraceptif fusionné avec une protéine mucosale de ciblage,
ii) et un adjuvant,
laquelle opération d'administration réduit d'au moins 50 % le nombre moyen de membres, par cycle de reproduction, d'une portée dudit animal.

8. Procédé conforme à la revendication 7, dans lequel ladite opération d'administration induit, chez ledit animal non-humain, une réponse immune mucosale à ladite protéine contraceptive.

9. Procédé conforme à la revendication 7 ou 8, dans lequel ledit acide nucléique est intégré dans un chromosome de plante.

10. Procédé conforme à l'une des revendications 1 à 3 et 9, dans lequel ledit matériau de plante transgénique est choisi dans l'ensemble constitué par les feuilles, racines, pousses, tiges, fruits, tubercules, fleurs et graines.

11. Procédé conforme à l'une des revendications 1 à 3, 9 et 10, dans lequel le matériau de plante transgénique est comestible.

12. Procédé conforme à l'une des revendications 1 à 3, dans lequel ledit matériau de plante transgénique est choisi dans l'ensemble constitué par les feuilles, racines, pousses, tiges, fruits, tubercules, fleurs et graines.

13. Procédé conforme à l'une des revendications 1 à 3 et 9 à 12, dans lequel ledit polypeptide contraceptif codé par ledit acide nucléique est choisi dans l'ensemble constitué par une glycoprotéine de zone pellucide, les hormones GnRH, LHRH et LH, l'enzyme LDH, et les antigènes anti-sperme.

14. Procédé conforme à la revendication 13, dans lequel ladite glycoprotéine de zone pellucide est choisie parmi les glycoprotéines ZP1, ZP2, ZP3 et ZP4.

15. Procédé conforme à la revendication 14, dans lequel ladite glycoprotéine de zone pellucide est la glycoprotéine ZP3.

16. Procédé conforme à l'une des revendications 1 à 3 et 9 à 15, dans lequel ladite protéine contraceptive présente une spécificité d'espèce.

17. Procédé conforme à l'une des revendications 7 à 16, dans lequel ladite protéine mucosale de ciblage est la sous-unité B de l'entéro-toxine d'*E. coli.*

18. Procédé conforme à l'une des revendications 7 à 10, dans lequel ledit matériau de plante transgénique est administré sous forme de plante entière.

19. Procédé conforme à l'une des revendications 7 à 10, dans lequel ledit matériau de plante transgénique est administré par voie trans-muqueuse.

20. Procédé conforme à la revendication 19, dans lequel ledit matériau de plante transgénique est administré par la bouche, le nez ou l'oeil.

21. Procédé permettant d'induire une réponse immune mucosale, lequel procédé comporte le fait d'administrer à un animal non-humain :
i) un homogénat sec et stable d'un matériau de plante transgénique comprenant une séquence d'acide nucléique qui code un polypeptide contraceptif fusionné avec une protéine mucosale de ciblage,
ii) et un adjuvant,
laquelle opération d'administration induit, chez ledit animal non-humain, une réponse immune mucosale à ladite protéine contraceptive, et laquelle opération d'administration réduit d'au moins 50 % le nombre moyen de membres, par cycle de reproduction, d'une portée dudit animal non-humain.

22. Procédé conforme à l'une des revendications 7 à 10, dans lequel ledit matériau de plante transgénique est choisi dans l'ensemble constitué par les feuilles, racines, pousses, tiges, fruits, tubercules, fleurs et graines.

23. Procédé conforme à l'une des revendications 7 à 10, dans lequel le matériau de plante transgénique se présente sous forme de plante comestible.

24. Procédé conforme à l'une des revendications 21 à 23, dans lequel ledit polypeptide contraceptif codé par ledit acide nucléique est choisi dans l'ensemble constitué par une glycoprotéine de zone pellucide, les hormones GnRH, LHRH et LH, l'enzyme LDH, et les antigènes anti-sperme.

25. Procédé conforme à l'une des revendications 21 à 23, dans lequel ladite glycoprotéine de zone pellucide est choisie parmi les glycoprotéines ZP1, ZP2, ZP3 et ZP4.

26. Procédé conforme à l'une des revendications 21 à 23, dans lequel ladite glycoprotéine de zone pellucide est la glycoprotéine ZP3.

27. Procédé conforme à l'une des revendications 21 à 26, dans lequel ladite protéine contraceptive présente une spécificité d'espèce.

28. Procédé conforme à l'une des revendications 21 à 27, dans lequel ladite protéine mucosale de ciblage est la sous-unité B de l'entéro-toxine d'*E. coli.*

29. Composition comprenant un adjuvant de type saponine, mélangé avec un homogénat sec et stable obtenu par traitement d'un matériau de plante transgénique exprimant une protéine contraceptive hétérologue fusionnée avec une protéine mucosale de ciblage, conçue pour être administrée par la bouche à un animal non-humain, laquelle opération d'administration réduit d'au moins 50 % le nombre moyen de membres, par cycle de reproduction, d'une portée dudit animal non-humain.
